(19)

Europäisches
Patentamt
European
Patent Office
Office européen
des brevets

(11) **EP 2 289 348 A2**

(12) **EUROPEAN PATENT APPLICATION**

(43) Date of publication:
**02.03.2011 Bulletin 2011/09**

(51) Int Cl.:
***A23K 1/14*** *(2006.01)*

(21) Application number: **10179095.4**

(22) Date of filing: **24.03.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL LT LV MK**

(30) Priority: **25.03.2003 DK 200300453
12.08.2003 US 494082 P
11.08.2003 DK 200301154**

(62) Document number(s) of the earlier application(s) in
accordance with Art. 76 EPC:
**04722870.5 / 1 610 623**

(71) Applicant: **Aarhus Universitet
8000 Århus C (DK)**

(72) Inventors:
 • **Hansen, Laurits Lydehøj
   8830, Tjele (DK)**

 • **Jensen, Mogens T
   8830, Tjele (DK)**
 • **Byrne, Derek
   2670, Greve (DK)**
 • **Roepstorff, Allan
   2610, Rødovre (DK)**
 • **Thamsborg, Stig Milan
   2820, Gentofte (DK)**
 • **Mejer, Helena
   1916, Frederiksberg C (DK)**

(74) Representative: **Høiberg A/S
St. Kongensgade 59 A
DK-1264 Copenhagen K (DK)**

Remarks:
This application was filed on 24-09-2010 as a
divisional application to the application mentioned
under INID code 62.

(54) **Methodologies for improving the quality of meat, health status of animals and impact on
environment**

(57)     Disclosed is a method and a product of a chicory root product for reducing taint in animals, said method comprising feeding to an animal a chicory root product during at least one day prior to slaughtering the animal. By feeding animals with the chicory root product this improves the quality of meat, prevents or reduces female and male animal taint, primarily boar taint caused by skatole and/or androstenone. The invention also relates to methods for improving the health status of animals e.g. by reducing infections by pathogens in the gastrointestinal tract and to methods for reducing animal caused odours in general. The chicory root product comprises inulin/fructan (fructooligosaccarides) other low molecular weight sugars and secondary metabolites.

**EP 2 289 348 A2**

## Description

All patent and non-patent references cited in the present application are hereby incorporated by reference in their entirety.

## Field of invention

The present invention relates to methods and compositions for improving the quality of meat, to methods and compositions for preventing or reducing male animal taint, primarily boar taint caused by skatole and/or androstenone. The invention also relates to methods for improving the health status of animals e.g. by reducing infections by pathogens in the gastrointestinal tract and to methods for reducing animal caused odours in general.

## Background of invention

### Boar taint

Boar taint is a large problem in agriculture. The phenomenon referred to as "boar taint" is an ill-defined complex problem from a causal mechanistic standpoint that is characterised in pork meat by off-odours and flavours from a human sensory perspective. In addition, the live animals that lead to boar taint in meat also impart highly unacceptable off-odours to their environmental surroundings.

Although the term "boar taint" implies that the problem is restricted to boars (sexually mature male pigs), the problem is by no means exclusive to such animals. Male pigs in general and to a lesser extent female and castrated male pigs also exhibit the phenomena associated with boar taint or pig off odour. In addition, the negative effects of boar taint increase with the increasing age of the animals.

Boar taint is generally believed to be caused by at least two contributing factors, skatole and androstenone (Bonneau et al., 2000; Dijksterhuis et al., 2000). Skatole is formed by microbial breakdown of tryptophane in the gastrointestinal tract of pigs, in particular in the colon and in the caecum. Androstenone is synthesised in the testicles. Both compounds are metabolised in the liver. Some boars have a lower rate of metabolism in the liver and consequently these animals result in meat that contains boar taint to a higher extent than the average pig.

The phenomena associated with boar taint and/or pig off-odour are several. First and foremost the odour and flavour of pork meat is affected negatively in particular due to the presence of skatole and/or androstenone over certain levels. The odour and flavour may be affected to such an extent that the meat is not acceptable for human consumption. In addition, live (fattening) pigs are associated with an unpleasant odour caused by volatile microbial metabolites in their excreta. The unpleasant odours mainly stem from microbial produced volatiles in liquid manure (mixture of faeces and urine). Two important volatile components of liquid manure are p-Cresol and skatole plus ammonia. The net result of this aspect of the pig off-odour phenomena constitutes an environmental problem in terms of publicly unacceptable negative odours imparted to the surroundings of large pig farms (Hartung & Rokicki, 1984; Hidaka et al., 1986; Sutton et al., 1999). Ammonium evaporates as $NH_3$ and acidifies the environment. Fixing of nitrogen to less volatile compounds during passage through the gastrointestinal tract would thus be desirable.

Danish (and other) slaughterhouses have set thresholds for the allowable amount of skatole in entire male pigs backfat. The limit today is 0.25 ppm of skatole in the backfat of entire male pigs. In the past, the limit was set to 0.20 ppm and with this limit approximately 8 % of all male pigs had to be discarded. With the present level of 0.25 ppm approximately 5 % of all male pigs are discarded as boar tainted meat. The meat is then used in sausage manufacture in association with boar taint free meat, such that the negative boar taint odours and flavours are masked and thus are not a problem in human acceptability terms any longer especially when eaten cold. However, in this context the pork meat does not realise its original potential economic value. Pigs with elevated skatole contents thus constitute a substantial economic loss to agriculture. Therefore, there is a large monetary incentive to reduce and minimise the percentage of animals with high levels of skatole in the pig population.

The limit of 0.20-0.25 ppm skatole has been more or less arbitrarily set thus far and in practice skatole also negatively affects the sensory properties of pork meat from entire and castrated male pigs at concentrations of as low as 0.15 ppm (Gibis et al., 1998) and maybe has negative effects at even lower levels when in combination with higher concentrations of androstenone and other negative odorous compounds. Reducing the concentration of skatole below 0.15 ppm to as close to zero as possible will result in elevated quality of all pork meat from a human sensory perspective and consequently allow higher prices to be obtained for pork meat per se. In addition, the on-farm pig odour problems will also be reduced substantially with great benefit to the public.

**Existing methods for boar taint control**

Methods for boar taint control comprise castration of male pigs and feeding with inulin and fructooligosaccharides.

**Castration of male pigs**

The phenomena of boar taint associated negative effects have been addressed in the state of the art. The most common preventive measure is to castrate male pigs either physically through removal of the testicles during the first week of the male pig's life, or chemically through immunovaccination (Bonneau and Carelli, 1987). Immunological castration of male pigs with a synthetic aqueous vaccine is possible (Dunshea et al., 2001). Immunization of pigs against gonadotrophin releasing factor (GnRF) prevents boar taint and affects boar growth and behaviour (Metz et al., 2002). Overall, today the costs of immunovaccination are prohibitively high. Furthermore, it is only allowed by authorities in a few countries (USA, Australia) due to animal welfare problems, and thus not a realistic alternative in other countries.

Physical castration is commonly carried out by the farmer without sedation or anaesthetics. The consequences of this include in some cases infections of the wounds with resulting costs for treating the higher level of infections in the stock. Moreover, physical castration is carried out rapidly and the efficiency is not always 100 %.

Castration reduces the boar taint problems of skatole and androstenone in the meat and fat, but it does not eliminate the negative effects. Furthermore, castration does not address the problem of elevated p-Cresol and skatole levels and live pig off-odour problems (stable and manure offensive-odour) found in all pig stables with especially fattening pigs.

It is expected that mass castration of piglets will be forbidden in the near future for reasons of animal welfare at least in the EU area. In Norway such castration is forbidden from 2009. In the interim period, authorised veterinarians can only perform castration. Castration by veterinarians makes the costs prohibitively high for industrial scale pig farming.

**Inulin and fructooligosaccharides (FOS)**

It is known that the production of skatole from tryptophan in the intestine can be reduced by feeding pigs with inulin (Claus, 1992; Claus 1994) and fructooligosaccharides, FOS, (see e.g. Jensen & Jensen, 1998; Knarreborg et al., 2002; Xu et al, 2002). However, to date a sufficient efficiency in reducing boar-taint remains to be demonstrated for these compounds. In for example Claus (1992), DE 42 23 051 it was demonstrated that the skatole content of backfat could be reduced only by 55% by feeding 140 kg pigs 2x35 g of inulin (from Dahlia tubers) daily.

**Live pig odour reduction**

The malodorous volatile compounds emitted from pig production units are an increasing problem in areas with intensive animal production. Several strategies for reduction of emission of odour have been tried e.g. (I) Biofilters, (II) Continuous aerobic treatment, (III) using oil and foam layers, (IV) additives to manure (e.g. acids), and (V) feed or change of feed composition. Although some improvement in ambient air quality has been obtained by these methods, none of them have found widespread use in practical conditions.

The solution for odour reduction should both be economically feasible and fit into the production systems without major investments. In addition the quality of the resulting meat product should remain at the same level, ideally with an increased product quality.

The most efficient solution would be to stop the production of malodorous compounds before the compounds end up in the manure, i.e. in the pig itself. This should be achieved with a suitable feed composition, which changes the spectrum of produced odorous compounds so the odour impression is changed to a less disagreeable composition. The need for investment in mechanical deodorising equipment in connection with the stable can therefore be omitted.

The odour active compounds originate from microbial degradation of residual feed components in the manure. The odour compounds can be divided in two groups depending on their origin: (I) compounds from fermentation of carbohydrates, and (II) compounds originating from fermentation of proteins. Degradation compounds from fermentable carbohydrates are usually short chain fatty acids (acetic acid, propionic acid, butyric acid and valeric acid) and short chain alcohols. The degradation products from proteins are a more complex mixture. They are branched short chain fatty acids (isobutyric acid), indoles (skatole and indole), phenols (p-cresol) and sulfur compounds (hydrogen sulfide, dimethyl disulfide). The compounds from the last group (protein fermentation products) have more disagreeable odours than the first group (carbohydrate fermentation products) and lower odour thresholds. This means they have a relatively higher negative impact on the air quality. The compounds produced can also be combined with each other e.g. volatile fatty acids can be combined with alcohols and result in esters which have other odour characteristics usually with less offensive odour notes. This process is facilitated by esterases, which can be produced by microorganisms.

The strategy for changing the composition of the odour active compounds (and thereby increase the air quality) would then be to increase the amount of less odour offensive compounds (from carbohydrate degradation) at the expense of the more odour active compounds (from protein degradation). If the odour active compounds also include synthesis of esters the odour quality would be further improved.

Accordingly there is need in the art for developing methods which are compatible with modern industrial scale farming for addressing the problems of taint in animals especially taint in male animals, primarily boar taint, including stable malodour, and meat taste.

**Control of parasite infections in pigs the state of the art**

Infections with intestinal parasites, including nematodes such as *Ascaris suum, Trichuris suis* and *Oesophagostomum dentatum,* are common throughout the world (Nansen & Roepstorff, 1999). The infections can cause significant economic losses to pig producers, as the nematodes may affect the overall growth rate and feed utilisation efficiency (e.g. Hale & Stewart, 1979; Hale & Marti, 1984; Hale et al., 1981, 1985; Stewart et al., 1985). In extreme cases the nematodes may also cause the death of infected animals (e.g. Jensen & Svensmark, 1996). This problem is particularly significant for the organic pig husbandry, as a goal of organic production is to minimise or entirely eliminate the use of medical drugs, including anthelmintics, and because nematode occurrence is generally increased in organic animals systems and other alternatives to industrial husbandry systems, as these generally offer better conditions for development and survival of infective parasite stages (deep litter systems, outdoor facilities), whereby the animals are much more exposed to infection (Thamsborg & Roepstorff, 2003). However, especially a parasite such as *A. suum* may also be found in indoor production systems (Roepstorf, 1997).

To reduce the problem there have been many alternative approaches towards new methods for nematode control, and one of the more promising and practical solutions is the manipulation of dietary composition. Previously published data has demonstrated that diets varying in carbohydrate source and in contents of insoluble fibre may influence nematode infection levels. Petkevič ius *et al.* (2003) found a markedly reduced excretion of parasite eggs and an almost complete elimination of *O. dentatum* from pigs fed a diet with added purified inulin (Raftiline®). Similar results have also been obtained for *T. suis* by Thomsen *et al.* (in preparation). Unfortunately, high contents of fibre and partially undegradable carbohydrates, as found in standard organic swine diets, seem to be favourable for the parasites, while parasite unfavourable diets composed of highly degradable carbohydrates are not normally fed to pigs (Bj∅rn et al., 1995; Petkevič ius *et al.,* 1997, 1999, 2001). Overall, novel feeding strategies that include continuous or periodical supplements of diets rich in fructooligosaccharides may contribute to future sustainable nematode control in pigs. It may be possible to identify organically relevant and economically competitive carbohydrate sources with high contents of fructooligosaccharides, on which the pigs grow well while reducing infection levels.

Though a promising product, purified inulin has up till now been an expensive product and therefore probably not likely to be used as a feed supplement in commercial pig production. Though the price may decrease with increased demand in the production units there is also the basis for an alternative product that can be produced at a competitive cost.

**Chicory root product**

US 4,971,815 (Tamatani et al) and US 4,865,852 (Tamatani et al) describe an additive for stock feeds containing decomposition products of chicory roots in which the total content of polysaccharides and inulooligosaccharides of tri- and higher saccharides obtained by decomposing the chicory roots is 40% by weight or more of the total solids content and is 80% by weight or more of the total saccharides. The stock feed preferably contains 0.1 to 10% by weight of the additive. The additive is prepared by a process which comprises the steps of chopping and then heating/drying chicory roots in order to form chicory flakes.

**Summary of invention**

The present invention relates to a method for reducing or removing off-odour and off-flavour in animals, said method comprising feeding to an entire male, castrate male and female animals a chicory root product during at least one day such as at least two days prior to slaughtering the animal. Preferably the animal is a domesticated animal, more preferable the animal is a pig.

Feeding animals with chicory root products reduces or removes boar taint in animals and improves the meat quality according to use of the meat as human food. The reduction of boar taint is also connected with reducing malodour in the environment of the live animals due to offensive-smelling compounds in the mixture of faeces and urine of the animals (liquid manure). A chicory root product may be a cheap product and the effect of the product is more effective and efficient in reducing such taints than feeding animals with compounds such as inulin isolated from chicory plants, thus an alternative product to pure inulin is chicory roots. Also the chicory root product has beneficial effects on the animals, effects which can not be obtained by pure inulin, one of these effects are effect on meat taste.

In another embodiment the invention relates to a method for reducing the skatole content in animals, said method comprising feeding to an animal a chicory root product for at least one day such as at least two days prior to slaughtering.

In a further embodiment the invention relates to a method for reducing the androstenone content in meat and fat and blood said method comprising feeding to an animal a chicory root product for at least one day such as at least two days.

Skatole and androstenone are two of the compounds resulting in boar taint of entire male pig meat, and are connected to off-odour and flavours of meat. Reducing skatole and androstenone content in meat also decreases the amount of animals being rejected at slaughter for use in meat cuts.

In yet another embodiment the invention relates to a method for improving the odour, flavour, taste and aftertaste of meat from a human sensory acceptability perspective, said method comprising feeding to an animal a chicory root product for at least one day such as at least two days prior to slaughter. The chicory root product has an effect on taste and aftertaste of meat, which can not be obtained by feeding animals with pure inulin.

In a further embodiment the invention relates to a method for reducing malodour as related to the live animals environment, said method comprising feeding a chicory root product to animals for at least one day such as at least two days. Reducing malodour compounds coming from pig stables and manure lead to environmental benefits in relation to the public.

In another embodiment the invention relates to a method for reducing the amount of infections with pathogens of the gastrointestinal tract in a non-human animal, said method comprising feeding to a non-human animal a chicory root product for at least one day such as at least two days.

The invention relates to animal welfare by a friendly, humane, low cost and highly effective feeding methodology when compared to all the presently utilised methods for boar taint control.

In another aspect the invention relates to the chicory root product it self comprising inulin and other low molecular sugars as well as secondary metabolites.

**Brief description of Figures**

Fig. 1. (a) The scores of odorous compounds of raw data from colon contents of control-fed and chicory-fed pigs. (b) The loadings of the odorous compounds of control-fed and chicory-fed pigs.

Fig. 2. (a) The scores of low threshold values of odorous compounds from colon contents of control-fed and chicory-fed pigs. (b) The loadings of low threshold values of the odorous compounds of control-fed and chicory-fed pigs.

Fig. 3. (a) The scores of high threshold values of odorous compounds from colon contents of control-fed and chicory-fed pigs. (b) The loadings of high threshold values of the odorous compounds of control-fed and chicory-fed pigs.

Fig. 4. Skatole in blood plasma of pigs due to short time feeding of dried chicory roots. The pigs were feed with 25% dried chicory roots plus 70% concentrate.

Fig. 5. Mean *O. dentatum* egg counts (eggs per gram faeces) in five groups of eight pigs fed different diets. The first 28 days after infection with 3000 *O. dentatum* L3-larvae all pigs were given concentrate and grass silage. Thereafter the concentrate control group was given only concentrate and the long-term chicory group had the silage substituted for shredded chicory roots. This was also done for the short-term chicory group 28 days before slaughter.

Fig. 6. Mean egg excretion (epg=eggs per gram faeces) of *O. dentatum* in 4 groups of pigs (n=8) fed different diets.

Fig. 7. Female *O. dentatum* with mating caps in four groups of pigs fed different diets. Ten females were examined per pig except for one pig in the dried chicory group where only five worms were recovered. The median percentages are illustrated by the solid lines.

Fig. 8. Principal Component Analysis (PCA) of sensory profiling data from freshly cooked entire male pork meat samples for each of four feeding treatments, 1). Non-Bioactive Control, 2). Silage, 3). Chicory, and 4). Chicory/Silage.

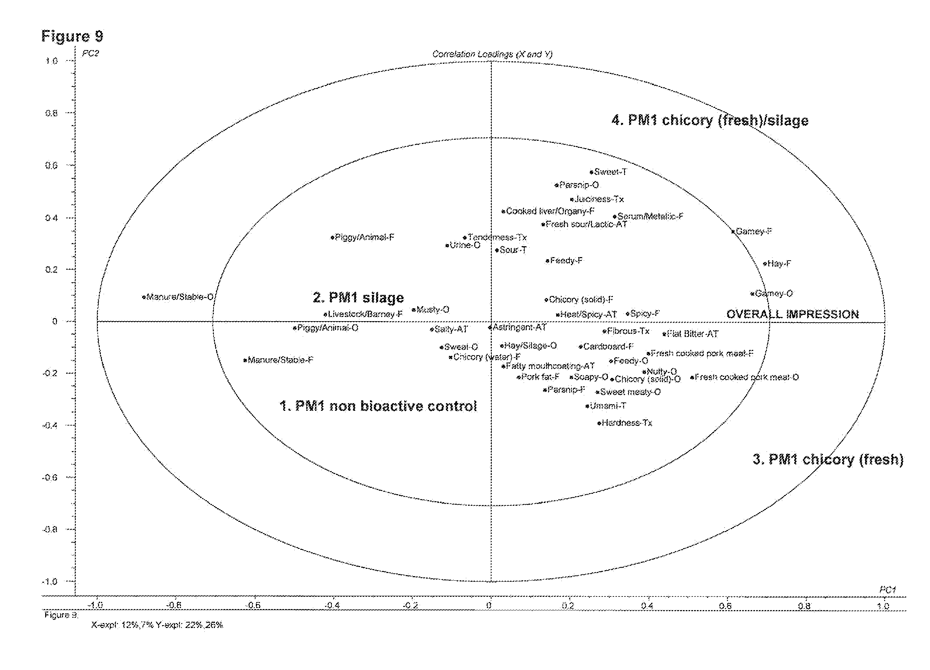

Fig. 9. Psoas Major 1 (PM1) (PC 1 v 2). Discriminant Partial Least Squares Regression (DPLSR) correlation loadings plot of sensory profiling variables (X-matrix) versus feeding treatment design variables (Y-matrix), displayed that the animals fed treatment 1. non bioactive control feed and 2. silage were high in *boar taint* descriptors such as e.g. *manure/stable odour/flavour, piggy/animal odour/flavour, musty odour, urine odour and livestock/barny flavour,* whereas animals fed chicory (treatments 3 and 4) were, relative to 1. non bioactive control feed and 2. silage treatments, not high in boar taint descriptors and were described by *fresh cooked pork meat odour/flavour* and thus, displayed a high overall impression. Ellipses represent $r^2$ = 50 and 100%.

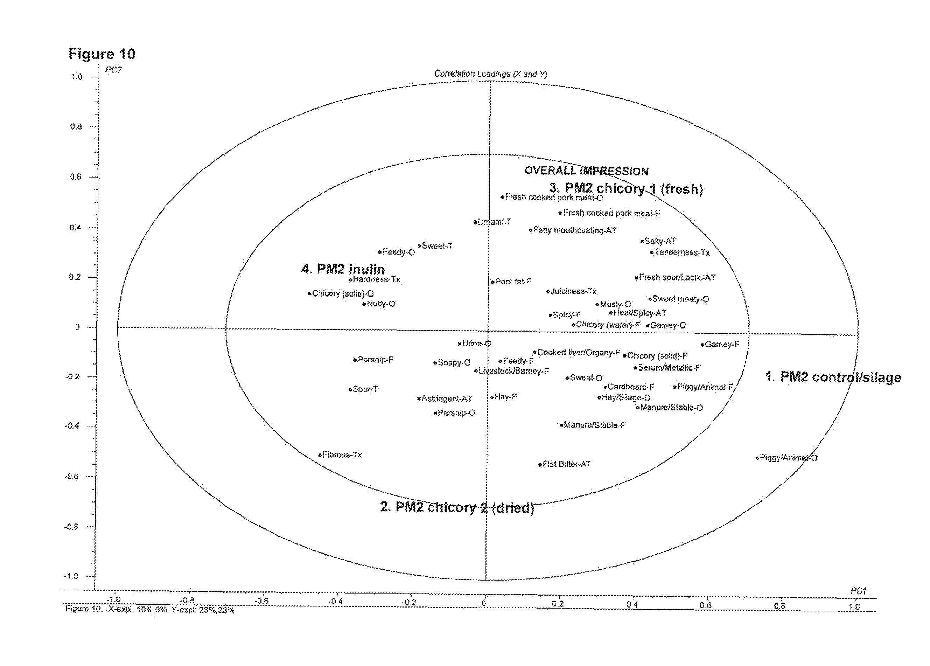

Fig. 10. Psoas Major 2 (PM2) (PC 1 v 2). Discriminant Partial Least Squares Regression (DPLSR) correlation loadings plot of sensory profiling variables (X-matrix) versus feeding treatment design variables (Y-matrix), displayed that the animals fed treatment 1. control/silage were high in boar taint terms such as e.g. *manure/stable odour/ flavour, Gamey-F, Flat bitter-AT* and *animal/piggy odour/flavour,* whereas animals fed 3. chicory 1 (fresh) were, relative to the 1. control/silage treatments, not high in boar taint descriptors and were described by fresh cooked pork meat *odour/flavour* and thus, and displayed a higher overall impression. Ellipses represent $r^2$ = 50 and 100 %.

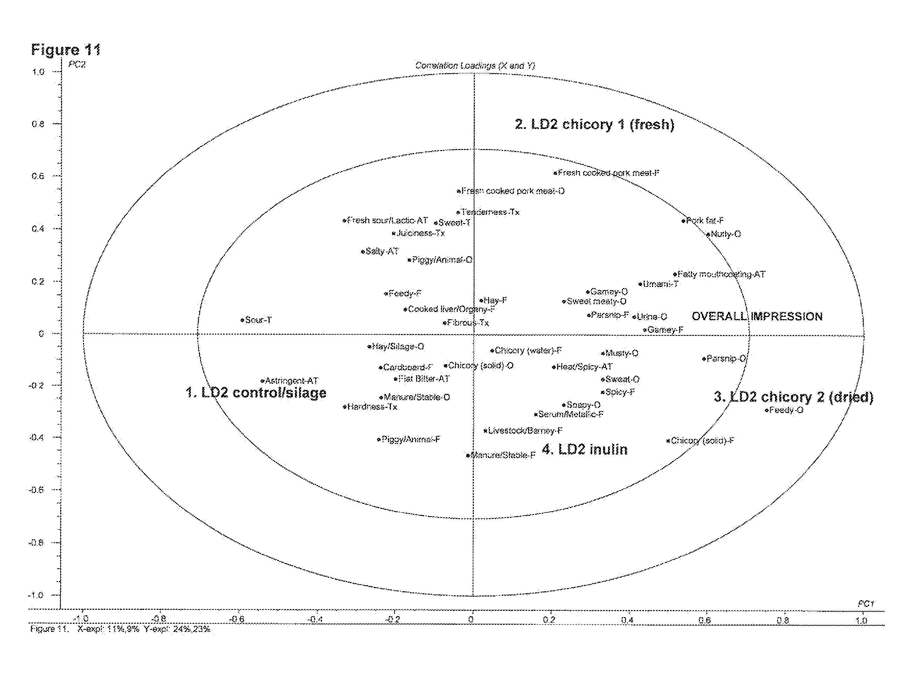

Fig. 11. Longissimus Dorsi 2 (LD2) (PC 1 v 2). Discriminant Partial Least Squares Regression (DPLSR) correlation loadings plot of sensory profiling variables (X-matrix) versus feeding treatment design variables (Y-matrix), displayed that the animals fed treatments 1. control/silage and 4. inulin were high in boar taint terms such as e.g. *manure/ stable odour/flavour, animal/piggy odour/flavour and livestock/barny flavour,* whereas animals fed 2. chicory 1 (fresh) and 3. chicory 2 (dried) were, relative to 1. control/silage and 4. inulin, not high in boar taint descriptors and were described by fresh cooked pork meat *odour/flavour* and thus, and displayed a higher overall impression. Moreover, treatments 2. chicory 1 (fresh) and 3. chicory 2 (dried) appeared to be similarly effective in reducing bore-taint. Ellipses represent $r^2$ = 50 and 100 %.

## Definitions:

A chicory root product: By a chicory root product is intended first and foremost the complete chicory roots. Also fractions of chicory root are included. Also encompassed by the present invention are processed products thereof, e.g. pulp, flakes, powder, flour, dried pulp, dried flakes, dried tubers, silage, enzymatically processed products, microbiologically processed products.

A chicory root extract: An extract made from chicory roots, wherein the extract comprises an inulin and/or FOS fraction as well as a low molecular weight fraction. Low molecular weight compounds are compounds below 2000 Dalton. Preferably the extract comprises the coumarins i.e. esculetin, sesquiterpenes, terpene, phytosterol, polyamine and flavonoid. More preferably the extract comprises low molecular weight sugars and terpenes.

A pig: An animal belonging to the group of animals characterised by the Latin name *Sus scrofa.*

Bitter chicory: By bitter chicory is to be understood chicory with a bitter taste. Bitter chicory need not be different from chicory or chicory root product.

Boar taint: is a distinctive and unacceptable taint perceived through a combination of sensory off-odour, flavour and taste in meat and meat products during cooking and eating, it is variously described as 'animal', 'urine', and/or 'manure' like in character

Domesticated animals: Examples of domesticated animals are cattle, sheep, goat, pig, horse, donkey, dog, cat, poultry, chicken, duck, goose, turkey, steer, mink.

Pigs can be classified according to age and partly according to weight. For the purposes of the present invention the following classification is used:

| | |
|---|---|
| Suckling piglet: | 0-4 weeks or until 7 weeks of age (until weaning) |
| Weaned pigs: | 4-8 weeks of age |
| Growing pigs: | above 8 weeks. |

Growing pigs are often referred to as Porkers (50-60 kg), finishers or fatteners (both up to 160 kg).

Chicory: By a Chicory plant is intended any species, subspecies or variety, which is a member of the Genus Cichorium L. belonging to the Compositae. Some botanists place the Cichorium family in the Asteraceae. Known species include at least: Cichorium alatum Hochst. & Steud.ex DC.

Cichorium ambiguum Schult.

Cichorium aposeris E.H.L.Krause

Cichorium arnoseris E.H.L.Krause

Cichorium balearicum Porta

Cichorium barbatum E.H.L.Krause

Cichorium bottae Deflers
Cichorium bottae Deflers
Cichorium byzantinum Clem.
Cichorium caeruleum Gilib.
Cichorium callosum Pomel
Cichorium calvum Sch.Bip.
Cichorium casnia C.B.Clarke
Cichorium cicorea Dum.
Cichorium commune Pall.
Cichorium cosnia Buch.-Ham.
Cichorium crispum Mill.
Cichorium dichotomum Link
Cichorium divaricatum Heldr.ex Nym.
Cichorium divaricatum Schousb
Cichorium dubium E.H.L.Krause
Cichorium endivia Linn.
Cichorium endivia subsp. divaricatum (Schousboe) P.D.Sell
Cichorium endivia subsp. pumilum (Jacq.) C.Jeffrey
Cichorium esculentum Salisb.
Cichorium glabratum Presl
Cichorium glandulosum Boiss. & Huet
Cichorium glaucum Hoffmgg. & Link
Cichorium hirsutum Gren.
Cichorium intybus convar. foliosum (Hegi) J.Holub
Cichorium intybus convar. radicosum (Alef.) J.Holub
Cichorium intybus forma alba Farw.
Cichorium intybus forma rubicunda Farw.
Cichorium intybus L.
Cichorium intybus Linn.
Cichorium intybus subsp. glabratum (C.Presl) G.Wagenitz & U.Bedarff
Cichorium minimum Portenschl.
Cichorium nanum Portenschl.ex Nym.
Cichorium noeanum Boiss.
Cichorium officinale Gueldenst.ex Ledeb.
Cichorium perenne Stokes
Cichorium polystachyum Pomel
Cichorium pumilum Jacq.
Cichorium rhagadiolus E.H.L.Krause
Cichorium rigidum Salisb.
Cichorium spinosum Linn.
Cichorium sylvestre Garsault
Cichorium sylvestre Lam.

A commonly used agricultural variety of Cichorium is: Cichorium intybus L. var. Orchies

Plant varieties of Cichorium for which Plant variety protection has been granted or is about to be granted at the Community Plant Variety Office, Angers, France

**Cichorium endivia L.**

| File Number | Application Date | Denomination | Grant Number | Grant Date | End of Protection |
|---|---|---|---|---|---|
| 19971402 | 28/11/1997 | ARIGA | 3152 | 02/06/1998 | 31/12/2023 |
| 19951973 | 25/08/1995 | ATRIA | 1635 | 15/01/1997 | 31/12/2022 |
| 19950129 | 31/05/1995 | BOLDIE | 1088 | 15/10/1996 | 01/10/2017 |
| 19970359 | 12/03/1997 | BOOGIE | 3047 | 06/07/1998 | 31/12/2023 |
| 19980139 | 23/01/1998 | CENTURY | 3605 | 19/10/1998 | 31/12/2023 |
| 19990460 | 22/03/1999 | EMILIE | 7833 | 11/06/2001 | 31/12/2026 |

(continued)

| File Number | Application Date | Denomination | Grant Number | Grant Date | End of Protection |
|---|---|---|---|---|---|
| 19950621 | 09/08/1995 | EXCEL | 1459 | 16/12/1996 | 31/12/2021 |
| 19970357 | 12/03/1997 | FOXIE | 3134 | 02/06/1998 | 31/12/2023 |
| 19971458 | 15/12/1997 | FREHEL | 5639 | 20/12/1999 | 31/12/2024 |
| 20001725 | 21/11/2000 | g | 9370 | 06/05/2002 | 31/12/2027 |
| 20001830 | 06/12/2000 | ISADORA | 7998 | 06/08/2001 | 31/12/2026 |
| 20001831 | 06/12/2000 | ISOLA | 7999 | 06/08/2001 | 31/12/2026 |
| 19990309 | 01/03/1999 | KETHEL | 7446 | 09/04/2001 | 31/12/2026 |
| 19991249 | 08/09/1999 | KIBRIS | 10192 | 21/10/2002 | 31/12/2027 |
| 20000439 | 23/03/2000 | LASSIE | 8505 | 03/12/2001 | 31/12/2026 |
| 20000809 | 31/05/2000 | LILIE | 7390 | 05/03/2001 | 31/12/2026 |
| 19950622 | 09/08/1995 | MISTRAL | 1460 | 16/12/1996 | 31/12/2021 |
| 19991604 | 15/11/1999 | MONTREAL | 8500 | 03/12/2001 | 31/12/2026 |
| 19950623 | 09/08/1995 | NAOMI | 1461 | 16/12/1996 | 31/12/2021 |
| 19951225 | 29/08/1995 | NATACHA | 1089 | 15/10/1996 | 01/02/2018 |
| 19950294 | 27/04/1995 | NUANCE | 975 | 02/09/1996 | 01/09/2016 |
| 20001829 | 06/12/2000 | OLIVIA | 7997 | 06/08/2001 | 31/12/2026 |
| 19951972 | 25/08/1995 | PRADA | 1634 | 15/01/1997 | 01/10/2027 |
| 19971403 | 28/11/1997 | SACHA | 3151 | 02/06/1998 | 31/12/2023 |
| 19950258 | 06/07/1995 | SARDANA | 1942 | 15/05/1997 | 01/06/2017 |
| 19981452 | 29/10/1998 | SNOOPIE | 5566 | 06/12/1999 | 31/12/2024 |
| 19991208 | 27/08/1999 | STOMIE | 5801 | 14/02/2000 | 31/12/2025 |
| 19970360 | 12/03/1997 | TRUDIE | 3048 | 06/07/1998 | 31/12/2023 |
| 19970358 | 12/03/1997 | WOODIE | 3132 | 02/06/1998 | 31/12/2023 |

**Cichorium endivia L.**

| File Number | Application Date | Breeder's Reference | Proposed Denomination |
|---|---|---|---|
| 2001/0741 | 25/04/2001 | e 02 2216 | ATLETA |
| 2000/1908 | 10/01/2001 | bejo 1978 | CARLOS |
| 2002/1355 | 30/10/2002 | 11-122 rz | CASAL |
| 2000/1911 | 10/01/2001 | bejo 1895 | DAVOS |
| 2002/1356 | 30/10/2002 | 11-510 rz | LASKO |
| 2000/1907 | 10/01/2001 | bejo 1979 | LEXOS |
| 2002/1354 | 03/09/2002 | 11-194 rz | MARCONI |
| 2000/1910 | 10/01/2001 | bejo 1894 | MONOS |

**Cichorium intybus L. partim**

| File Number | Application Date | Denomination | Number | Grant Date | End of Protection |
|---|---|---|---|---|---|
| 19980340 | 11/03/1998 | AUG133 | 7466 | 09/04/2001 | 31/12/2026 |
| 19980339 | 11/03/1998 | CES4731 | 7465 | 09/04/2001 | 31/12/2026 |
| 19970157 | 28/01/1997 | CPZ 4641 | 6897 | 20/11/2000 | 31/12/2025 |
| 19970166 | 28/01/1997 | CPZ 6722 | 6898 | 20/11/2000 | 31/12/2025 |
| 19952585 | 24/08/1995 | CRP 308-2 | 1464 | 16/12/1996 | 31/12/2021 |
| 1952584 | 24/08/1995 | CRP 609-3 | 1463 | 6/12/1996 | 31/12/2021 |
| 19980990 | 17/07/1998 | FASTE | 7472 | 09/04/2001 | 31/12/2026 |
| 19960978 | 03/09/1996 | FLA A1-1 | 2444 | 01/09/1997 | 31/12/2022 |
| 19960977 | 03/09/1996 | FRAN B1-2 | 2443 | 01/09/1997 | 31/12/2022 |
| 19980991 | 17/07/1998 | OESIA | 5844 | 03/04/2000 | 31/12/2025 |
| 19970156 | 28/01/1997 | SISTA153 | 6896 | 20/11/2000 | 31/12/2025 |

**Cichorium intybus L. partim**

| File Number | Application Date | Breeder's Reference | Proposed Denomination |
|---|---|---|---|
| 2001/1199 | 19/07/2001 | bejo 2202 | FOX 14 |
| 2000/1913 | 10/01/2001 | bejo 2196 | FURB 21 |
| 2000/1909 | 10/01/2001 | bejo 2197 | NER261 |
| 2002/0434 | 19/03/2002 | nun 9001 cm | NUN9001CM |
| 2002/0435 | 19/03/2002 | nun 9002 cm | NUN9002CM |
| 2002/0436 | 19/03/2002 | nun 9003 cm | NUN9003CM |
| 2002/0437 | 19/03/2002 | nun 9004 cm | NUN9004CM |
| 2002/0438 | 19/03/2002 | nun 9005 cm | NUN9005CM |
| 2002/0439 | 19/03/2002 | nun 9006 cm | NUN9006CM |
| 2002/0440 | 19/03/2002 | nun 9007 cm | NUN9007CM |
| 2000/0303 | 28/02/2000 | e 84.025 | REDORIA |
| 2000/1914 | 10/01/2001 | bejo 2195 | SISTA 159 |
| 2001/0740 | 25/04/2001 | wo118 | WO 118 |
| 1999/08/19 | 08/06/1999 | wo 125 | WO125 |
| 1999/0818 | 08/06/1999 | wo 126 | WO126 |

**Detailed description of the invention**

The present invention relates to a method for reducing taint in animals, said method comprising feeding to an animal a chicory root product during at least one day such as at least two days prior to slaughtering the animal. The taint is connected to malodour in places where animals are living especially in indoor locations e.g. in stables, other houses or hiding-places for pigs. The taint is also connected to off-odour and flavour in meat from a human sensory perspective.

By using the wording 'reducing taint in animals' it is not only meant to limit the reduction of taint to the inside of the animals e.g. in all food related items contained in the animal in particular the meat, also the stables and outdoor areas where animals are living are intended to be included as well as the manure/slurry kept in tanks and spread on the soil. In general the reduction of taint in the environment of animals is included.

Feeding the animals with the chicory root product reduces taint in animals, males as well as females. Surprisingly the effect of the chicory root product on skatole in backfat is higher than expected when comparing to results of an experiment using purified inulin (Claus, 1992 & 1994).

Feeding male and female animals with chicory root product reduces the off odour and off flavour of the meat and hereby increases the human sensory enjoyment in eating the untainted meat. The reduction of off odour and off flavour also reduces the amount of animals that are being degraded as boar tainted meat discharged due to unsuitability to be used directly as a human food.

Furthermore, castration of the animals can be avoided, which increase the animal welfare due to avoiding the pain male animals are subjected to at the time of castration. The chicory root product is a cheap alternative to castration especially in countries where authorised veterinarians perform the castration.

Feeding the animals with the chicory root product reduces infections with intestinal pathogens such as parasites.

**Feeding time**

The chicory root product can be produced from plants of one or more of the species, genus or plant families mentioned above. This chicory root product is fed to the animal for at least 1 day, such as at least 2 days, such as at least 3 days, such as at least 4 days, such as at least 5 days, such as at least 6 days, such as at least one week, for example at least 1.5 weeks, such as at least 2 weeks, preferably at least 3 weeks, such as at least 4 weeks, for example at least 5 weeks, such as at least 6 weeks, for example at least 7 weeks, such as at least 8 weeks, for example at least 9 weeks, such as at least 10 weeks, for example at least 15 weeks, such as at least 20 weeks.

Feeding animals with the chicory root product within a short period just prior to slaughtering reduces the amount of chicory root product to be used, simultaneously with reducing taint of the meat of the animal. Feeding animals with the chicory root product within a long period prior to slaughtering generally reduces taint of the meat and malodour of the stables or living place of the animal as well as the parasite load.

Strategic feeding with the chicory root product such as a dried chicory during specific warm summer time periods or within other periods reduces the amount of chicory root product to be used compared to continuously feeding the whole year. Feeding in periods reduces boar taint of meat simultaneously with reducing the most severe malodour of the stables or living place of the animal of all ages.

To reduce taint in the animal the chicory root product is fed to the animal substantially until slaughter. The initiation of the feeding by the chicory root product can be at any time during the life of the animal. The amount of chicory root product per kg animal eaten by said animal may vary during the life of the animal or during the year due to the need of the chicory root product or due to fluctuation in the quality of the feed, where the quality can be of the chicory plants or the other feed components.

In the period where the animal is fed by the chicory root product as outlined elsewhere, the chicory root product is fed to the animal daily. The frequency of daily feeding may vary from one portion which is eaten up by the animal within a short period or the animal can have admission to the chicory root product all day long, preferred is that the chicory root product is fed to the animal several times daily, such as 2 times, 3 times, 4 times, 5 times, or more than 5 times. Also preferred is feeding by a dried chicory root product once a day.

The chicory root product can be fed to the animals every day, every second day, every third day, every fourth day, every fifth day, every sixth day or once a week. Preferred is when the animals are fed by the chicory product every day.

Preferred is when the animals are fed by the chicory product every day within a period of 2 to 3 weeks before slaughter. Further preferred is when this chicory product is a dried chicory product as described elsewhere herein. Yet further preferred is when the animals are fed by a dried chicory product every day within a period of 3 weeks before slaughter.

The animals may also be allowed to crop an area with growing chicory plants, hereby the animals can eat the leaves of the plants and/or eat the roots by first digging up or drawing up the plants and/or roots.

The animals may also crop an area where chicory plants are harvested. The animals can eat the remaining chicory plants or the remaining chicory plant parts. Remaining plant parts can be due to topping the plants when harvesting, removing the roots and leaving the leaf part on the area. Also non-removed roots can be eaten by the animal.

**Feed ration**

The chicory root product can constitute a part of the daily feed ration, preferably the chicory root product part of the ration of the animal is at least 2.5 % on a daily energy basis. Also preferred is when the chicory root product part of the ration of the animal is at least 5% on a daily energy basis. Also preferred is when the chicory root product part of the ration of the animal is at least 10% on a daily energy basis.

Further, when feeding with the chicory root product the ration based on a daily energy basis can be that the chicory root product part comprises at least 15 % of the ration, more preferably at least 20%, more preferably at least 25%, more preferably at least 30 %, for example at least 35%, such as at least 40%, for example at least 50%, such as at least 60%, for example at least 75%, such as at least 90%, for example substantially 100%.

The chicory root product does not seem to result in a reduction in the growth rate of the animals; furthermore the animals do not show signs of avoiding eating the chicory root product.

The chicory root product is a low protein or substantially protein-free product. Surprisingly, when the animals are fed with the chicory root product, the animals need not be fed with an additional protein supplying product to obtain the weight of an animal fed by ordinary feeding products.

**Animals**

In one embodiment the animal as described herein may be any higher animals at any stage of life, preferable the animals is domesticated animals and more preferred is when the animal is a ruminant, such as cow, sheep, goat, buffalo, deer, cattle, antelope.

In another preferred embodiment the animal is a monogastric species, such as horse, pig, poultry, dog, cat. Further preferred is when the monogastric species is a pig. Yet further preferred is when the pig is an entire male pig.

Piglets can eat the chicory root product from none, one or several days before weaning from the sow as a part of the ration as described elsewhere. Preferred is feeding pigs with the chicory root product wherein weight of the pig is from 25 to 300 kg, more preferably as from 55 to 130 kg, which is the weight of fatteners at slaughtering. Pigs of all ages can be feed with the chicory root product such as to suckling piglet of 0-4 weeks of age (or until weaning), weaned pigs of 4-8 weeks of age, growing pigs above 8 weeks for instance is growing pigs often referred to as porkers (50-60 kg), finishers or fatteners (both up to 130 kg). The pigs can be feed with the chicory root product when the pigs are ranging in weight from 4 to 350 kg, such as 5 to 150 kg, such as 5 to 170 kg, e.g. such as 5 to 30 kg, further such as 30 to 50 kg, such as 50 to 80 kg, such as 80 to 110 kg, such as 110 to 140 kg, such as 140 to 170 kg, such as 170 to 200 kg, such as 200 to 275 kg, such as 275 to 350 kg.

The animals fed by the chicory root product of the invention may live in organic or non-organic production systems. The animals may be in a stable all day or have access to outdoor equipment such as a fence or live in an outdoor area.

**Chicory plants**

The chicory root product described herein can be prepared from plants of the family *Compositae,* the chicory root product can be produced from plants of one or more genus of the family *Compositae,* preferred is plants from the genus *Cichorium.* In this context chicory is used to describe plants belonging to the genus *Cichorium.* As just mentioned the plants may belong to a single or more genus of family *Compositae* as well as from a single or more species of the genus *Cichorium,* as well as from a single or more varieties of the species *Cichorium intybus L.* Preferably the plants are of the species *Cichorium intybus L.* The genera and species referred to are that mentioned previously. The varieties are any chicory variety, which are being cultivated at a time. Preferred are plants of agricultural varieties. More preferred are plants with large roots, most preferred are varieties with a high biomass yield by area e.g. 60 ton per ha. Further preferred are varieties with a large inulin content, such as at least 15% inulin on a dry matter basis, e.g. at least 20% inulin, such as at least 30% inulin, e.g. at least 40% inulin, such as at least 50% inulin, for instance at least 60% inulin, such as at least 70% inulin, for instance at least 80% inulin, such as at least 90% inulin, for instance at least 95% inulin.

Chicory plants are easy to grow and many agriculture varieties have a high yield, hereby the chicory root product becomes a cheap product. Furthermore the chicory plants can be handled by equipment used in sugar beet production.

All parts of the chicory plant can be used to prepare a chicory root product; the phrase 'chicory root product' is used to indicate that preferably the main part of the product is prepared from the roots of the chicory plants. This root part of the amount of chicory plant used to produce the chicory root product may constitute more than 20% of the total dry weight of chicory plant used, such as more than 30%, such as more than 40%, such as more than 50%, such as more than 60%, such as more than 70%, such as more than 80%, such as more than 90%, such as substantially 100%.

In the description of the chicory roots e.g. characterisation of the contents of compounds these characteristics may be valid for portions including entire plants or portions only including roots and small parts of the leafs.

The chicory root product is prepared from chicory plants, wherein the chicory roots contain at least 5% inulin, more preferably at least 10% inulin, more preferably at least 15 % inulin, more preferably at least 20 % inulin, such as at least 25% inulin, for example at least 30 % inulin on wet weight basis of the root.

The chicory root product is prepared from chicory plants, wherein the chicory roots contain at least 5% FOS, more preferably at least 10% FOS, more preferably at least 15 % FOS, more preferably at least 20 % FOS, such as at least 25% FOS, for example at least 30 % FOS on wet weight basis of the root. FOS is fructooligosaccharides.

## Processed chicory root products

The chicory root product used according to this invention can be a processed chicory root product comprising a silage product of chicory roots, such as a silage product of essentially whole chicory roots.

## Silage

Silage is prepared by anaerobic fermentation this can be in a pit, silo or other enclosure or by chemical preservation e.g. by lactic acid, propionic acid, and formaldehyde. The chicory plant parts or chicory roots can be ensiled alone meaning without other plant species or it can be ensiled together with different plant species of forage crops such as ryegrass, maize, sorghum, alfalfa, potatoes, beets e.g. sugar beets and sugar beet pulp/refuse. The plant material is harvested green and stored as fresh material, enclosed in air-proof conditions (pit, or under a plastic or similar covering) and allowed to ferment, with most of the soluble sugars converted to low molecular weight volatile fatty acids, such as acetic acid. Various additives may be used, either to increase the concentration of fermentable carbohydrate (molasses), to increase the proportion of beneficial bacteria e.g. lactic acid in the ensiled material, or to artificially lower the pH of the mixture. Additional fermentable carbohydrate may be added as molasses. Alternatively, enzymes such as xylanases and cellulases may be added to release low molecular weight fermentable substrates from the cell wall polysaccharides. Synthetic volatile fatty acids e.g. propionic acid may also be added to lower pH.

The chicory root silage can also be produced by mixtures of chicory plants and straw or by adding pellets from sugar beet pulp to the chicory plants. Other dried products can also be used e.g. potato starch.

The silage can constitute the chicory root product or the chicory root product can be produced from silage and other chicory products described herein.

## Fermented product

The chicory root product of the invention can be a product, wherein the chicory root product comprises a fermented product of chicory roots. The fermentation can be initiated with fractions of fresh roots, fractions of dried roots and extracts.

A fermented chicory root product can be obtained by fermentation with bacteria such as *Bacillus, Acetobacter,* etc also yeast can be used to the fermentation process. Preferred is fermentation with *Lactobacillus casei alactosus, Lactobacillus cellobiosus, Leuconostoc destranicum, Leuconostoc mesenteroids , Streptococcus lactis, Streptococcus diacelylactis,* or *Saccharomyces florentinus.* Methods of fermentation of chicory roots are described in US 4,671,962.

## Decomposition of chicory roots

Heating and/or drying chopped chicory roots may carry out decomposition of the chicory roots. Furthermore, the decomposition may be performed by first chopping and grinding the chicory roots into fine pieces, then preparing a slurry of the pieces, and enzymatically decomposing the slurry; or alternatively by first chopping the chicory roots into fine pieces, then heating and drying them, adding thereto water to form a slurry, and enzymatically decomposing the slurry.

If necessary, a further treatment may be conducted by the use of pectinase and/or cellulase. Afterward, an endo-type inulase is added to the slurry, and the enzymatic decomposition is then performed at a temperature of 40°C to 80°C for 12 to 36 hours. Preferred is a product in which 50 weight % or more of the solids content comprises the fructooligosaccharide (FOS).

Usable examples of the endo-type insulase include enzymes produced by mold fungi such as those of the genus *Aspergillus* (*A. niger* and the like) and those of the genus *Penicillium* (*P. trzebinskii* and the like), and bacteria such as *Bacillus* (*B. circulans* and the like). In a preferable case, the endo-type inulase wherein the optimum temperature is from 30°C to 80°C and the optimum pH is from 4 to 7 is used, so that the oligosaccharide is effectively produced from the chicory flakes. In the practical enzyme decomposition, it is preferable that the temperature of the enzymatic decomposition is high for the sake of preventing contamination with various bacteria. Therefore, the enzymatic decomposition is suitably performed at a temperature of 40° to 80°C. Enzymatic preparation is further described in US 4,971,815.

The chicory root product of the invention can be a product, wherein the chicory root product comprises flour of chicory root. This invention therefore provides a process for the preparation of a flour from tubers of chicory or similar inulin-containing plants, which process comprises the steps of: (a) macerating the tubers to a homogenate; (b) heating the homogenate at a temperature ranging from about 150°C to about 90°C for a time ranging, respectively from about 15 seconds to about 10 minutes; (c) subjecting the heated homogenate to spray drying in a stream of hot gas; and (d) recovering a flour comprising a mixture of monosaccharides, small oligosaccharides and large oligosaccharides. Flour production is further described in US 4,871,574.

The chicory root product of the invention can be a product, wherein the chicory root product comprises pulp of chicory root. Suitable pulps include those where some of the inulin has been removed (extracted) to leave a chicory pulp. The present invention includes all chicory pulp, which can be obtained from chicory plants, including the whole range of possible fibre and inulin content. The pulp is preferably obtained from at least chicory root material. The chicory pulp may be incorporated into a chicory root product with the same composition as directly produced from the extraction procedure. Alternatively, the pulp may undergo one or more steps to obtain a pulp of a different composition and/or form. For example, the pulp may be dried and then ground up to provide a dry product of small particle size, which may be used to produce a chicory root product.

**Dried chicory roots**

The chicory root product of the invention can be a product, wherein the chicory root product comprises a dried product of chicory roots, such as a dried product of essentially whole chicory roots. The chicory roots or disintegrated chicory roots can be dried by any drying method, such as sun dried, dried by heat, dried by air, dried by heated air, dried in a heating chamber or freeze dried. Drying processes may be those used when drying beets or other drying processes know to the person skilled in the art.

Drum drying can be applied to the disintegrated chicory roots. Drum dryers are often used for drying wet materials e.g. green chopped alfalfa and grass. Drum drying is a continuous drying process. What differentiates this drying method from other drying methods are in particular a very high drying air temperature and a short duration of the treatment. The inside of the drum is fitted with flights that by rotation of the drum lift the material and shower it down through the drying air. The motion of material and drying air is concurrent. Temperatures as high as 800°C may be used. The material does not reach air temperatures due to evaporative cooling. The capacity of a drum dryer depends on rate of airflow, rate of material, moisture content and drying air temperature.

The drying conditions when drum drying disintegrated chicory roots has to be adapted to the size and water content of the chicory roots. If it is difficult to obtain a sufficient low moisture content of the chicory roots the chicory roots can be dried several times (2-5 times) in the drum dryer, interrupted by a cooling period within or outside of the drum dryer.

The drying air temperature is of outmost importance as the material temperature may not exceed a level where the compounds of importance (e.g. inulin and other saccharides) will be decomposed. In a drum dryer one preferred combination of treatment conditions are a temperature of about 300°C and a treatment time of about 5-10 minutes. This will resulted in a maximum material temperature of about 65°C.

The temperature of the drum dryer or other drying means may be between 50°C to 800°C, the low temperature resulting in long term treatment (hours to days) of the entire chicory roots or disintegrated chicory roots (everything from flour to sections up to 20 cm in one direction) and the high temperatures resulting in short term treatment (seconds to hours).

Preferred pieces of chicory roots are sliced sections of the roots. It is not to be expected the slices can be perpendicular to the main direction of the chicory root. The slices may be anything between e.g. 0.5 and 10 cm in each dimension but smaller as well as larger dimensions may occur, preferred is between 1 and 8 cm in each dimension, more preferred is between 1.5 and 5 cm in each dimension. The main part of the of the chicory root pieces to be dried may have dimensions between about 1 and 2 cm in each dimension, about 2 and 3 cm in each dimension, about 3 and 4 cm in each dimension, about 5 and 6 cm in each dimension, about 2 and 5 cm in each dimension, about 2 and 8 cm in each dimension, about 3 and 5 cm in each dimension, about 3 and 8 cm in each dimension.

The temperature of the drum dryer or other drying means may be between about 50 °C to 800 °C as mentioned above, such as between about 50°C to 800°C, between about 100°C to 200 °C, between about 200°C to 300 °C, between about 300°C to 400 °C, between about 400°C to 500 °C, between about 500°C to 600 °C, between about 600°C to 700 °C, between about 700°C to 800 °C.

The temperature of the drum dryer or other drying means may be at least about 50°C, at least about 100°C, at least about 150°C, at least about 200°C, at least about 250°C, at least about 300°C, at least about 350°C, at least about 400°C, at least about 450°C, at least about 500°C, at least about 550°C, at least about 600°C, at least about 650°C, at least about 700°C, at least about 750°C, at least about 800°C.

The temperature of the drum dryer or other drying means may be less than about 900°C, less than about 850°C, be less than about 800°C, less than about 750°C, be less than about 700°C, less than about 650°C, be less than about 600°C, less than about 550°C, be less than about 500°C, less than about 450°C, be less than about 400°C, less than about 350°C, be less than about 300°C, less than about 250°C, be less than about 200°C, less than about 150°C, be less than about 100°C, less than about 50°C.

The temperature of the drum dryer or other drying means and the time of treatment must not result in a maximum material temperature that decompose the compounds having the effect as described elsewhere herein, the material temperature may be less than about 95°C, such as less than about 90°C, such as less than about 85°C, such as less than about 80°C, such as less than about 75°C, such as less than about 70°C, such as less than about 65°C, such as less than about 60°C, such as less than about 55°C, such as less than about 50°C, such as less than about 45°C, such as less than about 40°C, such as less than about 35°C.

The treatment time in the drum dryer or other drying means is determined due to the size of the chicory root pieces, the water content to be obtained, the temperature of the drum dryer and the maximum material temperature of the chicory root pieces. The treatment time may be about 1 min, such as about 2 min, such as about 3 min, such as about 4 min, such as about 5 min, such as about 6 min, such as about 7 min, such as about 8 min, such as about 9 min, such as about 10 min, such as about 15 min, such as about 20 min, such as about 25 min, such as about 30 min, such as about 40 min, such as about 50 min, such as about 60 min, such as about 70 min, such as about 80 min, such as about 90 min, such as about 2 hours, such as about 3 hours, such as about 4 hours, such as about 5 hours, such as about 6 hours, such as about 7 hours, such as about 8 hours, such as about 9 hours, such as about 10 hours, such as about 12 hours, such as about 14 hours, such as about 16 hours, such as about 18 hours, such as about 20 hours, such as about 22 hours, such as about 24 hours, such as about 26 hours, such as about 28 hours, such as about 30 hours, such as about 32 hours, such as about 34 hours.

The chicory root product of the invention can be a product, wherein the chicory root product is a disintegrated product, such as a powder, flakes, pulp, slices, flour, and pellets. The chicory root product can be disintegrated before a possible production process or the processed chicory root product can be disintegrated at a stage within the processing steps or followed processing. One example of drying of chopped chicory roots is at 60°C for 3 days in a heating chamber, which results in 3-4 % water content. The chicory roots can be homogenised, cut into strips, planed or disintegrated in other ways.

The chicory root product of the invention can be a product, wherein the chicory root product comprises fresh chicory roots. By fresh is meant a period of time from the chicory plants has been harvested to some months of storage such as 1 month, e.g. 2 months, such as 3 months, such as 4 months, such as 5 months such as 6 months, such as 7 months, such as 8 months, such as 9 months, such as 10 months, such as 11 months, such as 12 months. At the storage period the chicory roots can be stored at options where the roots do not ensilage, and/or ferment and/or dry. Some of the roots within the storage pile can locally ensilage, ferment or dry, which is accepted. One storage option is to collect the chicory roots in heaps or piles at conditions preventing silage formation, fermentation or drying. A certain degree of drying is acceptable, such as loss of 50% of the water content of the freshly harvested chicory roots.

### Fractions of chicory roots

The chicory root product of the invention can be a product, wherein the chicory root product comprises a fraction and/or an extract of chicory roots. The fraction of the chicory root product comprises inulin and oligofructose and at least one other compound from the chicory roots.

As mentioned elsewhere the chicory root product need not only to be produced from chicory roots or parts of chicory plants. To produce a chicory root product fraction and/or extract of chicory roots can be added to other feeding components.

Extract can be produced by extraction of compounds in an aqueous mixture of disintegrated chicory roots and a liquid or in a mixture of different liquids. The disintegrated chicory roots are described above.

The fraction and/or extract of chicory root preferably comprise inulin and oligofructose fractions and a low molecular weight fraction comprising coumarins and/or sesquiterpenes. The fraction and/or extract of chicory root can also comprise other secondary metabolites as mentioned below.

### Secondary metabolites

Secondary metabolites are compounds which are not a part of the primary metabolism of the organism e.g. they are not amino acids, carbohydrates, lipids and nucleic acids. The secondary metabolites in chicory can be divided in several chemical classes: terpenes, phytosterols, polyamines, coumarins and flavonoids. The content of secondary metabolites in a plant can vary according to season, growth conditions, variety, anatomical part of the plant, age of the plant and degree of attack of insects, herbivores or plant diseases e.g. bacteria or fungi.

Preferred secondary metabolites of chicory root fractions of the invention are selected from the groups mentioned in the following paragraphs:

Terpenes: *Sesquiterpene lactones:* 8-Deoxylactucin, crepidiaside, lactucin, lactupicrin, crepidraside, 11-β-13-dihydrolactucin, picriside, sonchuside A, sonchuside C, cichoriolide A, cichoriosides A, cichorioside B, cichorioside C and lactucopicrin.

*Phytosterols:* Sitosterol, stigmasterol, and campersterol.

Coumarines: Esculetin (=aesculetin), esculin (the glucon of esculetin), cichoriin-6'-p-hydroxyphenyl acetate and cichoriin.

Flavonoids: Luteolin 7-glucuronide, quercetin 3-galactoside, quercetin 3-glucuronide, kaempferol 3-glucoside, kaempferol 3-glucuronide, isorhamnetin 3-glucuronide.

*Anthocyanins:* Cyanidin 3-O-β-(6-o-malonyl)-D-glucopyranoside and four delphinidin derivatives.

Caffeic acid derivatives: Caffeic acid, chicoric acid, and chlorogenic acid.

Polyamines (biogenic amines): Putrescine, spermidine, spermine.

More preferred is secondary metabolites selected from the groups of terpenes, coumarines and caffeic acid derivatives. The most preferred secondary metabolites from these groups comprise:

Terpenes: *Sesquiterpene lactones:* 8-Deoxylactucin, crepidiaside, lactucin, lactupicrin, crepidraside, 11-β-13-dihydrolactucin, picriside, sonchuside A, sonchuside C, cichoriolide A, cichoriosides A, cichorioside B and cichorioside C.

Coumarines: Cichoriin-6'-p-hydroxyphenyl acetate, Esculetin (=aesculetin), and esculin.

Caffeic acid derivatives: Caffeic acid, and chicoric acid.

The content of 8-Deoxylactucin in the chicory food product may be at least 0.02 % of the dry weight, more preferred at least 0.04 %, further preferred at least 0.06 %, most preferred at least 0.08 %.

The content of Lactupicrin in the chicory food product may be at least 0.05 % of the dry weight, more preferred at least 0.07 %, further preferred at least 0.09 %, most preferred at least 0.11 %.

The content of Lactucin in the chicory food product may be at least 0.01 % of the dry weight, more preferred at least 0.03 %, further preferred at least 0.05 %, most preferred at least 0.07 %.

The content of Crepidiaside in the chicory food product may be at least 0.01 % of the dry weight, more preferred at least 0.03 %, further preferred at least 0.05 %, most preferred at least 0.07 %.

The content of Lactucopicrin in the chicory food product may be at least 0.01 % of the dry weight, more preferred at least 0.03 %, further preferred at least 0.05 %, most preferred at least 0.07 %.

The content of 11-β-13-Dihydrolactucin in the chicory food product may be at least 0.005 % of the dry weight, more preferred at least 0.007 %, further preferred at least 0.009 %, most preferred at least 0.011 %.

The content of Picriside B in the chicory food product may be at least least 0.01 % of the dry weight, more preferred at least 0.03 %, further preferred at least 0.05 %, most preferred at least 0.07 %.

The content of Sonchuside A in the chicory food product may be at least 0.008 % of the dry weight, more preferred at least 0.01 %, further preferred at least 0.015 %, most preferred at least 0.02 %.

The content of Cichoriolide A in the chicory food product may be at least 0.001 % of the dry weight, more preferred at least 0.003 %, further preferred at least 0.005 %, most preferred at least 0.007 %.

The content of Cichorioside A in the chicory food product may be at least 0.005 % of the dry weight, more preferred at least 0.007 %, further preferred at least 0.009 %, most preferred at least 0.011 %.

The content of Sonchuside C in the chicory food product may be at least 0.01 % of the dry weight, more preferred at least 0.03 %, further preferred at least 0.05 %, most preferred at least 0.07 %.

The content of Cichorioside B in the chicory food product may be at least 0.02 % of the dry weight, more preferred at least 0.04 %, further preferred at least 0.06 %, most preferred at least 0.08 %.

The content of Cichorioside C in the chicory food product may be at least 0.02 % of the dry weight, more preferred at least 0.04 %, further preferred at least 0.06 %, most preferred at least 0.08 %.

In an embodiment the chicory root product may contain two or more secondary metabolites of the types mentioned above in concentrations as mentioned.

**Skatole**

Another aspect of the invention is a method for reducing the skatole content in animals, said method comprising feeding to a animal a chicory root product for at least one day such as at least two days prior to slaughtering. With regard to this aspect, it can be combined with the characteristics described above, especially in condition to feeding of animal and production of chicory root product.

By feeding animals with the chicory feed product the skatole content of blood plasma is reduced by at least 25%, more preferably at least 40%, more preferably at least 50%, more preferably at least 75%, more preferably at least 80%, more preferably at least 90%, more preferably at least 95%, more preferably at least 98%, more preferably to substantially 0. Surprisingly the reduction of skatole in the blood plasma is greater than expected.

It is preferred that the method of feeding animals with chicory root product is one wherein the skatole content of blood and/or fat is reduced to below the unacceptable human off odour and flavour sensory threshold and maybe even to zero in meat produced from the animals, this is of additional importance as skatole functions as an enhancer of the sensory off-odour/flavour producer androstenone and maybe other off odour/flavour components of unknown origin.

Preferred is that the skatole content of backfat and/or meat is reduced by at least 25%, more preferably at least 40%, more preferably at least 50%, more preferably at least 75%, more preferably at least 80%, more preferably at least 90%, more preferably at least 95%, more preferably at least 98%, more preferably to substantially 0.

Also preferred is that the skatole content of manure is reduced. Skatole in manure (mixture of faeces and urine) can be picked up through the skin when the animals lie in or roll/wallow in the manure (Hansen et al., 1994). Some animals lie in manure to be cooled in the summer, this especially concerns pigs. By this contact between skin and manure the skatole is absorbed through the skin and further transported to the blood, fat and meat. In this way the skatole content in animals can be too high and influence the meat quality e.g. boar taint. Also female and castrated male pigs can obtain a too high content of skatole in the blood, fat and meat e.g. by uptake through the skin (Hansen et al., 1994 & 1995). Reduction of skatole content of backfat and meat of female and castrated male pigs are preferred.

The chicory root product also has an effect on female and castrated male animals resulting in a reduction of skatole content of blood, fat and meat too.

**Androstenone**

The inventors have surprisingly discovered that the amount of androstenone in the blood might be significant lowered by feeding animals with the chicory root product, thus another aspect of the invention is a method for reducing the androstenone content in meat and/or fat and/or blood said method comprising feeding to an animal a chicory root product for at least one day such as at least two days.

Preferred is that the androstenone content is reduced by at least 10%, more preferably at least 25%, more preferably at least 40%, more preferably at least 50%, more preferably at least 75%, more preferably at least 80%, more preferably at least 90%, more preferably at least 95%, more preferably at least 98%.

Preferred is that the androstenone content in blood, meat and/or fat is reduced to below the human off odour/ flavour sensory threshold around 1.0 ppm in backfat. However, the off odour/flavour sensory threshold is different from one person to another. Furthermore the off odour/flavour sensory threshold is unknown when skatole concentration is nearly zero.

It is further preferred that the method as described is used until the animal is subsequently slaughtered.

The aspect of the invention comprising a method for reducing the androstenone content in meat, and/or fat and/or blood can be combined with any characteristic of animal and chicory root product as described elsewhere herein.

**Sensory characteristics**

Another aspect of the invention is a method for improving the odour, flavour, taste and aftertaste of meat from a human sensory perspective, said method comprising feeding to an animal a chicory root product for at least one day such as at least two days prior to slaughter.

The improvement of sensory characteristics comprises reduction/removal of negative boar taint related sensory characteristics defined as Unacceptable and having a Decreased Overall Impression and classified as: Piggy/Animaly-odour and flavour, Manure/Stable-odour and flavour, Livestock/Barney-flavour, Cooked liver/Organy-flavour, Musty-odour, Urine-odour, Sweat-odour, Flat Bitter-aftertaste, White pepper-flavour, Chemical/medicinal-aftertaste. Also the improvement of sensory characteristics comprises reduction/removal of negative lipid oxidation related sensory characteristics classified as: Cardboard-odour and flavour and Linseed oil-odour.

Also the improvement of sensory characteristics comprises increasing the relative levels of positive sensory characteristics defined as Acceptable, having and and Increased Overall Impression and classified as: Fresh cooked pork meat like-odour and flavour, Sweet meaty-odour, Sweet-taste, Umami-taste, Meat/Gamey-odour and flavour, Herby-flavour, Spicy-flavour and Heat/spicy aftertaste, Nutty-odour, Metallic-flavour, Meat/Gamey-flavour, Herby-flavour, Spicy-flavour, Lactic/fresh sour-flavour,

Moreover, the improvement of sensory texture characteristics defined as Acceptable and increasing Overall Impression can be classified as a decrease in Hardness-texture with a resultant relative increase in Tenderness and Juiciness texture attributes. The relative increase in Tenderness and Juiciness texture attributes may be involved in improving acceptability.

Reduction of sensory unacceptable characteristics is of interest in production of meat animals wherein the animal is a ruminant such as cattle, buffalo, sheep, and goat.

Improving the odour, flavour, taste and aftertaste of meat and meat products is of interest in animal production where the animal is a monogastric species.

It is further preferred that the monogastric animal is an animal used for meat, such as pig, poultry, rabbit, hare, more preferably wherein the monogastric animal is a pig.

The aspect of the invention comprising a method for improving the sensory characteristics as defined above in odour, taste and flavour and aftertaste of meat from a human sensory perspective can be combined with any characteristic of animal and chicory root product as described elsewhere herein

**Stable malodour**

Another aspect of the invention is a method for reducing malodour, said method comprising feeding a chicory root product to animals for at least one day such as at least two days.

Reduction of malodour can be caused by a relative reduction in skatole and/or p-cresole and/or indole in the gastrointestinal tract of the animal.

Reduction of malodour directed to the environment especially in areas where humans are living has been performed by different methods as mentioned above. With the chicory root product as food for the animals, the reduction of malodour is obtained by elimination of the problem at the source that is by avoiding the production of the offensive-smelling compounds or reducing the amount of said compounds to a level, which is not perceived as a malodour by humans. Hereby expensive equipment to reduce the malodour from the air e.g. from stables before emission to the surroundings, can be avoided.

Reduction of malodour can be caused by a relative increase in the amount of 2-pentanon and/or ethylbutyrate and/or propylpropionate and/or propylbutyrate and/or butanoic acid 2-methyl-ethylester in the gastrointestinal tract of the animal.

Reduction of malodour can also be caused by a reciprocal change in the relative amounts of odorous compounds ie. decrease in skatole and/or p-cresol and/or indole and increase in the amount of 2-pentanon and/or ethylbutyrate and/or propylpropionate and/or propylbutyrate and/or butanoic acid 2-methyl-ethylester in the gastrointestinal tract of the animal.

Reduction of malodour is of interest in production of animal wherein the animal is a ruminant such as cattle, buffalo, sheep, and goat.

Also reduction of malodour is of interest in animal production where the animal is a monogastric species.

It is preferred that the monogastric animal is a furred animal, such as mink, fox, rat, mouse, muskrat, rabbit, hare, wolf, dog.

It is further preferred that the monogastric animal is an animal used for meat, such as pig, poultry, rabbit, hare, more preferably wherein the monogastric animal is a pig.

Reduction of malodour can occur within the animal with different influences on the surroundings. The surrounding is most influenced when the animal is indoors, according to the invention preferred is wherein the malodour is stable malodour and the animal is kept in a stable. Preferred is when the malodour is manure malodour and the manure originates from animals fed with the chicory root product.

Reducing malodour in manure influences both the conditions in stables and outdoors. When manure is collected and stored e.g. in slurry tank, until it can be spread on land or field, malodour from the slurry tank is possible, also when spreading the manure or slurry on the fields malodour often occurs. Feeding the animal with the chicory root product reduces these malodour problems.

The aspect of the invention relating to a method for reducing malodour can be combined with any characteristic of animal and chicory root product as described elsewhere herein.

**Infections**

Another aspect of the invention is a method for reducing the amount of infections of the gastrointestinal tract in a non-human animal, said method comprising feeding to a non-human animal a chicory root product for at least one day such as at least two days.

Reducing infections of animals is an un-expected effect of the chicory root product, and it reduces the need for administering medicines to the animals such as anthelmintics. This is especially important in organic production systems. Both in organic and non-organic production systems the use of chicory root product as feed will increase animal welfare. The chicory root product is a cheap alternative to the medicines.

Preferred is a method for reducing the amount of infections of the gastrointestinal tract, where the infections are parasites.

Further preferred is a method for reducing the amount of infections of the gastrointestinal tract, where the parasites are worms.

One way of measuring reduction of infections is when the reduction is a reduction of the number of eggs in the animal faeces.

Preferred is reducing the amount of infections where the infections are microbiological infections selected from Coli, Salmonella, Campylobacter and Yersinia.

Further preferred is reducing the amount of infections where the infections are nematode infections selected from *Ascaris suum, Oesophagostomum dentatum, Oesophagostomum quadrispinulatum, Oesophagostomum brevicaudum, Oesophagostomum granatensis, Oesophagostomum georgianum, Hyostrongylus rubidus, Trichuris suis,* and *Strongyloides ransomi* and *Trichinella* sp.

The aspect of the invention comprising a method for reducing the amount of infections of the gastrointestinal tract in a non-human animal can be combined with any characteristic of animal and chicory root product as described elsewhere herein.

**Products**

In an aspect of the invention is described a chicory root product comprising components from chicory roots, where said components comprises at least inulin, one or more low molecular sugars and one or more secondary metabolites. Inulin is considered to be a mixture of oligofructosaccharides and polyfructosaccharides.

In an embodiment the chicory root product further includes fructo-oligosaccharides. Some of these fructo-oligosaccharides may be similar to inulin but are not limited to inulin and break down products of inulin.

The saccharides in the chicory root product may have from 2-200 sugar units, such as from 2 to 20 units, such as 20-40 units, such as 40-60 units, such as 60-80 units, such as 80-100 units, such as 100-120 units, such as 120-140 units, such as 140-160 units, such as 160-200 units. Preferred is 2-20 units, 20-40 units and 40-60 units.

The oligofructosaccharides and polyfructosaccharides may be branched or non-branched, preferred is non-branched saccharides.

In an embodiment the low molecular sugars of the chicory root product are selected from but not limited to the group of glucose, fructose, sucrose, maltose, maltotriose, maltotetraose, inulin, fructan (tri to octasaccharides).

In another embodiment the chicory root product also include secondary metabolites selected from the group of terpenes, phytosterols, polyamines, coumarins and flavonoids.

The secondary metabolites may be selected from the group of Sesquiterpene lactones such as 8-Deoxylactucin, crepidiaside, lactucin, lactupicrin, crepidraside, 11-β-13-dihydrolactucin, picriside, sonchuside A, sonchuside C, cichoriolide A, cichoriosides A, cichorioside B and cichorioside C; Phytosterols such as Sitosterol, stigmasterol, and campersterol; Coumarines such as Esculetin (=aesculetin), esculin (the glucon of esculetin), cichoriin-6'-p-hydroxyphenyl acetate and cichoriin; Flavonoids such as Luteolin 7-glucuronide, quercetin 3-galactoside, quercetin 3-glucuronide, kaempferol 3-glucoside, kaempferol 3-glucuronide, isorhamnetin 3-glucuronide; Anthocyanins such as Cyanidin 3-O-β-(6-o-malonyl)-D-glucopyranoside and four delphinidin derivatives; Caffeic acid derivatives such as Caffeic acid, chicoric acid, and chlorogenic acid; Polyamines (biogenic amines) such as Putrescine, spermidine, spermine.

The chicory root product as described herein may have the concentration of low molecular sugar, inulin, and secondary metabolites as described elsewhere herein.

In an embodiment of the chicory root product the components from chicory comprises at least 50 % of the chicory root product.

The chicory root product as described may contain chicory roots that are dried. Drying processes may be one that are generally known in the art, especially drying processes used for drying sugar beets, sugar beet pulp and grasses are suitable.

In the production of the chicory root product the chicory roots may be fractionated.

The chicory root product as described may further comprise acids used for conservation of organic feed components.

Another aspect of the invention is the use of chicory roots as a feed product for "grown up" (>> 7 weeks) pigs.

The aspect of the invention comprising use of chicory roots as a feed product for "grown up" pigs can be combined with any characteristic chicory root product as described elsewhere herein.

Another aspect of the invention is a use of chicory roots for preparing a feed product for "grown up" pigs.

The aspect of the invention comprising use of chicory roots as a feed product for "grown up" pigs can be combined with any characteristic chicory root product as described elsewhere herein.

Another aspect of the invention is the use of chicory roots for preparing a product for the prevention of boar taint. This product may be a food product.

The aspect of the invention comprising use of chicory roots for preparing a product for the prevention of boar taint can be combined with any characteristic chicory root product as described elsewhere herein.

Another aspect of the invention is a use of chicory roots for preparing a product for reduction of skatole content in pigs, in particular in boar fat.

The aspect of the invention comprising use of chicory roots for preparing a product for reduction of skatole content in pigs can be combined with any characteristic chicory root product as described elsewhere herein.

Another aspect of the invention is a use of chicory roots for preparing a product for reduction of androstenone in pigs.

The aspect of the invention comprising use of chicory roots for preparing a product for reduction of androstenone in pigs can be combined with any characteristic chicory root product as described elsewhere herein.

Another aspect of the invention is a use of chicory roots for preparing a product for reduction or prevention of gastrointestinal tract infections in pigs.

The aspect of the invention comprising use of chicory roots for preparing a product for reduction or prevention of gastrointestinal tract infections in pigs can be combined with any characteristic chicory root product as described elsewhere herein.

**Examples**

**Example 1**

**Feeding with chicory roots reduces the amount of odorous compounds in colon contents of pigs**

Alcohols and carboxylic acids are compounds with relatively negative odour impressions. When alcohols and carboxylic acids react, pleasant smelling esters are created and the result can be a less offensive odour impact. This can be illustrated by the reaction between ethanol and butyric acid, which results in ethylbutyrate, or by the reaction between propanol and butyric acid, which results in propylbutyrate.

**Animals and feed**

The inulin content of chicory roots (variety Orchies) for the pig odour experiment was 15% on wet basis and the content of feed units for pigs was 27 FUp (pigs) per 100-kg chicory roots measured by chemical analysis. The experiment is a subset of an experiment, which consisted of 4 treatments each of eight pigs. The 32 pigs (16 intact male and 16 female pigs) were kept in litters of 8 pigs and fed 100 % organic concentrate and semi ad libitum grass silage the first 5 weeks. From week 6 the 32 pigs were distributed to the four treatments according to litter and sex in individual pens. Treatment 1 and 3 were selected for the present odour study as they represented the extremes of the treatments (Table 3). Treatment 1 was a (conventional) control group given 100-energy % organic concentrate and no roughage from week 6 until slaughter. Composition of the organic concentrate diet during the whole experiment was (g/kg): 145.5 rapeseed cake, 240.0 peas organic, 223.0 wheat organic, 220 barley organic, 50 oat organic, 100.0 GMO-free toasted soybeans, 2 Sv.vit-411 organic, 3.75 salt, 12 limestone and 3.63 monocalcium phosphate. The concentrate diet contained 8.57 MJ net energy (1.11 feed units (FUp)) and 149,7 g digestible protein per kg food. The 25 % blended organic chicory roots on energy basis plus 70 % organic concentrate were given from week 6 until slaughter of treatment 3.

Finally, the pigs were slaughtered 15 weeks from initiation of the experiment for measuring meat and eating quality as well as parasites. The pigs ate the high amount of fresh and bitter blended chicory roots without problems after one week of adaptation by giving individually increasing amounts of chicory roots during the first week.

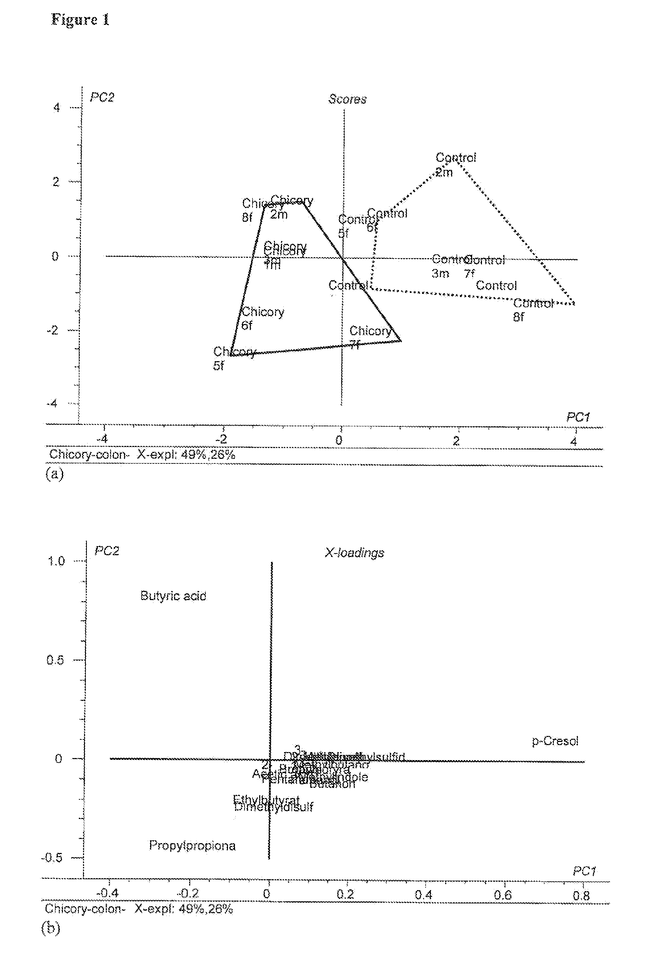

The raw GC-MS areas in Table 1 and Figure 1 show that feeding pigs with the inulin containing chicory roots the fermentation pattern in the colon is shifted from protein fermentation to carbohydrate fermentation. The result is a change in composition of odorous compounds from the obnoxious protein fermentation products as p-cresol and skatole to the less offensive esters. The PCA-plot also confirms that the fermentation product pattern is well separated and mostly controlled by p-cresol and butyric acid.

**Table 1.** GC-MS areas of selected compounds from the colon in chicory roots and control fed fattening pigs.

| Treatment | 1 | | 3 | | 1/3 | |
|---|---|---|---|---|---|---|
| No. of pigs Food components | 8 | | 7 | | | |
| | 100 % organic concentrate | | 70 % organic concentration plus 25 % chicory roots | | Significant difference between treatments | Factorial ratio between treatments |
| Compound: | SMEAN | Std.err. | LSMEAN | Std.err. | P-value | |
| Dimethylsulfide | 83736 | 6827 | 48145 | 8078 | NS | 1.74 |
| 2-Butanon | 54274 | 7681 | 59512 | 9088 | NS | 0.91 |
| Acetic acid | 252338 | 42504 | 286741 | 50292 | NS | 0.88 |
| 2-Pentanon | 22742 | 7513 | 48500 | 8889 | * | 0.47 |
| Dimethyldisulfide | 132354 | 52309 | 128911 | 61893 | NS | 1.03 |
| 1-Pentanol | 29277 | 6201 | 47543 | 7337 | NS | 0.62 |
| 2-Methylpropanoic acid | 43571 | 9416 | 29886 | 11141 | NS | 1.46 |
| *Ethylbutyrate (ester)* | *5026* | *28026* | 48440 | *33161* | *NS* | *0.10* |

(continued)

| Treatment | 1 | | 3 | | | 1/3 |
|---|---|---|---|---|---|---|
| No. of pigs Food components | 8 100 % organic concentrate | | 7 70 % organic concentration plus 25 % chicory roots | | Significant difference between treatments | Factorial ratio between treatments |
| Propylpropionate (ester) | 23718 | 40419 | 174429 | 47824 | (*) | 0.14 |
| Butanoic acid | 935596 | 118921 | 878861 | 140710 | NS | 1.06 |
| Butanoic acid, 2-methyl-,ethylester | 2663 | 1599 | 8679 | 1892 | * | 0.31 |
| Propylbutyrat (ester) | 3208 | 1145 | 7760 | 1355 | * | 0.41 |
| 3-Methylbutanoic acid | 96309 | 12822 | 64413 | 15171 | NS | 1.50 |
| Dimethyltrisulfide | 7196 | 2755 | 6252 | 3260 | NS | 1.15 |
| p-Cresol | 347725 | 27566 | 72516 | 32616 | ** | 4.8 |
| Indole | 19943 | 2487 | 6690 | 2942 | (*) | 3.0 |
| 3-methylindole = skatole | 25322 | 4954 | 3740 | 5862 | ** | 6.8 |

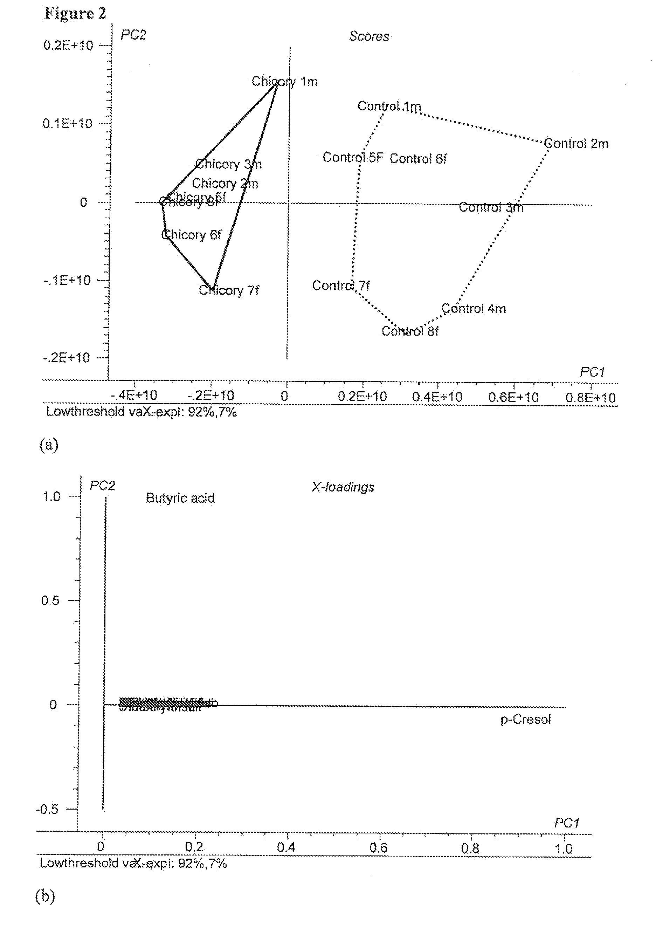

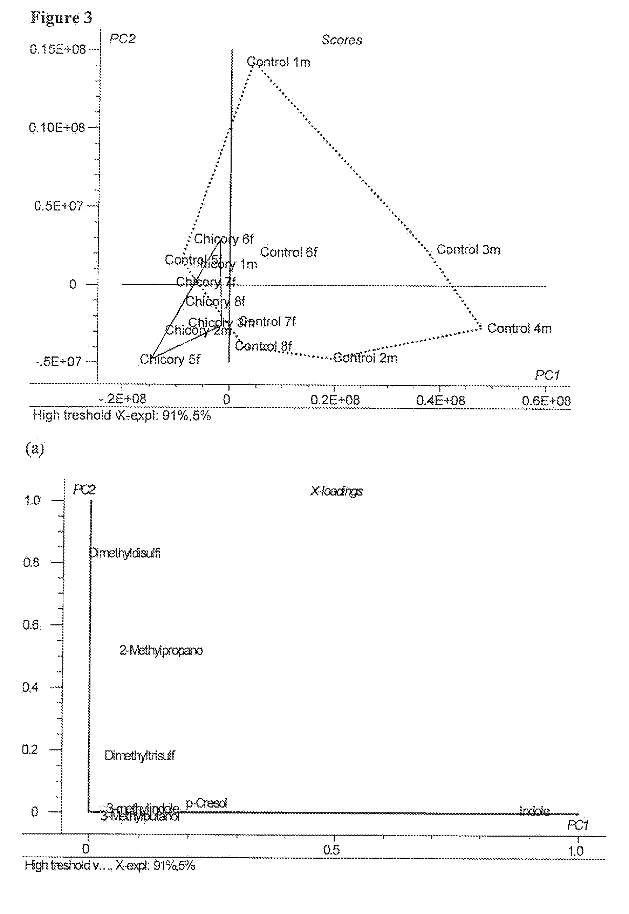

Although the sensory impression of a mixture of odourous compounds is a combination of all compounds in the mixture, some of the compounds can have a higher impact on the odour impression due to their low threshold values. In addition to the threshold values of the odourous compounds the odour quality of the compounds should be taken into consideration. The odour quality of a compound can change by concentration e.g. skatole has a pleasant flower-like odour at very low concentrations whereas the same compound is nauseating at higher concentrations. In contrast some groups of compounds have a relatively pleasant odour description, even at higher concentrations e.g. esters, which usually have fruity odour notes. By dividing the raw GC-MS data by the odour thresholds of selected compounds we try to illustrate the impact of odours with widely different odour thresholds (Table 2 and Figure 2 and 3). As the reported values in the literature of odour thresholds can vary widely the Figures 2 and 3 is illustrating the extremes. By incorporating the odour thresholds in the raw data an illustration of the impact of sensory impression of the mixture is created, in contrast to the individual compounds. In both figure 2 and 3 the chicory fed pigs are more confined than in the raw data, in contrast to the control fed pigs, which are more scattered. The chicory roots are therefore able to control the production of odorous compounds in the colon, and effectively turn the fermentation from protein fermentation to carbohydrate fermentation.

**Table 2.** Odour descriptor and odour thresholds in air of chemical compounds.

| | Odour descriptor | Odour threshold air, mg/m3 | |
|---|---|---|---|
| | | Low (4) | High |
| Dimethylsulfide (=methylthiomethane) | Cooked vegetable, garlic, hydrogen sulfide (1) | 0,002 | 0,65 |
| 2-Butanon | Acetone, varnish (1) | 0,75 | 250 |
| Acetic acid | Vinegar (1) | 0,025 | 76 |
| 2-Pentanon | Jasmine, Geranium, varnish (1) | 11 | 48 |
| Dimethyldisulfide (=methyldithiomethane) | Decayed vegetables (3) | 0,003 | 0,029 |
| 1-Pentanol | Alcohol, medicinal (1) | 0,1 | 1100 |
| 2-Methylpropanoic acid (=isobutyric acid) | Sweaty, bitter, sour (1) | 0,00072 (3) | 0,0072 (3) |
| Ethylbutyrate (=Ethylbutanoate) | Butter, sweetish, apple, perfumed (1) | 0,13 | 0,28 |

(continued)

| | Odour descriptor | Odour threshold air, mg/m3 | |
|---|---|---|---|
| | | Low (4) | High |
| Propylpropionate (=propylpropanoate) | Complex fruity odour (apple banana) (2) | 0,23 | 0,26 |
| Butyric acid (butanoic acid) | Buttery, cheesy, sweaty (1) | 0,0004 | 9 |
| Butanoic acid,2-methyl-,ethylester (*) Propylbutyrat 3-Methylbutanoic acid (=isovaleric acid) | Pineapple, apricot (2) Cheese, sweaty (1) | 0,005 | 3 |
| Dimethyltrisulfide (=methyltrithiomethane) | Fresh onion (2) | 0,0073 | 0,0073 |
| p-Cresol (4-methyl-phenol) | Phenol like (2) | 0,00005 | 0,04 |
| Indole | Floral (highly pure) otherwise fecal (2) | 0,0006 | 0,0006 |
| 3-Methylindole | Fecal (high concentration) floral (low concentration) (2) | 0,00035 | 0,1 |

(1): Meilgaard, 1975
(2): Fenaroli's Handbook of Flavor Ingredients 3. Ed. 1995
(3): Zahn et al. 2001
(*) Ethyl-2-methylbutyrate is mentioned in Fenaroli's but not with odour descriptor.
(4) Gemert+Nettenbreijer, 1977

In addition to the reduction of the odorous compounds, the feeding with chicory roots may reduce the production of ammonia. The fermentation of inulin in the caecum and colon of pigs results in production of short chain fatty acids. The higher amount of short chain fatty acids reduces the pH. This reduction has a positive influence on the retention of ammonia in the faeces and manure. This results in an improved environment in the stable and in the surroundings (Lenis and Jongbloed, 1999; Sutton et al. 1999). The ammonia emission is further reduced as the bacteria switch from protein-fermentation to carbohydrate fermentation when feeding with chicory roots. Furthermore, as the bacteria grow the nitrogen will be used for production of proteins in the bacterial biomass and is therefore not available for production of ammonia or odorous compounds.

It is not necessary to completely eliminate the presence of odourous compounds in the colon of pigs to reduce the odour impact on ambient air quality. The reduction should only be sufficient to improve the ambient air quality to an acceptable level. The amount of chicory roots necessary for a sufficient reduction of odourous compounds in the colon contents of pigs remains therefore to be determined. If the amount of chicory roots necessary for sufficient reduction can be reduced the method will be more cost effective. In addition to the odour-reducing effects the chicory roots have following benefits: Easy to grow in the present agricultural systems, can be handled by equipment used for other crops as sugar beets, is in it self a valuable feed component, and contain bioactive secondary metabolites (Bais and Ravishankar, 2001).

**Table 3.** Experimental design for the final feeding period of the 2 treatments feeding with or without the chicory roots for different periods from 55 -120 kg live weight (9 weeks).

| Treatment | No. of pigs | Food composition and energy level compared to semi ad lib. (100 %) (from 55-120 kg) | Roughage |
|---|---|---|---|
| 1 | 8 4 female + 4 male | 100 % organic concentrate | None |
| 3 | 8 4 female + 4 male | 70 % Organic concentrate + chicory roots (25 %) from 55 kg until slaughter | Chicory roots (2.1-3.0 kg per day) from 55 kg until slaughter |

## Collection of samples and sample preparation

Immediately after slaughter, the gastrointestinal tract (GIT) was removed and the colon and rectum was separated from the rest of the GIT. The contents from colon and rectum was quantitatively transferred to a basket and mixed so a representative sample could be obtained. The samples were stored at -20°C before preparation for analysis. To prepare the samples for analysis 3 gram were transferred to 10 ml vials with addition of 3 ml saturated NaCl, the samples were mixed and stored at -80°C before analysis. The saturated NaCl was added to increase the transfer of volatiles to the gas phase and to stop further microbial activity in the samples. On the day of analysis the samples were transferred to an oven hold at 40°C (approximately the body temperature of pigs) and thawed and equilibrated at this temperature for 25 minutes with occasional shaking to increase the transfer of volatiles from the medium to the headspace. For extraction a solid phase microextraction (SPME) fiber (75 $\mu$m polydimethylsiloxane/carboxen, Supelco) was exposed to the headspace for 1 minute and immediately transferred to the injection port of the gas chromatograph for desorption.

**GC-MS measurement of volatiles**

The gas chromatograph was a Varian model STAR 3400 CX. The column was a HP5-MS (Agilent) 30 m long, 0.25 mm internal diameter and with a 0.25 $\mu$m film thickness. Injection temperature was set to 250°C and the column temperature program was as follows: Hold at initial temperature 35°C for 10 minutes, then increase to 130°C with 3°C/minute, finally increase to 250°C with a rate of 40°C/minute and hold at this temperature for 5.34 minutes. The carrier gas was helium with a linear flow rate of 29 cm s$^{-1}$ at 35°C, the samples were run one at a time to secure the samples were treated in exactly the same way. The temperature of the transferline between the gas chromatograph and the mass spectrometer was set to 275°C. The mass spectrometer was a Varian model Saturn 2000 operated in electron impact mode, with the following settings: detection mass range: 35 to 300 m/z; mulitplier voltage: 1800, axial modulation: 4V, trap temperature 200°C; and manifold temperature of 52°C.

The compounds were identified by comparison with standard spectra from NIST/EPA/NIH or by comparison with spectra from original standards.

**Statistical analysis**

The statistical analyses were carried out with the Statistical Analysis System version 8.2 (SAS Institute, 1999-2001 by SAS Institute Inc., Cary, NC, USA). The GLM procedure was used to calculate the least squares means and standard error of the means for the odour impact compounds from colon. The models included the fixed effect of diet, sex and animal replicate (litter) as well as interaction between diet and sex (model 1).

$$Y = \mu + a_{diet} + b_{litter} + c_{sex} + ac_{diet*sex} + e_{rror} \text{ (model 1)}$$

Y = dimethylsulfide, 2-butanone, acetic acid, 2-pentanone, dimethyldisulfide, 1-pentanol, 2-methylpropanoic acid, ethylbutyrate, propylpropionate, butyric acid, 3-methylbutanoic acid, propybutyrate, ethyl-2-methyl butanoate ethylester, dimethyltrisulfide, p-cresol, indole, and skatole.

The raw data of the GC-MS areas of the odour compounds as well as values corrected for low and high threshold values were analysed by the GLM- model 1 to investigate the effect of the two diets.

Principal component analysis (PCA) were carried out also using the data of the raw GC-MS area, as well as data corrected for low and high odour threshold, to investigate the effect of the two diets. Full cross validation (leave one out) was applied. Data analysis was carried out with the software The Unscrambler version 7.8 (Camo AS, Oslo, Norway).

**Results**

Table 1 show the peak mean area of GC-MS analyses of selected odour impact compounds found in headspace over the colon samples. The compounds 2-pentanone, ethylbutyrate, propylpropionate, butanoic acid, ethyl-2-methylbutyrate, p-cresol, indole and skatole show significant difference between the two treatments. The esters, which have relatively pleasant odours, are increased in treatment 3 (factorial difference below 1), whereas the malodorous compounds, p-cresol, indole and skatole were decreased in treatment 3 (factorial difference above 1).

The amounts of odour-active compounds found in colon contents does not give a realistic impression of the odour intensity of the mixture as the various compounds can have very different odour thresholds and odour descriptors. Table 2 shows odour threshold values and odour descriptors of the selected compounds found in colon contents. The relative odour activity of the individual compounds can be calculated by dividing the area of the compound with the odour threshold. Thereby can a compound, which is present in low amount result in a high odour impact if the odour threshold is low. The relative "odour-activity" of the two experimental treatments can therefore be compared. It has not been possible to find odour threshold values for ethyl-2-methylbutyrate and propylbutyrate they are therefore omitted in the calculations.

Figure 1 shows the PCA analysis of the dataset from the raw data. Treatment 1 (control) and treatment 3 (chicory addition) are clearly separated with no overlap between the treatments. The first principal component (x-axis) is controlled by p-cresol (protein degradation product) whereas the second (y-axis) is controlled by butyric acid and propyl propionate which both are degradation products of carbohydrate.

The raw data does not give an impression of the odour of a mixture of volatile compounds, as the compounds can have widely different odour thresholds. The raw data was therefore divided by the odour threshold values found in the literature (Gemert and Nettenbreijer, 1977 and Zahn et al. 2001). The values found in the literature wary widely, the lowest and highest values have therefore both been applied to give an impression of the effect on the potential odour impression. Figure 2 shows the PCA-analysis of the raw data divided by the low odour threshold values to give odour-activity corrected values. The two treatments are clearly separated and the clusters of points are more confined, especially with the pigs given a diet containing chicory. The first principal component is controlled by p-cresol whereas the second is controlled by butyric acid.

Figure 3 shows the PCA-analysis of the raw data divided by the high odour threshold values. The pigs fed control diet are more dispersed and overlap the chicory fed pigs. In contrary to the controls the pigs fed the chicory diet are highly confined. The first principal component is in this case controlled by indole (protein degradation product) whereas the second is controlled by dimethyl disulfide, 2-methyl propanoic acid and to a lesser degree dimethyl trisulfide (all protein degradation products).

**Example 2A**

**Influence of chicory roots on boar taint (skatole and androstenone) in pigs**

**Methods**

**Animals and feed**

An inulin-rich variety Orchies of chicory (Cichorium intybus L. var. Orchies) for fattening pig diets has been used in this experiment. The yield of the organically grown crop varied from 30 t/ha in one year and 40 t/ha the following year. The first year the inulin content (fructan) of the chicory roots of the variety Orchies was around 150 g per kg feed and contained 2.11 MJ net energy (0.27 feed units (FUp)) and 23.4 g digestible protein per kg feed of chicory roots. The pigs ate the high amount of fresh and bitter blended chicory roots (from 2.1-3.0 kg per day during the experimental period) without problems after one week of adaptation by giving individually increasing amounts of chicory roots during that week.

The first of two pig experiments consisted of 40 pigs (20 entire male and 20 female pigs), all free of parasite infections. The 40 pigs were kept in litters and fed 100 energy % organic concentrate according to scale (Madsen et al., 1990) and ad libitum grass silage. Composition of the organic concentrate diet during the whole experiment was (g/kg): 145.5 rapeseed cake, 240.0 peas organic, 223.0 wheat organic, 220 barley organic, 50 oat organic, 100.0 GMO-free toasted soybeans, 2 Sv.vit-411 organic, 3.75 salt, 12 limestone and 3.63 monocalcium phosphate. The concentrate diet contained 8.57 MJ net energy (1.11 feed units (FUp)) and 149,7 g digestible protein per kg food. All 40 pigs were then infected with a specific parasite within a period of 5 weeks from initiation of the experiments. Eight pigs, four of each sex, were slaughtered on the 10th December due to the parasite experiment.

5 weeks from initiation of the experiments the 32 pigs were distributed according to live weight, litter and sex to four treatments in individual pens (Table 4). Treatment 1 was a conventional control group given 100-energy % organic concentrate and no roughage from week 6 until slaughter. Treatment 2 was an organic control group given 95 energy % organic concentrate and ad libitum grass silage from week 6 until slaughter. The 25% blended chicory roots on energy bases plus 70% organic concentrate were given to treatment 3 from week 6 until slaughter. However, the first week the pigs had to adapt to eating chicory roots. Treatment 4 was given 95 energy % organic concentrate and semi ad libitum roughage from week 6 until week 12. In week 12 the pigs increased the intake of chicory roots (adaptation period), and from week 13 until slaughter of treatment 4, 25% blended chicory roots on energy bases plus 70% concentrate were given. Blood samples for measuring androstenone and skatole in blood plasma were collected in week 5 and in week 14 (one week before slaughter) of male and female pigs. Finally, the 16 male pigs were slaughtered 15 weeks from initiation of the experiment and the 16 female pigs the day after. Skatole was measured in backfat, and a sensoric panel evaluated eating quality (see Table 4).

After one week of adaptation in which the pigs were fed increasing amounts of chicory roots, the pigs ate the high amount of fresh and bitter blended chicory roots without problems. The health status and production results of the chicory treatments were as good as the control treatments, and the daily gain corresponded to the results of treatment 2. The chicory-fed pigs ate after the one-week adaptation period 2.1 kg chicory per day from the beginning of treatment 3 and finally 3.0 kg per day during the final three weeks of both treatment 3 and 4. All the planned meat and eating quality measurements have been carried out and analysed. Furthermore, several additional measurements, have been analysed e.g glycogen, driploss, pH, temperature, Minolta colour values L*, a* b* in *M. long. dorsi* and fatty acids.

**Table 4.** Experimental design for the final feeding period of the 4 treatments fed diets with or without bioactive chicory roots for different periods from 55-120 kg live weight (9 weeks).

| Treatment | No. of pigs | Food composition and energy level compared with 100 energy % according to scale (55-120 kg) | Bioactive food |
|---|---|---|---|
| 1 Non Bioactive Control | 8 4 females + 4 males | 100% organic concentrate | None |
| 2 Silage | 8 4 females + 4 males | 95% organic concentrate + ad lib. clover-grass silage | Clover-grass silage from 55 kg until slaughter |
| 3 Chicory | 8 4 females + 4 males | 70% organic concentrate + chicory roots (25%) from 55 kg until slaughter | Chicory roots (2.1-3.0 kg per day) from 55 kg until slaughter |
| 4 Chicory/ Silage | 8 4 females + 4 males | 95% organic concentrate + ad lib. clover-grass silage from 55 kg until 4 weeks before slaughter | Clover-grass silage ad lib. from 55 kg until 4 weeks before slaughter |
| | | 70% organic concentrate + adaptation to chicory roots from 4-3 weeks before slaughter | 4-3 weeks before slaughter, adaptation to chicory roots |
| | | 70% organic concentrate + chicory roots (25%) the last 3 weeks before slaughter | chicory roots (25%) (3.0 kg per day) |

**Statistical analysis**

The statistical analyses were carried out with the Statistical Analysis System version 8.2 (SAS Institute, 1999-2001 by SAS Institute Inc., Cary, NC, USA). The GLM procedure was used to calculate the least squares means and standard error of the means for the odour impact compounds from colon. The models included the fixed effect of diet, sex and animal replicate (litter) as well as interaction between diet and sex (model 1).

$$Y = \mu + a_{diet} + b_{litter} + c_{sex} + ac_{diet \cdot sex} + e_{rror} \text{ (model 1)}$$

Y = skatole in blood and backfat and androstenone in blood.

**Results**

The effect of feeding 25% chicory roots plus 70% organic concentrate for a long (treatment 3) or a short time (treatments 4) on skatole and androstenone in blood plasma from *Vena jugularis* and skatole from backfat, and meat and eating quality have been compared with results of the two control treatments, feeding 100% organic concentrate (treatment 1) or 95% organic concentrate plus clover grass silage (treatment 2) (Table 5 to Table 8).

After one week of adaptation, it was possible to feed 25% minced chicory roots and 70% concentrate on energy bases without problems during the finishing period from 55 kg live weight until slaughter around 120 kg. In the final period, the pigs ate 3 kg minced chicory roots. Some of the pigs found the chicory so palatable that they ate the chicory before the concentrate.

Irrespective of sex and experimental period, all chicory-fed pigs showed skatole concentrations in backfat (after 8 and 3 weeks) and skatole concentrations in blood plasma (after 7 and 2 weeks) which were not significantly different from zero in a statistical GLM analysis in SAS (see Table 5, 6, and 7). A decrease in the androstenone level in treatment 3 compared with treatment 1 seems to be significant, when the results are corrected by the covariate androstenone in blood just before the feeding experiment started. More importantly, none of the chicory-fed male pigs showed androstenone results above the critical limit for off flavour from androstenone as opposed to some of the control-fed male pigs in treatments 1 and 2, which also had skatole concentrations above the off odour limit of 0.20 μg/g in backfat (see table 8).

**Table 5.** Skatole in backfat (μg/g) according to treatment and sex (Mean and Std. Dev.)

| Treatment | Sex | N | Mean | Std. dev. |
|---|---|---|---|---|
| 1 | Male | 4 | 0.115 | 0.04 |
| 1 | Female | 4 | 0.05 | 0.0282 |
| 2 | Male | 4 | 0.1325 | 0.1117 |
| 2 | Female | 4 | 0.0375 | 0.022 |
| 3 | Male | 4 | 0.0125 | 0.005 |
| 3 | Female | 4 | 0.0125 | 0.005 |
| 4 | Male | 4 | 0.0175 | 0.0096 |
| 4 | Female | 4 | 0.01 | 0.00 |

**Table 6.** Skatole in backfat (μg/g) (Ismeans and error)

| Treatment | N | LS Mean | Std. error | Pr> |t| |
|---|---|---|---|---|
| 1 | 8 | 0.0825 | 0.015 | <.0001 |
| 2 | 8 | 0.085 | 0.015 | <.0001 |
| 3 | 8 | 0.0125 | 0.015 | 0.4081 |
| 4 | 8 | 0.01375 | 0.015 | 0.3639 |

**Table 7.** Skatole in blood 1 week before slaughter (μg/l) (Ismens and error)

| Treatment | N | LS Mean | Std. error | Pr> |t| |
|---|---|---|---|---|
| 1 | 8 | 1.8225 | 0.36 | 0.0001 |
| 2 | 8 | 2.12875 | 0.36 | <.0001 |
| 3 | 8 | 0.0825 | 0.36 | 0.8230 |
| 4 | 8 | 0.13 | 0.36 | 0.7248 |

**Table 8.** Skatole in blood and backfat from the male and female pigs and androstenone in blood from the male pigs plus some performance results in 16 male and 16 female finishing pigs

| Pig no. | Slaughter date | Treatment* | Feeding[1] | Sex | Live weight | Skatole backfat at slaughter ($\mu$g/g) | Skatole blood 1 week before slaughter ($\mu$g/l) | Percentage of meat in carcass | Androstenone in blood 1 week before slaughter (ng/ml) |
|---|---|---|---|---|---|---|---|---|---|
| 53 | 11.02.02 | 1 | 100% Concentrate | male | 135.8 | 0.15 | 1.37 | 58.4 | 11.4 |
| 7 | 11.02.02 | 1 | 100% Concentrate | male | 134.5 | 0.15 | 1.72 | 57.2 | 24.4 |
| 27 | 11.02.02 | 1 | 100% Concentrate | male | 112.8 | 0.10 | 1.68 | 59.4 | 11.0 |
| 38 | 11.02.02 | 1 | 100% Concentrate | male | 125 | 0.06 | 0.84 | 58.6 | 19.8 |
| 52 | 13.02.02 | 1 | 100% Concentrate | female | 126 | 0.09 | 2.19 | 57.3 | |
| 9 | 13.02.02 | 1 | 100% Concentrate | female | 123.3 | 0.03 | 2.16 | 59.5 | |
| 34 | 13.02.02 | 1 | 100% Concentrate | female | 113.3 | 0.05 | 3.9 | 60.2 | |
| 50 | 13.02.02 | 1 | 100% Concentrate | female | 116.9 | 0.03 | 0.72 | 61.6 | |
| 54 | 11.02.02 | 2 | 95% Concentrate +silage | male | 113.2 | 0.08 | 1.53 | 59.0 | 7.0 |
| 12 | 11.02.02 | 2 | 95% Concentrate +silage | male | 145.5 | 0.30 | 5.42 | 57.4 | 25.1 |
| 19 | 11.02.02 | 2 | 95% Concentrate +silage | male | 102.5 | 0.08 | 4.44 | 60.0 | 9.6 |
| 36 | 11.02.02 | 2 | 95% Concentrate +silage | male | 120.9 | 0.07 | 0.62 | 60.3 | 9.3 |

(continued)

| Pig no. | Slaughter date | Treatment* | Feeding[1] | Sex | Live weight | Skatole backfat at slaughter ($\mu$g/g) | Skatole blood 1 week before slaughter ($\mu$g/l) | Percentage of meat in carcass | Androstenone in blood 1 week before slaughter (ng/ml) |
|---|---|---|---|---|---|---|---|---|---|
| 55 | 13.02.02 | 2 | 95% Concentrate +silage | female | 119.2 | 0.03 | 1.6 | 58.1 | |
| 22 | 13.02.02 | 2 | 95% Concentrate +silage | female | 126.7 | 0.01 | 0.75 | 59.0 | |
| 37 | 13.02.02 | 2 | 95% Concentrate +silage | female | 102.7 | 0.06 | 1.4 | 62.2 | |
| 45 | 13.02.02 | 2 | 95% Concentrate +silage | female | 108.2 | 0.05 | 1.27 | 63.0 | |
| 56 | 11.02.02 | 3 | 70% Conc. + 25% chicory | male | 113 | 0.01 | 0.19 | 59.1 | 6.1 |
| 14 | 11.02.02 | 3 | 70% Conc. + 25% chicory | male | 134 | 0.02 | 0.02 | 57.4 | 16.1 |
| 33 | 11.02.02 | 3 | 70% Conc. + 25% chicory | male | 115.4 | 0.01 | 0.1 | 59.8 | 11.9 |
| 43 | 11.02.02 | 3 | 70% Conc. + 25% chicory | male | 112 | 0.01 | 0.07 | 59.8 | 18.5 |
| 57 | 13.02.02 | 3 | 70% Conc. + 25% chicory | female | 113.8 | 0.01 | 0.05 | 58.7 | |
| 15 | 13.02.02 | 3 | 70% Conc. + 25% chicory | female | 116.5 | 0.01 | 0 | 60.4 | |
| 20 | 13.02.02 | 3 | 70% Conc. + 25% chicory | female | 119.3 | 0.01 | 0.16 | 60.8 | |
| 31 | 13.02.02 | 3 | 70% Conc. + 25% chicory | female | 97.8 | 0.02 | 0.07 | 62.5 | |
| 58 | 11.02.02 | 4 | 70% Conc. + 25% chicory | male | 108.4 | 0.02 | 0 | 60.2 | 10.2 |

| Pig no. | Slaughter date | Treatment* | Feeding[1] | Sex | Live weight | Skatole backfat at slaughter ($\mu$g/g) | Skatole blood 1 week before slaughter ($\mu$g/l) | Percentage of meat in carcass | Androstenone in blood 1 week before slaughter (ng/ml) |
|---|---|---|---|---|---|---|---|---|---|
| 21 | 11.02.02 | 4 | 70% Conc. + 25% chicory | male | 120.3 | 0.01 | 0.12 | 59.0 | 14.7 |
| 35 | 11.02.02 | 4 | 70% Conc. + 25% chicory | male | 122.4 | 0.03 | 0.24 | 59.1 | 13.7 |
| 49 | 11.02.02 | 4 | 70% Conc. + 25% chicory | male | 116.8 | 0.01 | 0.05 | 60.3 | 10.3 |
| 59 | 13.02.02 | 4 | 70% Conc. + 25% chicory | female | 118.7 | 0.01 | 0.11 | 60.1 | |
| 13 | 13.02.02 | 4 | 70% Conc. + 25% chicory | female | 133.9 | 0.01 | 0.1 | 59.2 | |
| 23 | 13.02.02 | 4 | 70% Conc. + 25% chicory | female | 115 | 0.01 | 0.24 | 60.3 | |
| 51 | 13.02.02 | 4 | 70% Conc. + 25% chicory | female | 96.1 | 0.01 | 0.18 | 62.3 | |

*) Treatment 4 got 25 % chicory roots the last three weeks before slaughter, while Treatment 3 got 25 % chicory roots the last eight weeks before slaughter.

**Example 2B**

**Sensory and chemical investigations of eating quality of pork in relation to the influence of bioactive forage feeding**

**Influence of chicory roots (fresh and dried) and inulin on production and boar taint (skatole and androstenone) in entire male pigs**

The experiment was consisted of 4 treatments each of 8 entire male pigs. The male pigs were distributed to the 4 treatments according to litter and initial weight and the pigs were kept in individual pens. Four weeks prior to initiation of the experiment the 32 pigs were fed 100% organic concentrate diet according to scale plus ad libitum grass silage and were infected twice with parasites. Then the last 6 weeks prior to slaughter the pigs were fed according to the plan (see Table 9). Treatment 1 was an organic "control" treatment fed 95% organic concentrate plus clovergrass silage. Treatment 2 was fed 70% organic concentrate plus 25% bioactive blended fresh chicory roots. Treatment 3 was fed 70% organic concentrate plus 25% dried chicory roots. Treatment 4 was fed 70% organic concentrate plus 14% pure inulin corresponding to the amount of inulin in the chicory roots of treatment 2 and 3. The pigs had an initial weight of 83-84 kg and were slaughtered at 120-kg liveweight to secure sexual maturity of the entire male pigs after a 6 weeks experimental period. Strategic blood and meat samples have been collected according to plan before start of the experiment 6 weeks before slaughter and just before and after slaughter. Analysis (chemically and statistically) of the meat and eating quality measurements has been conducted according to the plan. The traditional meat quality measurements collected just before (glycogen) and after slaughter (meat percent in carcass, pH, temperature, Minolta-colour values and driploss in the loin) has been statistical analysed. Furthermore the sensory profile of the loin, androstenone analysis in blood plasma and analysis of skatole in blood plasma has been performed. Vitamin E, selenium (gluthatione peroxidase) and fatty acids analysis has also been performed. The androstenone and skatole analysis in blood plasma collected before start of the experiment and just before slaughter has been analysed for a better evaluation of the boar taint aspects of the pig experiment.

**Table 9.** Example 2B design for the final feeding period of the 4 treatments.

| Treat ment | No. of entire male pigs | Food composition and energy level compared to "ad libitum" feeding[1] (100%) | Parasits (O.dentatu m and A.suum) | Bioactive feed |
|---|---|---|---|---|
| 1 | 8 | Control treatment 95% organic concentrate plus semi ad libitum clovergrass silage | Yes | |
| 2 | 8 | 70% organic concentrate plus chicory roots[2] (25%) (6 weeks prior to slaughter) | Yes | Chicory roots (2.6 kg per day the first week and 3.0 kg per day the rest of the experiment until slaughter) |
| 3 | 8 | 70% organic concentrate plus chicory dried roots[3] (25%) (6 weeks prior to slaughter) | Yes | Dried chicory roots (770 g per day the first week and 880 g per day the rest of the experiment until slaughter) |

(continued)

| Treat ment | No. of entire male pigs | Food composition and energy level compared to "ad libitum" feeding[1] (100%) | Parasits (O.dentatu m and A.suum) | Bioactive feed |
|---|---|---|---|---|
| 4 | 8 | 70% organic concentrate plus pure inulin[4] (6 weeks prior to slaughter) | Yes | Inulin (390 g per day the first week and 450 g per day the rest period until slaughter |

1: Energy level of the experiment is 95 % according to scale of Madsen et al., (1990).
2: The chicory roots (not dried) had a clear bitter taste.
3: The dried chicory roots had a clear sweet and bitter taste.
4: The pure inulin was totally without a bitter taste.

**Statistical analysis**

The statistical analyses were carried out with the Statistical Analysis System version 8.2 (SAS Institute, 1999-2001 by SAS Institute Inc., Cary, NC, USA). The GLM procedure was used to calculate the least squares means and standard error of the means for the skatole in blood plasma and backfat at slaughter and androstenone in blood plasma at slaughter. The models included the fixed effect of diet, replicate (litter) and slaughterday as well as interaction between diet and replicate and between diet and slaughter day (model 1).

$$Y = \mu + a_{diet} + b_{replicate} + c_{slaughterday} + ab_{diet*replicate} + ac_{diet*slaughterday} + e_{rror} \text{ (model 1)}$$

Y = skatole in blood and backfat and androstenone in blood plasma all at slaughter.

**Results**

The pigs ate the high amount of fresh and bitter blended chicory roots without problems after 1 week of adaptation by giving increasing amounts of chicory roots. The dried chicory roots were given without an adaptation period presumably because the dried chicory roots were less filling and had a sweet taste besides a bitter taste like the fresh chicory roots. The health status and production results of the chicory treatments were as good as the control treatment and especially the pigs fed dried chicory showed the same growth rate (daily gain) and feed conversion ratio as the control treatment and the lean meat content was not negatively influenced by feeding 25% chicory without supplementation with extra protein.

The effect of feeding 25% chicory roots fresh (treatment 2) and dried (treatment 3) plus 70% organic concentrate for six weeks on skatole and androstenone in blood plasma from Vena jugularis and skatole from backfat, and meat and eating quality have been compared with results of the control treatment (treatment 1), feeding 95% organic concentrate plus clover grass silage and (treatment 4) feeding 14% pure inulin corresponding to the amount in chicory plus 70% organic concentrate (see Table 9).

Irrespective of fresh or dried chicory, all chicory-fed entire male pigs showed skatole concentrations in blood plasma (after 6 weeks), which were not significantly different from zero in a statistical GLM analysis in SAS (see Table 10). Also the inulin fed showed a very significant decrease in skatole level compared to the control (treatment 1) (P<0.001). In Table 11 all chicory and inulin fed entire male pigs showed skatole concentrations in backfat (after 5-6 weeks), which were very significantly decreased compared to control fed (P<0.001). However, a decrease in the blood plasma androstenone level in treatment 3 and 4 compared with treatment 1 and 2 seems not to be significant.

**Table 10.** Skatole in blood at slaughter ($\mu$g/l) (Ismens and error)

| Treatment | N | LS Mean | Std. error | Pr > \|t\| |
|---|---|---|---|---|
| 1 Control | 8 | 3.49 | 0.3 | 0.0001 |
| 2 Fresh chicory | 8 | 0.32 | 0.3 | 0.2950 |
| 3 Dried chicory | 8 | 0.11 | 0.3 | 0.7068 |
| 4 Inulin | 8 | 0.68 | 0.3 | 0.0319 |

**Table 11.** Skatole in backfat (µg/g) (Ismeans and error)

| Treatment | N | LS Mean | Std. error | Pr > \|t\| |
|---|---|---|---|---|
| 1 Control | 8 | 0.088 | 0.007 | <.0001 |
| 2 Fresh chicory | 8 | 0.025 | 0.007 | 0.0009 |
| 3 Dried chicory | 8 | 0.02 | 0.007 | 0.0055 |
| 4 Inulin | 8 | 0.026 | 0.007 | 0.0005 |

**Example 2C**

**Effect of short time feeding dried chicory to entire male pigs**

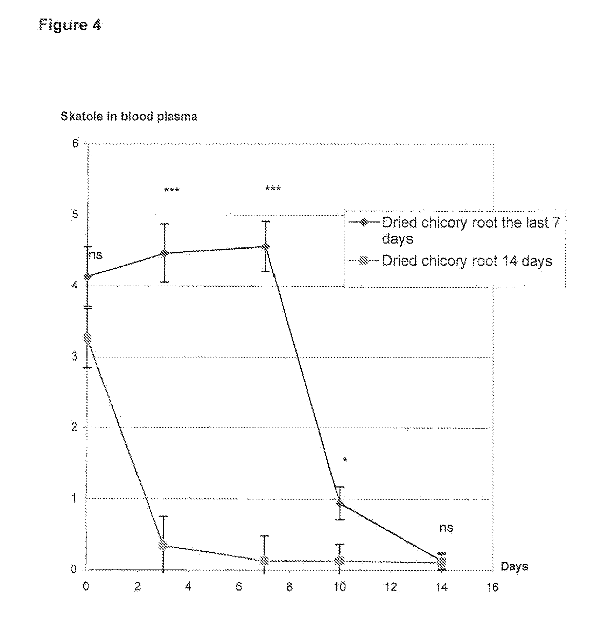

A short time experiment finishing feeding entire male pigs with dried chicory either one or two weeks before slaughter has been conducted. The 8 pigs fed dried chicory 14 days before slaughter began the chicory feeding day 0 (see Figure 4), while the 8 pigs fed dried chicory 7 days before slaughter began chicory feeding day 7 so that 16 entire male pigs were fed dried chicory the last 7 days before slaughter.

1. Finishing feeding of 8 individually kept entire male pigs at 70 % concentrate plus 25 % dried chicory for 3 days results in a highly significant decrease in blood plasma skatole levels (see Figure 4). Moreover, after a full week the skatole concentration was very close to zero both in blood plasma and backfat and the effect continued the second week too.

2. It was concluded that dried chicory, as it had a comparable effect to fresh chicory was the form of chicory that had the best potential for development to commercial product in terms of the economic and practical viability of the chicory root as a feedstuff ingredient. Furthermore the dried chicory root feed has the advantage that the pigs do not need an adaptation period before eating the full amount of 25% dried chicory roots on energy basis.

3. Thus, chicory feeding in the dried format provides a potentially viable solution to eradicating the consumer sensory off-flavour problem known as boartaint, in female, castrated male and more importantly in entire male pork meat.

**Example 3A**

**The effect of *Cichorium intybus* on helminth infections in pigs**

**Animals and infection**

Five groups of eight parasite naive pigs (four intact males and four females) were infected with 3000 *Oesophagostomum dentatum* L3-larvae while all the animals were on a diet of restricted concentrate + *ad lib* grass silage (week -4) (Table 12). Four weeks later (week 0), one group (infection control group) was slaughtered to assess if the worms had developed to the adult stage and to estimate worm establishment. The animals in the remaining four groups were moved to individual pens and some diets modified. Two groups continued on the concentrate + grass silage diet, while the other two groups were given either concentrate + roughly chopped chicory roots (long term chicory group, 9 weeks) (*Cichorium intybus* L. var. Orchies) or only concentrate (conventional control group). Five weeks later (week 5) one of the concentrate + grass silage groups had the silage changed to chicory (short term chicory group, 4 weeks), the second group remained on the concentrate + silage diet (organic control group) to the end of the experiment. The surviving pigs were infected a second time (week 7) with approximately 3000 *O. dentatum* and 2000 *Ascaris suum* eggs two weeks before slaughter for worm recovery (week 9). This was done to examine the effect of diet on both established (= 1st infection, adult worms at slaughter) and establishing (= 2nd infection, immature worms at slaughter) *O. dentatum.* Only the effect on establishing (immature) *A. suum* was investigated in this study.

The pigs used in the experiment were conventionally reared, but all experimental feeds were organically produced. According to the energy level in the feedstuffs 70% and 25% of the daily energy intake was based on concentrate and chicory roots, respectively (Table 12). The total amount of feed given was adjusted according to bodyweight once a week. The pigs fed chicory were adapted to the bitter taste of the root by increasing the chicory proportion to the desired 25% during the first week of the feeding period. At the beginning of the feeding period the long-term chicory group ate 2.1 kg roots and at the end they willingly ate up to 3.0 kg per day.

During the experiment the animals were weighed regularly and faecal samples were collected twice a week for quantification of parasite eggs using a concentration McMaster technique (Nansen & Roepstorff, 1998). Both species of parasite were recovered from the intestinal contents using an agar-gel technique (Slotved *et al.,* 1996 & 1997).

**Statistical analysis**

The area under the curve was calculated for different periods of the experiment to compare the egg excretion levels between groups. All data were analysed for an overall difference between groups by the Kruskall Wallis and for differences between individual groups by the Mann Whitney test using the software GraphPad Prism 3.0.

**Results**

At slaughter 4 weeks post infection the infection control group had a median worm burden (min-max) of 635 (60-2110). Almost all worms were adult. The population of adult and immature *O. dentatum* resulting from the first and second experimental infection, respectively, was easily differentiated in all the pigs at the end of the experiment.

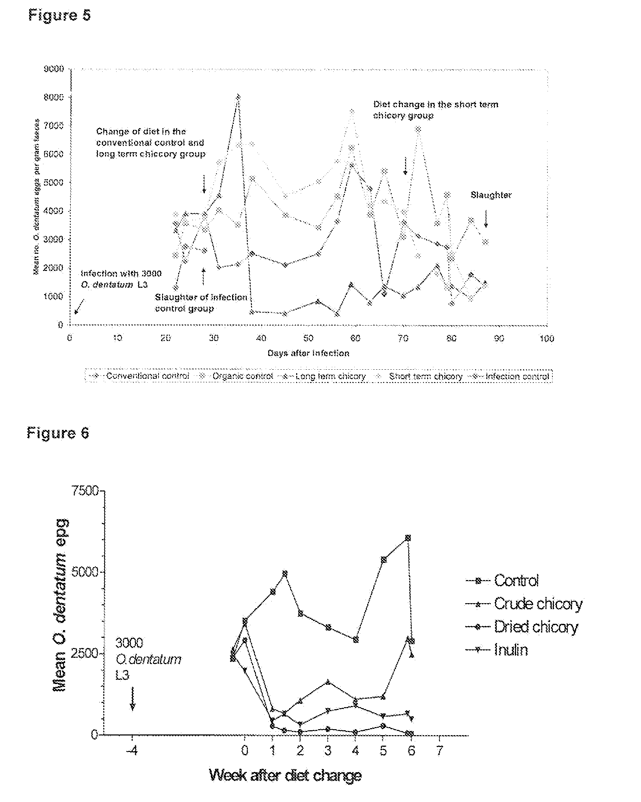

Ten days after the introduction of chicory, the long-term chicory group showed a large and rapid reduction in egg excretion compared to the other groups (Figure 5). Though increasing slightly, the egg counts remained at a low level during the remaining part of the experiment. Though a decrease in egg production was also seen in the short-term chicory group, both control groups also showed similar decreases. Overall, the egg excretion converged for all four groups towards the termination of the study. For the first 2½ weeks after the initial diet change the organic control and short term chicory group (both fed concentrate and grass silage in this period) had a higher egg excretion than the conventional control group. Overall, there were unusually large fluctuations in the egg excretion. As a result no statistical differences were detected despite apparent tendencies when comparing the area under the curve for the two control groups and the long term chicory group after the diet change week 0 (p=0.40). The same comparison for the two control groups and the short term chicory group after the diet change week 5 was also not significantly different (p=0.52). All eggs were produced by the adult *O. dentatum* that established after the first infection dose, as worms derived from the second infection dose did not fully mature.

At the end of the experiment there was no difference (p=0.86) in the populations of established adult *O. dentatum* in the four groups (see Table 13a and 13b). In contrast, compared to the organic control group, significantly less worms were able to establish in the intestine in both the short (p=0.04) and long-term chicory (p=0.002) groups. Only the long-term chicory group differed from the conventional control group (p=0.015). There was no difference between the conventional and the organic control groups (p=1.0). For both the infection and conventional control group there was an unusually large variation in the establishment of *O. dentatum.* This indicates that the same may be true for the other groups. It may be the result of the varying degrees of diarrhea that some pigs experienced in the first week of experiment when the first infection took place.

Overall, there was a statistical difference between the *A. suum* larval counts (Table 13a and 13b) between the groups (p=0.004). This is primarily due to a significantly smaller recovery of *A. suum* in the long-term chicory group compared to both the conventional (p=0.002) and organic control group (p=0.009). In addition, the short-term chicory group was close to being significantly different from the conventional (p=0.054) and organic control group (p=0.053). No other differences were found. At slaughter four out of the eight long term chicory pigs had a total of 10 adult *A. suum* (5-15 cm) and one pig in the short term chicory group had 1 *A. suum* (3 cm). All 11 worms were older than two weeks and thus not derived from the experimental infections. These worms may be the result of *A. suum* contamination of the chicory roots and this contamination may have affected parasite establishment.

Production results were satisfactory and identical in all groups, the pigs increasing their mean bodyweight from 55 kg to 120 kg during the experiment. Analysis of the chicory roots showed an inulin content of approximately 150g/kg fresh root.

**Table 12.** Diet composition for five groups of pigs. The proportion of feed type is given as % of the daily energy requirement per animal.

| Group | Week post first infection | | |
| | -4-0 | 0-5 | 5-9 |
| --- | --- | --- | --- |
| Infection control | 100% concentrate semi *ad lib* grass silage | | |
| "Conventional" control with organic concentrate minus roughage | 100% concentrate semi *ad lib* grass silage | 100% concentrate | 100% concentrate |
| Organic control | 100% concentrate semi *ad lib* grass silage | 95 % concentrate semi ad lib grass silage | 95 % concentrate semi ad lib grass silage |
| Chicory, short term | 100% concentrate semi *ad lib* grass silage | 95 % concentrate semi ad lib grass silage | 70 % concentrate 25 % chicory roots |

(continued)

| | Week post first infection | | |
|---|---|---|---|
| Group | -4-0 | 0-5 | 5-9 |
| Chicory, long term | 100% concentrate semi *ad lib* grass silage | 70 % concentrate 25 % chicory roots | 70 % concentrate 25 % chicory roots |

**Table 13a.** Mean worm burden ± SD of *O. dentatum* and *A. suum* in groups of pigs fed different diets. The pigs were infected twice with 3000 *O. dentatum* L3-larvae (11 weeks apart) and once with 2000 *A. suum* eggs. The age of the adult *O. dentatum* populations and the immature *O. dentatum*/*A. suum* populations, are 13 and 2 weeks, respectively.

| | | *O. dentatum* | | *A. suum* Immature |
|---|---|---|---|---|
| Group | n | Adult | Immature | |
| "Conventional" control with organic concentrate minus roughage | 8 | 1043 ± 975 | 2893 ± 597 | 1072 ± 450 |
| Organic control | 8 | 1281 ± 994 | 3034 ± 479 | 1026 ± 464 |
| Short term chicory | 8 | 989 ± 379 | 2450 ± 469 | 556 ± 302 |
| Long term chicory | 8 | 810 ± 515 | 2017 ± 454 | 288 ± 144 |

**Table 13b.** Median worm burden (min-max) of *Oesophagostomum dentatum* and *Ascaris suum* in groups of pigs fed different diets. The pigs were infected twice with 3000 *O. dentatum* L3-larvae (11 weeks apart) and once with 2000 *A. suum* eggs. The age of the adult *O. dentatum* populations and the immature *O. dentatum*/*A. suum* populations, are 13 and 2 weeks, respectively.

| | | *O. dentatum* | | *A. suum* Immature |
|---|---|---|---|---|
| Group | n | Adult | Immature | |
| "Conventional" control with organic concentrate minus roughage | 8 | 433 (160-2539) | 2940 (1586-3546) | 1076 (330-1730) |
| Organic control | 8 | 1139 (42-2854) | 3184 (2295-3705) | 960 (170-1550) |
| Short term chicory | 8 | 1097 (337-1566) | 2321 (1774-3313) | 510 (150-1070) |
| Long term chicory | 8 | 702 (51-1731) | 2007 (1380-2890) | 315 (85-475) |

**Conclusions**

Feeding of pigs with crude chicory can result in a reduced establishment of *O. dentatum* and perhaps of *A. suum.* Furthermore, the egg production of *O. dentatum* may be reduced.

**Example 3B**

**The effect of crude and dried chicory roots on helminth infections in pigs**

**Materials and methods**

A total of 32 entire male pigs were allocated to four groups of eight animals according to live weight and litter (for further details on animals see example 2b). The pigs were parasite free and kept in individual pens. While on a diet of organically produced concentrate and semi *ad libitum* clover grass silage all pigs were infected with 3000 *O. dentatum* L$_3$-larvae four weeks before changing the diet (week -4) using a stomach tube. Four weeks later, when the *O. dentatum* larvae should have had time to mature into adult worms, the diet was changed for 3 of the groups while the fourth remained on the diet of concentrate and silage (control group) (week 0). The other 3 diets consisted of concentrate and either roughly chopped crude chicory roots (crude chicory group), finely chopped dried chicory roots (dried chicory group), or chemically purified inulin (Raftiline®) (inulin group)(table 14 and 15). The chicory was grown in a field that had not been fertilised with pig manure for many years. Four weeks after the diet change all pigs were infected with 2000 *A. suum* eggs and 3000 *O. dentatum* $L_3$-larvae (week 4). On day 13 and 15 after the second infection (week 6) four pigs from each group were slaughtered for recovery of established adult worms (1st infection) and establishing immature worms (2nd infection) from subsamples of intestinal contents using an agar-gel technique (Slotved *et al.,* 1996 & 1997). The developmental stage and species of the worms was determined and 10 (*O.dentatum*) -15 (*A.suum*) randomly selected worms were measured for each parasite species and stage. Excretion of *O. dentatum* eggs was followed by regular collection of faecal samples from the pigs. The samples were analysed using a concentration Mc-Master technique (Nansen & Roepstorff, 1998). Chrome was added to the feed for the last two weeks up to slaughter. Faecal samples where then collected and pooled for the last three days before slaughter. Wet faecal and diet samples were analysed for chrome content using the method of Schürch, Loyd & Crampton (1950).

**Table14.** Feeding schedule for four groups of pigs (g wet matter/day).

| Group | Diet | | |
|---|---|---|---|
| | Concentrate | Supplement[a] | Total |
| **Control:** | | | |
| Week 1 | 2710 | Semi *ad libitum* | 2710 |
| Week 2 | 2850 | Semi *ad libitum* | 2850 |
| Week 3 | 2950 | Semi *ad libitum* | 2950 |
| Week 4 | 3080 | Semi *ad libitum* | 3080 |
| **Week 5-6** | 3140 | Semi *ad libitum* | 3140 |
| **Crude chicory:** | | | |
| Week 1 | 2000 | 2600 | 4600 |
| Week 2 | 2100 | 3000 | 5100 |
| Week 3 | 2200 | 3000 | 5200 |
| Week 4-6 | 2300 | 3000 | 5300 |
| **Dried chicory:** | | | |
| Week 1 | 2000 | 770 | 2770 |
| Week 2 | 2100 | 880 | 2980 |
| Week 3 | 2200 | 880 | 3080 |
| Week 4-6 | 2300 | 880 | 3180 |
| **Inulin:** | | | |
| Week 1 | 2000 | 390 | 2390 |
| Week 2 | 2100 | 450 | 2550 |
| Week 3 | 2200 | 450 | 2650 |
| Week 4-6 | 2300 | 450 | 2750 |

[a] Silage, fresh chicory roots, dried chicory roots or inulin

Diet samples were also collected at the time of slaughter and frieze-dried before chemical analysis. Protein was determined according to the Kjeldahl method using a Kjeltec autosampler system 1035 (Foss Tecator, Höganäs, Sweden). Fat (hydrochloric acid-fat) was extracted with diethyl ether after acid hydrolysis (Stoldt, 1952) and ash analysed using the AOAC method (Association of Official Analytical Chemists, 1990). Fructans were determined as described by Bach Knudsen & Hessov (1995) while analysis for starch was done by an enzymatic colorimetric method and non-starch polysaccharides (NSP) by an enzymatic-chemical method (Bach Knudsen, 1997). Sugars (glucose, fructose, sucrose and fructans were determined using a modification of the enzymatic-colorimetric method of Larsson & Bengtsson (1983). Two parallel samples were extracted by either acetate buffer or acetate buffer containing 5 U/mg sample β-fructosidase (EC 3.2.1.26, Roch Diagnostics GmbH, Mannheim, Germany) and used to estimate free glucose, fructose and sucrose respectively. The acetate buffer extract was further hydrolysed by sulphuric acid (0.037 mol/L, 80 °C, 70 min.) and the released glucose and fructose quantified as for free glucose and fructose. The difference in sucrose between the measurements without and with β-fructosidase was added to the fructans. Starch was analysed by an enzymatic-colorimetric method and non-starch polysaccharides (NSP) by an enzymatic-chemical method (Bach Knudsen, 1997). Klason lignin was measured gravimetrically as the insoluble residue after 12 M sulphuric acid treatment (Theander *et al.* 1994) and crude fibre according to the Weende-method as described by Hansen & Sørensen (1996). All samples were analysed in duplicate. Feed units were calculated according to Boisen & Fernandez (1998).

**Table 15.** Composition of the diets (concentrate+supplement of silage, crude chicory, dried chicory or inulin) given to four groups of pigs. Silage was given semi *ad libitum* to the control group but was rarely eaten and is therefore not included.

| | Group | | | |
|---|---|---|---|---|
| | Control | Crude chicory | Dried chicory | Inulin |
| Dry matter (%) | 88[a] | 88[a] | 90[c] | 90[d] |
| | | 25[b] | | |
| *g/kg wet matter* | | | | |
| Crude chicory | 0 | 566 | 0 | 0 |
| Dried chicory | 0 | 0 | 276 | 0 |
| Inulin (Raftiline®) | 0 | 0 | 0 | 163 |
| Rape seed cake | 145 | 63 | 105 | 121 |
| Peas | 240 | 104 | 173 | 200 |
| Wheat | 223 | 97 | 161 | 186 |
| Barley | 220 | 95 | 159 | 184 |
| Oat | 50 | 22 | 36 | 42 |
| Soybean | 100 | 43 | 72 | 83 |
| Salt | 4 | 2 | 3 | 3 |
| Chalk | 12 | 5 | 9 | 10 |
| Monocalciumphosphate | 4 | 2 | 3 | 3 |
| Solivit Micro-59 | 2 | 1 | 1 | 2 |
| Marker (Chromium oxide) | 2 | 1 | 2 | 2 |

[a] concentrate
[b] crude chicory roots
[c] concentrate mixed with dried chicory roots
[b] concentrate mixed with inulin

**Statistical analysis**

The area under the curve was calculated for individual pigs to compare the levels of *O. dentatum* egg excretion. Most comparisons of all four groups have been done using the Kruskall-Wallis test while pair-wise comparisons have been done using the Mann Whitney U-test. The exception is the body length data for *A.suum* as these were successfully log-transformed to normality and thereafter tested by a parametric GLM model in Statistical Analysis System version 8.2 (SAS Institute, 1999-2001 by SAS Institute Inc., Cary, NC, USA). Not all body length data for *O.dentatum* could be log-transformed and all data have therefore been tested using non-parametric tests. Analysis of the length of all immature worms was carried out separately for animals that were slaughtered day 13 and 15 after the second infection as the larvae are know to grow considerably during the two day interval.

**Results and discussion**

Chemical analysis of the diets showed that the most marked difference between the control diet and the three experimental diets was their level of fructan (low molecular (LM) sugars such as inulin)(table 16). The overall concentration of fructan in the diets were such that the individual pigs were given a total of 36 g, 429 g, 446 g and 428 g per day (dry matter) in the control, crude chicory, dried chicory and inulin groups, respectively.

There was no difference between the four groups with respect to *O. dentatum* egg excretion up to the point when the experimental diets were introduced (p=0.9). However, within a week after the introduction of the diets the egg counts had dropped drastically in the inulin, crude and dried chicory groups compared to the control group (Figure 6). Thereafter, the egg counts in the crude chicory group increased again with time and ended at the same level at slaughter as the control group, while the egg excretion remained depressed in the inulin group and especially in the dried chicory group. Still, for the entire period after the feed change the control group had an overall higher egg excretion than in the inulin, crude and dried chicory groups (p=0.0006, p=0.002 and p=0.0002, respectively). In addition, the crude chicory group differed from both the dried chicory group (p=0.0003) and the inulin group (p=0.02). There was no significant difference between the dried chicory and inulin group.

**Table 16.** Chemical analysis of the diets (concentrate plus supplement of silage, crude chicory, dried chicory or inulin) given to four groups of pigs at the time of slaughter. The control group only ate their concentrate and the silage component is therefore not included in the analysis. Data in brackets denote the fraction of non-cellulosic polysaccharides that was insoluble.

| | | Group | | | |
|---|---|---|---|---|---|
| | | Control | Crude chicory | Dried Chicory | Inulin |
| *g/kg dry matter* | | | | | |
| Protein | | 197 | 163 | 157 | 165 |
| Fat | | 68 | 55 | 50 | 54 |
| Ash | | 57 | 54 | 57 | 50 |
| Crude fibre | | 61 | 58 | 60 | 38 |
| Low molecular sugars: | | | | | |
| | Glucose | 1 | 1 | 2 | 1 |
| | Fructose | <1 | 3 | 16 | <1 |
| | Sucrose | 29 | 69 | 62 | 24 |
| | Fructan (inulin) | 13 | 140 | 156 | 173 |
| Total LMS | | 43 | 213 | 236 | 198 |
| Starch | | 379 | 287 | 263 | 318 |
| Dietary fibres | | | | | |
| Non-starch polysaccharides: | | | | | |
| | Cellulose | 44 | 41 | 45 | 42 |
| Non-cellulosic polysaccharides: | | | | | |
| | Rhamnose | 1 (0) | 1 (0) | 2 (1) | 1 (1) |
| | Fucose | 1 (0) | 1 (0) | 1 (0) | 1 (0) |
| | Arabinose | 30 (9) | 27(10) | 27 (12) | 27 (10) |
| | Xylose | 31 (5) | 25 (4) | 23 (3) | 31 (5) |
| | Mannose | 3 (1) | 3 (1) | 3 (1) | 3 (1) |
| | Galactose | 12 (6) | 12 (6) | 12 (7) | 11 (5) |
| | Glucose | 17 (9) | 18 (7) | 14 (2) | 17 (5) |
| | Uronic acids | 18 (1) | 15 (1) | 33 (25) | 16 (8) |
| | Total NCP | 113 (31) | 101 (30) | 115 (51) | 107 (35) |
| Total NSP | | 158 | 142 | 160 | 148 |
| Klason lignin | | 49 | 46 | 34 | 37 |
| Total dietary fibres | | 207 | 188 | 193 | 185 |
| Feed units/kg dry matter | | 1.14 | 1.15 | 1.14 | 1.13 |

The total feed volume was very high in the crude chicory group and this may have led to a dilution of the parasite eggs and thereby exaggerated the apparent depression of egg excretion. The same problem should not be as marked for the inulin and dried chicory groups. To assess the differences in faecal output, chrome was added to the concentrate given to the pigs for the last 2 weeks before slaughter. By analysing the chrome content in both faeces and feed an estimate of the total faecal output per day was calculated for four pigs per group. Due to a high water content (75%) in the crude chicory roots the total faecal volume in the crude chicory group was comparable to both the control and the dried chicory group. The silage given to the pigs has not been included in the calculations as very little of it was actually eaten. The total faecal volume was lower in the inulin group than the other three groups. Still, statistical analysis showed a difference between the four groups with respect to the estimated total number of eggs excreted by the pigs at slaughter (p=0.007) (table 17). The egg excretion in the dried chicory group was lower than all other groups (p=0.03 in all cases), while the inulin group also differed from the control group (p=0.03). As dry matter content of the collected faecal samples was similar in the four groups, correction for differences in dry matter does not change the relative egg excretion patterns.

**Table 17.** Median number (with min and max) of *Oesophagostomum. dentatum* eggs excreted per gram pig faeces (wet weight) at slaughter per female *O. dentatum* recovered at slaughter and the estimated total egg excretion at slaughter per in groups of pigs fed different diets.

| | Eggs per g faeces per female worm | | Total *O. dentatum* egg excretion per day | |
|---|---|---|---|---|
| Group | n | Median (min-max) | n | Median (min-max) |
| Control | 8 | 3.41 (1.72-8.92 ) | 4 | $12.2\times10^6$ (8.0-27.8 $\times10^6$) |
| Crude chicory | 8 | 2.48 (0.13-5.71) | 4 | $5.9\times10^6$ (0.3-12.6 $\times10^6$) |
| Dried chicory | 8 | 0.06 (0.01-0.38) | 4 | $0.17\times10^6$ (0.08-0.21 $\times10^6$) |
| Inulin | 8 | 0.80 (0.11-2.93) | 4 | $1.4\times10^6$ (1.1-1.9 $\times10^6$) |

The estimated number of eggs excreted in the faeces by each female *O. dentatum* found at slaughter (table 17) was significantly different in the four groups (p=0.0001). Further analysis showed significantly lower values in the dried chicory group compared to the control (p=0.0002), crude chicory (p=0.0006) and the inulin group (p=0.005), while the inulin group also differed from the control group (p=0.001).

Ignoring the lack of overall difference (p=0.31) between the groups with respect to their populations of established adult *O. dentatum* (table 18), pair-wise comparison of the three experimental groups with the control group showed only that the inulin group was significantly different (p=0.04). In contrast, significantly fewer immature worms were recovered in the dried chicory group compared to the control group (p=0.0006), the crude chicory group (p=0.003) and the inulin group (p=0.005). It was also found that the immature larvae in the dried chicory group apparently did not develop from the $L_4$-stage to the final $L_5$-stage, which is the stage that matures with time into the adult worms. This development took place in all other groups, while only $L_4$-larvae and no $L_5$-larvae were found in the dried chicory group. This may be because the worms did not establish well in pigs fed dried chicory and/or that the worms were delayed in their development. The lifecycle of this parasite involves a tissue dwelling stage ($L_4$-larvae) and the changes in intestinal environment due to the diet may cause the worms to remain longer in and emerge later from the intestinal tissues.

**Table 18.** Median worm burden (with min and max) of immature *Ascaris. suum* (infection dose = 2000 eggs/pig) and immature ($L_4$- and $L_5$-larvae) and adult Oeoso*phagostomum. dentatum* (infection dose = 3000 larvae/pig twice eight weeks apart) in four groups of pigs fed different diets. The age of the immature and adult worms is 2 and 10 weeks, respectively.

| | | *O. dentatum* | | *A. suum* |
|---|---|---|---|---|
| Group | N | Adult | Immature | Immature |
| Control | 8 | 2523 (1334-2981) | 2281 (1916-2837) | 613 (360-1190) |
| Crude chicory | 8 | 1864 (1669-2660) | 2009 (1465-2561) | 275 (135-825) |
| Dried chicory | 8 | 2062 (177-2809) | 408 (123-2015) | 165 (190-685) |
| Inulin | 8 | 1924 (569-2336) | 2222 (428-2512) | 141 (145-535) |

Statistical comparison of all groups showed that the length of both male and female *O. dentatum* was different between groups (p=0.014 and p=0.002, respectively)(table 19). With respect to the males, the control group differed from the crude chicory group (p=0.021), the dried chicory group (p=0.003) and the inulin group (p=0.021), while only females from the dried chicory group were different from those of the control group (p=0.005). The immature *O. dentatum* populations were separated into subpopulations of $L_4$-larvae and $L_5$-larvae that were tested separately. No significant differences were detected for the $L_5$-larvae but the $L_4$-larvae varied significantly when comparing all groups on both day 13 (p=0.019) and 15 (0.045) after the second infection. This was due to the shorter length of the larvae in the dried chicory group compared to the control group (p=0.03 in both cases)(table 20).

**Table 19.** Median length in mm (min-max) of adult *Oesophagostomum dentatum* recovered week 10 post infection from pigs fed different diets the last 6 weeks of infection.

| Group | Males | Females |
|---|---|---|
| Control | 9.79 (8.96-10.05) | 12.35 (11.46-13.29) |
| Crude chicory | 9.49 (8.90-9.74) | 11.76 (10.81-13.25) |
| Dried chicory | 9.19 (8.56-9.58) | 11.08 (10.42-12.50) |
| Inulin | 9.47 (9.14-9.80) | 11.65 (11.02-12.63) |

**Table 20.** Median length in mm (min-max) of immature *Oesophagostomum dentatum* recovered day 13 (n=4) and 15 (n=4) post infection (pi) from pigs fed different diets.

| Group | $L_4$-larvae | | $L_5$-larvae | | | |
|---|---|---|---|---|---|---|
| | | | Males | | Females | |
| | Day 13 pi | Day 15 pi | Day 13 pi | Day 15 pi | Day 13 pi | Day 15 pi |
| Control | 3.70 (3.34-3.85) | 3.46 (2.21-3.81) | 4.54 (3.90-4.80) | 5.89 (4.87-6.33) | 4.75 (4.46-5.85) | 7.46 (6.50-7.47) |
| Crude chicory | 3.55 (3.36-3.64) | 3.40 (2.69-3.69) | 4.17 (4.14-4.89) | 5.70 (4.90-6.41) | 4.89 (4.48-5.44) | 6.91 (5.16-7.33) |
| Dried chicory | 2.65 (2.10-2.91) | 2.54 (2.31-2.76) | | | | |
| Inulin | 2.98 (2.25-3.36) | 3.38 (3.31-3.89) | 4.15 (3.96-4.33) | 5.51 (5.11-5.91) | 4.82 (4.20-5.03) | 6.34 (5.49-6.89) |

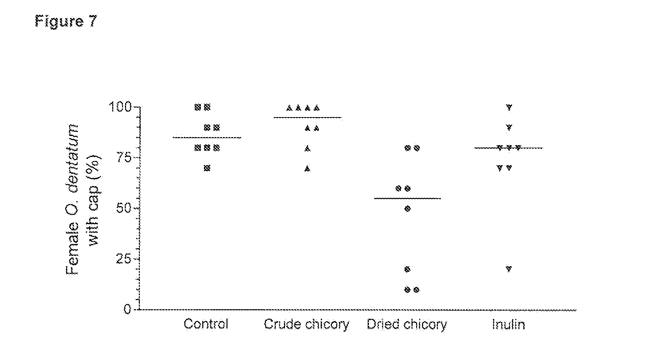

When *O. dentatum* worms mate the male leaves a "cement cap" encasing the genital area of the female. When comparing the fraction of females per pig with such a cap in the four groups (figure 7) there was an overall significant difference between groups (p = 0.0024). Pair-wise comparison of the groups revealed that the dried chicory group alone was different as it differed significantly from the groups given control (p = 0.003), crude chicory (p = 0.001) or inulin (p = 0.038), indicating that less female worms had a cap in the group fed dried chicory and that the mating frequency was reduced.

**Table 21.** Mean length in mm ($\pm$ SD) of immature *Ascaris suis* recovered day 13 (n=4) and 15 (n=4) post infection (pi) from pigs fed different diets.

| Group | Day 13 pi | Day 15 pi |
|---|---|---|
| Control | 2.46 $\pm$ 0.28 | 3.85 $\pm$ 0.68 |
| Crude chicory | 2.22 $\pm$ 0.27 | 3.77 $\pm$ 0.98 |
| Dried chicory | 2.13 $\pm$ 0.08 | 3.27 $\pm$ 0.47 |
| Inulin | 1.99 $\pm$ 0.17 | 3.65 $\pm$ 0.57 |

For *A. suum,* there were more larvae present in the pigs from the control group compared to the crude chicory group (p=0.015), the dried chicory group (p=0.015) and the inulin group (p=0.002) (table 18). The inulin group was only just significantly different from the dried chicory group (p=0.05). The length of the larvae was significantly different in the four groups on both day 13 and 15 after the second infection (p<0.0001 in both cases). Furthermore, the larvae recovered from the inulin group (p<0.0001), crude chicory group (p=0.005) and dried chicory group (p<0.0001) were significantly shorter than the larvae from the silage group. The larvae in the inulin group also proved to differ from the crude (p=0.0009) and dried chicory (p=0.045) groups. By day 15 post infection only the larvae in the dried chicory group remained smaller than the control group (p=0.0002), and they were also smaller than the larvae from the crude chicory group (p=0.003) and the inulin group (p=0.01). The largest overall reductions in larvae size were 20% for the inulin group (day 13) and 15% for the dried chicory group (day 15) (table 21).

**Conclusions and implications**

The results indicate that the dried chicory root is a much more effective product than the crude chicory root when given at the same level (app. 16% and 14% of dry matter, respectively). The dried roots significantly reduced not only the number of establishing *O. dentatum* and *A. suum* but also affected their growth and development. The population of adult established *O. dentatum* was not reduced by either diet although the egg excretion of the female worms at slaughter was markedly depressed by approximately 41 % and 99% in the crude and dried chicory group, respectively. Particularly in the dried chicory group where not only the egg production but perhaps also the mating frequency may have been impaired and the size of the worms reduced. The reason for the difference between the two diets may be that the dried and finely chopped roots were more readily digested and its active components released in higher quantities compared to the roughly chopped crude roots. The crude chicory diet was also more voluminous (according to wet matter) than the dried diet and the fructan may therefore have been more diluted although total intake of dry matter was the same in both groups.

Although there was a significant reduction in egg excretion, the overall effect of the purified inulin was not as marked as that of the dried chicory diet. This is surprising as the same purified inulin product has previously been shown to be very effective against *O. dentatum* (Petkevič ius, 2003). The reason may be that the study used a specially constructed diet (that may also have had an effect) with added inulin and not a standard commercial diet as in the present trial. The diet containing purified inulin generally had a larger effect than the diet including crude chicory roots.

The results from the present trial also show that all 3 experimental diets had reduced the establishment of *A. suum,* especially the inulin and dried chicory diet.

Overall, the diet containing dried chicory roots was considered to be the most effective of the three experimental diets reducing parasite establishment and egg production more severely than the other diets.

**Example 4A**

**Sensory profiling of the effects of silage and chicory (bioactive) feeding on boar-taint in cooked pork**

**Sensory characteristics**

Analytical chemists engaged in elucidating boar-taint require clearly defined terminology to describe the sensory characteristics that constitute boar-taint as it is in essence a sensory based off-flavour phenomenon. The development of such descriptors with definitions and references by sensory analysis has much potential in the further elucidation of sensory boar-taint perception and its level of negative effect on consumer acceptability of pork (Bonneau et al., 2000; Dijksterhuis et al., 2000). Sensory profiling, a method by which a panel uses a developed sensory vocabulary to describe perceived sensory characteristics in a sample set has been utilised in the present research (ISO, 1985; ISO, 1994; Meilgaard, et al., 1999; Byrne et al., 2001 b). The resultant profile is a perceptual map of the variations in a sample type that can be employed alone or in combination with chemical/instrumental measurements in the explanation and elucidation of underlying sensory and chemical relationships.

The objectives of the present study were to investigate the sensory variation that resulted from the effects of bioactive (silage and chicory) feeding in organically produced male cooked pork. Of particular interest was the effect of bioactive feeding on the 'off-flavour' referred to as boar-taint in the meat. To achieve these aims a descriptive sensory vocabulary was developed with an expert sensory panel and subsequently the panel were utilised to develop a sensory profile for the meat samples, derived from male animals fed various levels of silage and chicory. In the analyses of the sensory profiling data a strategy involving multivariate Principal Component Analysis was utilised to determine precisely how the various feeding treatments were described and discriminated from a sensory perspective.

**Meat preparation**

Pork muscles *Longissimus dorsi* (LD) were used for a sensory analysis. Batches of four muscles each batch from a different animal litter (4 male littermates in each) was obtained. Each of the four muscles in a batch, were from an animal subjected fed one of four treatments prior to slaughter (Table 4 of example 2A, although the female were not analysed).

All muscles were stored vacuum packed in darkness at -20°C. Muscles were held at 4°C for approx. 12 h prior to handling to allow ease of cutting and grinding. Visible fat and connective tissues were removed and muscles were cut into cubes (approx. 3 cm$^3$) and mixed thoroughly. Muscles from a specific treatment were utilised, and mixed together thoroughly once cubed. Each treatment batch of muscle cubes was ground in a rotary screw mincer (Model X 70, Scharfen GmbH & Co. Maschinenfabrik KG, Germany) through a 4.5 mm plate. The minced samples were shaped into patties of 100 g and approx. 1 cm thickness using a commercial pattie maker (i.d. 9 cm). Plastic packaging film was used in the making of the patties to help maintain their shape prior to vacuum bagging. Patties were subsequently removed from their plastic film wrapping and vacuum packed in oxygen impermeable plastic laminate bags. The vacuum-packed patties were then frozen at -30°C and stored for up to a week.

Prior to heat treatment, all patties were placed in a 25°C water bath until a core temperature of between 18 and 20°C had been reached. Subsequently patties were removed from their plastic vacuum bags and batch cooked in convection ovens set to 150°C. The ovens utilised were determined to have comparable heating cycles. The heating/cooking process took a total of 20 min and was carried out as per Byrne et al. (1999b). In each oven, a control patties core temperature was monitored throughout the heat treatment by a termocouple and data logger (Squirrel Series 1000, Grant Instruments Ltd., United Kingdom). The final internal temperature reached over all pattie batches was found to vary between 78 and 82°C. After cooking the samples were cooled to 5°C in oxygen impermeable plastic laminate bags for a short period (10-15 min) prior to reheating for sensory assessment.

To prepare the samples for descriptive vocabulary development and sensory profiling, patties were divided into 8 equal triangular pieces, which were then vacuum packaged in plastic laminate bags. These were placed in a steel tray filled with water at ambient temperature. For reheating the tray was placed in a convection oven at 140°C for 19 min. The mean serving temperature of the vacuum packed samples was 65°C.

## Sensory measurements

Prior to sensory profiling a sensory panel (8 persons) participated in the development of a sensory vocabulary to describe and discriminate the effects of conventional and bioactive feeding on the general flavour and in particular boar-taint in the pork meat of the present study (see Byrne et al., 1999a,b; Byrne et al., 2001a). The panel was recruited from the public and students of the Royal Veterinary and Agricultural University, Frederiksberg, Denmark. All sensory work was carried out in the sensory laboratory at the University, which fulfils requirements according to the international standards (ASTM, 1986; ISO, 1988).

Panel input, panel leader input, and multivariate statistical analyses were utilised to select a set of 24 descriptors plus an acceptability question from initial list of 32 terms (see Byrne et al., 2001 a). Each of the final list of terms was defined by a reference material and terms were divided into their mode of sensory assessment, i.e. odours, tastes, flavours and aftertastes (Table 22).

**Table 22.** List of 25 sensory descriptive terms with definitions developed for the evaluation of pork meat, oven cooked at 150°C for 20min., derived from male animals fed bioactive compounds, silage and chicory.

| Term[a][b] | Definitions and reference materiais[c] |
| --- | --- |
| **Odour** | **Odour associated with:** |
| 1.Piggy/Animaly-O | cooked pork containing boar-taint/dilute skatole solution |
| 2.Meat/Gamey-O | cooked game meat/wild boar |
| 3.Cardboard -O | wet cardboard |
| 4.Fresh cooked pork meat -O | oven cooked pork meat with on surface browning |
| 5.Linseed oil-O | warmed linseed oil/linseed oil based paint |
| **Taste** | **Taste sensation associated with:** |
| | sucrose, 1g/l solution in water[c] |
| 7.Sour-T | citric acid (monohydrate) 0.3g/l solution in water |
| 8.Salt-T | sodium chloride, 0.5g/l solution in water |
| 9. Bitter-T | quinine chloride, 0.05g/l solution in water |
| **Flavour** | **Aromatic taste sensation associated with:** |
| 10. Piggy/Animaly-F | cooked pork containing boar-taint/diluted skatole solution |
| 11. Metallic-F | ferrous sulphate, 0.1g/l solution in water |
| 12. Meat/Gamey-F | cooked game meat/wild boar |
| 13. Herby-F | dried mixed herbs |

(continued)

| Term[a][b] | Definitions and reference materials[c] |
|---|---|
| 14. Spicy-F | mixed spices |
| 15. Cooked Ham-F | cooked ham |
| 16. Fresh cooked pork meat -F | oven cooked pork meat with no surface browning |
| 17. Cardboard-F | wet cardboard |
| 18. Lactic/Fresh sour -F | natural yoghurt |
| 19. White pepper-F | white pepper |
| 20. Pork fat-F | cooked pork fat |
| **Aftertaste** | **Feeling factor in the oral cavity associated with:** |
| 21. Lactic/Fresh sour -AT | natural yoghurt |
| 22. Astringent-AT | aluminium sulphate 0.02g/l solution in water |
| 23. Spicy/heat-AT | mild warming effect of spices |
| 24. Chemical medicinal | cough syrup |
| 25. Fatty mouth coating-AT | a residual coating of fat after sample assessment |
| 26. Acceptibility | how acceptable do you find the sample? |

[a] Suffix to sensory terms indicates method of assessment by panellists; -O = Odour, -F = Flavour,-T = Taste, -AT = Aftertaste.

[b] Concentrations in g/l were devised to ensure panellists' could recognise clearly the sensory note involved.

[c] Definitions of sensory terms as derived during vocabulary development.

A sensory profile was developed for the pork patties for each of the 4 feeding treatments using the same 8-member paid panel as utilised in vocabulary development. The sample set presented at the profiling study contained the four treatments. This sample set (4) was assessed by each of the 8 panel assessors 4 times, as replicates ($4 \times 8 \times 4$) = 128 'objects' in the profile data set for each of the 25 sensory descriptors. Each replicate was presented on each of 4 days to each panellist, 4 samples per day. In all 4 days of panel sessions of 1.5 hr each were carried out in the development of the profile. Presentation to individual panellists on each day of profiling was in a randomised order. However, the full range of storage days and feeding treatments was included on each day.

Quantitative data was collected using the FIZZ Network data acquisition software (BIOSYSTEMS, Couternon, France). Unstructured line scales of 15 cm anchored on the left side by the term 'none' and on the right side by the term 'extreme' were used for the scoring of each sensory term (Meilgaard et al., 1999).

All multivariate analyses were performed using the Unscrambler Software, Version 7.5 (CAMO ASA, Trondheim, Norway). In PCA analysis, data were analysed, centred with full cross-validation.

## Results

Multivariate Principal Component Analysis was used to gain a qualitative overview of the relationships within the sensory data and the association of the descriptors with the experimental design variables, i.e. non-bioactive/control, silage, chicory/silage and chicory feeding.

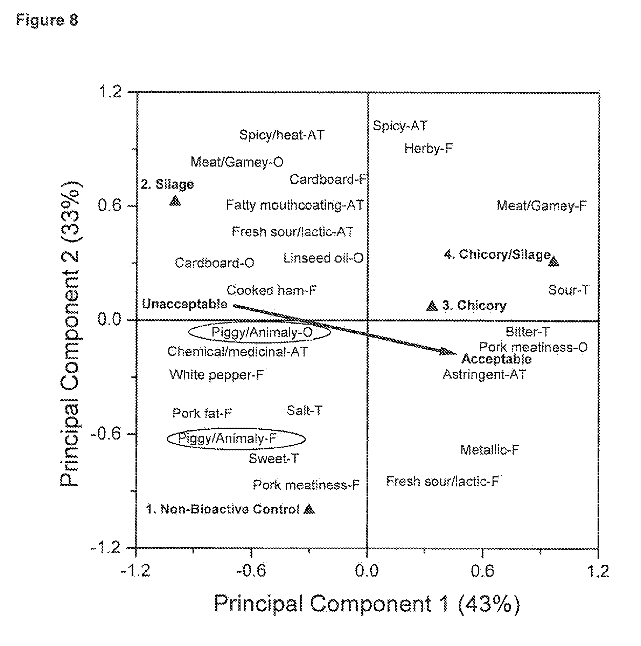

A sensory profiling of cooked pork derived from male animals was illustrated by Principal Component Analysis (PCA) plot (Figure 8). PCA was found present 2 significant Principal Components (PCs). PC1 and PC2 explaining 43 and 33% of the explained variation, respectively.

The general sensory description of the feeding treatments is shown in Figure 8.

## Experiment 4a

### *Longissimus dorsi 1 (LD1)*

1. Non-bioactive control diet (100% organic concentrates):
These samples were described by pork meatiness-flavour, sweet-taste, pork fat-flavour, salt-taste. These are typical 'fresh' and sweet meaty attributes of conventional feeding (Byrne et al., 2001 b). However, associated with this aspect of the samples was a high level of boar-taint as described by Piggy/Animaly-flavour and odour.

2. Control + silage:

These samples appear not as 'fresh' in relation to meatiness as control diet and contain a number of off-flavours, i.e. cardboard odour/flavour and linseed oil-like odour.

3. and 4. Control + chicory and control +silage +chicory, respectively:

These samples are described as having pork meatiness-odour, meat/gamey-flavour, spicy-aftertaste, herby-flavour, sour-taste, bitter-taste, astringent-aftertaste. These diets appear to have no off-flavours and have 'fresh' meat character as per the non-bioactive diet. Also 'pleasant' herby and spicy characteristics are present.

Overall, feed 3. Control + chicory was perceived as the most acceptable in its sensory characteristics relative to the other feeding treatments

Chicory and Silage/Chicory are similar in their sensory characteristics (bitter tasting and have freshly cooked meat odour), and are negatively correlated to boar-taint as described by Piggy/Animaly-flavour and odour. Thus, the chicory treatments are more acceptable as they have reduced boar-taint from a sensory perspective (Figure 8).

The non-bioactive control feeding treatment is the most boar tainted as indicated by the samples positive correlation to the descriptors Piggy/Animaly-flavour and odour.

Control and Silage have many common sensory characteristics, however, Silage appears to be related somewhat to have more lipid oxidation based off-note descriptors (cardboard and linseed-oil like), even in freshly cooked samples as were the samples in the present study. This was most likely related to higher levels of unsaturated phospholipids in the meat elevated through silage feeding. Thus, the silage fed samples had an increased potential for lipid-oxidation relative to all other feeding treatments (Byrne et al., 2001 b).

The improvement of sensory characteristics may be a reduction of lipid-oxidation comprising increasing acceptable sensory characteristics selected from the group of Cardboard-odour and flavour and Linseed oil-odour.

**Conclusions**

Treatments 3. chicory and 4. silage/chicory are very similar and are much lower in boar-taint from a sensory perspective than treatments 1. Non-bioactive and 2. Silage. Treatment 2. silage also appears to be the most prone to lipid oxidation of the samples.

Overall, chicory appears to reduce boar-taint and this is clearly noted by the sensory panel.

The most important aspect of this is the panel has indicated that the chicory effect on reducing boar-taint results in acceptable fresh pork meat from a sensory perspective.

The main point of course being that chicory having clearly reduced boar-taint from a sensory perception perspective did not lead to the imparting of other off-flavours in the freshly cooked meat of the chicory fed samples.

The non-bioactive control fed pigs were found to have a higher level of boar-taint as described by the term Piggy/Animaly-odour and flavour relative to the pigs fed chicory. Thus, the chicory fed pigs had a more acceptable sensory character than the pigs feed non-bioactive control from a 'consumer' perspective, in relation to boartaint.

**Example 4B**

**Sensory profiling of the effects of chicory (fresh and dried) and inulin (bioactive) feeding on boar-taint in entire male cooked pork**

**Experiment 1**

*Experiment 1a.* Sensory profiling study on Table 4 of example 2A feeding treatments (control, silage and fresh chicory 2 levels), muscle name *Longissimus dorsi* (LD 1) (presented as Example 4A above).

*Experiment 1b.* Sensory profiling study on Table 4 treatments (control, silage and fresh chicory 2 levels), muscle name *Psoas Major* (PM 1).

**Experiment 2**

*Experiment 2a.* Sensory profiling study on Table 9 of example 2B feeding treatments (control/silage, fresh chicory, dried chicory and inulin), muscle name *Longissimus dorsi* (LD 2).

*Experiment 2b.* Sensory profiling study on Table 9 feeding treatments (control/silage, fresh chicory, dried chicory and inulin), muscle name *Psoas Major* (PM 2).

**The objectives of the present studies**

**Experiment 1 a, b.**

Overall aim was to investigate the sensory variation that resulted from the effects of bioactive feeding (control, silage and fresh chicory 2 levels) in organically produced entire male cooked pork.

**Sensory profile**

The sensory profile was carried out with the specific aim to determine the effect of bioactive feeding on the sensory 'off-flavour' referred to as boar-taint in the meat. To achieve this aims a descriptive sensory vocabulary was developed with an expert sensory panel and subsequently the panel were utilised to develop a sensory profile for the meat samples, derived from male animals fed various levels of silage and 'fresh' chicory roots. In the analyses of the sensory profiling data a strategy involving multivariate Partial Least Squares Regression (PLSR) was utilised to determine precisely how the various feeding treatments were described and discriminated from a sensory perspective with respect to boar taint.

**Experiment 2 a, b.**

Overall aim was to investigate the sensory variation that resulted from the effects of bioactive feeding (control/ silage, fresh chicory, dried chicory and inulin) in organically produced entire male cooked pork.

The sensory profile was carried out with the specific aim to determine the effect of bioactive feeding on the sensory 'off-flavour' referred to as boar-taint in the meat. To achieve this aims a descriptive sensory vocabulary was developed with an expert sensory panel and subsequently the panel were utilised to develop a sensory profile for the meat samples, derived from male animals fed various levels of 'fresh' and 'dried' chicory and inulin. In the analyses of the sensory profiling data a strategy involving Partial Least Squares Regression (PLSR) was utilised to determine precisely how the various feeding treatments were described and discriminated from a sensory perspective with respect to boar taint.

**Materials and Methods**

For experiment 1 a the method conditions are described previously in example 4A.

The following considers experiment 1b, 2a and 2b.

**Meat samples**

Pork muscles *Psoas Major* (PM 1 and PM2) and *Longissimus dorsi* (LD 2) from entire male pigs were used for sensory analysis. Each muscle type was derived from animals fed one of four different feeding treatments (In the case of PM1, see Table 4 and PM2 and LD 2, see Table 9). A total of 8 individual animals muscles were obtained for each feeding treatment.

All muscles were stored vacuum packed in darkness at -20°C. Muscles were held at 4°C for approx. 12 h prior to handling to allow ease of cutting. Visible fat and connective tissues were removed and muscles were cut into chops (approx. 1 cm thickness). Individual chops were subsequently vacuum packed in oxygen impermeable plastic laminate bags. The vacuum-packed chops were then frozen at -30°C and stored for up to one week prior to use in profiling.

Prior to cooking treatment, all frozen vacuum packed chops were placed in a 25°C water bath until a core temperature of between 18 and 20°C had been reached. Subsequently chops were removed from their plastic vacuum bags and batch cooked in convection ovens set to 150°C. The ovens utilised were determined to have comparable heating cycles. The heating/cooking process at 150°C was determined to take a total of 8 min 4 minutes per side. The final internal temperature reached over all chop batches cooked was found to vary between 78 and 82°C. After cooking the samples were immediately served to the panelists such that the mean serving temperature of the samples was 65°C.

**Sensory measurements**

Prior to sensory profiling a sensory panel (10 persons) participated in the development of a sensory vocabulary to describe and discriminate the effects of conventional and bioactive feeding on the general flavour and in particular boar-taint in the pork meat of the present study (see Byrne et al., 1999a,b; Byrne et al., 2001a). The panel was recruited from the public and students of the Royal Veterinary and Agricultural University, Frederiksberg, Denmark. All sensory work was carried out in the sensory laboratory at the University, which fulfils requirements according to the international standards (ASTM, 1986; ISO, 1988).

Panel input, panel leader input, and multivariate statistical analyses were utilised to select a set of 42 descriptors plus an overall impression question from initial list of 45 terms (see Byrne et al., 2001 a). Each of the final list of terms was defined by a reference material and terms were divided into their modality of sensory assessment, i.e. odours, tastes, flavours and aftertastes (Table 23).

**Table 23.** List of 43 sensory descriptive characteristics with definitions developed for the evaluation of pork meat chops, oven cooked at 150°C for 6 min., derived from entire male pigss fed 4 different feeding treatments 1. control/ silage, 2. chicory 1 (fresh), 3. chicory 2 (dried), 4. Inulin, (see Table 9).

| Term [a] [b] | | Definitions and reference materials[c] |
|---|---|---|
| **Odour** | | **Aromatic associated with:** |
| **Fresh pork odours** | | |
| 1. | Fresh cooked pork meat like-O | Oven cooked pork meat with no on surface browning |
| 2. | Sweet meaty-O | Fresh cooked pork its sweetness characteristics |
| 3. | Nutty-O | Crushed roasted hazel nuts |
| **Boar taint odours** | | |
| 4. | Piggy/Animal-O | Cooked pork meat from entire male pigs |
| 5. | Gamey-O | Freshly cooked game meat as exemplified by deer, pheasant or wild boar |
| 6. | Urine-O | Male pig urine |
| 7. | Parsnip-O | Cooked parsnip/earthy/sweet |
| 8. | Musty-O | Stale damp/moist old fabric/cloth sealed in plastic for 5 days/moist celler |
| 9. | Manure/stable-O | Male pig excrement/faeces (presented in sealed vessel with perforated cover for assessment) |
| 10. | Sweat-O | Old human body sweat/Swiss cheese |
| **Feeding treatment odours** | | |
| 11. | Chicory (solid)-O | Flaked fresh chicory root |
| 12. | Feedy-O | Blended barley grains and water (50/50) |
| 13. | Hay/Silage-O | Dry hay/fermented hay (silage) |
| **Other odours** | | |
| 14. | Soapy-O | Non-perfumed liquid soap |

(continued)

| Texture | | Textural impression associated with: |
|---|---|---|
| *Initial mastication* | | |
| 15. | Hardness-Tx | Force required to bite completely through the sample with molars |
| 16. | Tenderness-Tx | Ease with which the meat is divided into fine particles when chewed |
| 17. | Juiciness-Tx | The amount of liquid exudate in the mouth when one has chewed the sample 5 times |
| *During mastication* | | |
| 18. | Fibrous-Tx | The amount of fibers appearing during mastication |
| *Preference* | | *Preference associated with:* |
| 19. | Overall Impression | Question: to which degree do you like the pork sample you have just tasted in the context of pork of this type? Scored as dislike very much to like very much on an unstructured 15cm line scale. |

Table 23 (contd.). List of 43 sensory descriptive characteristics with definitions developed for the evaluation of pork meat chops, oven cooked at 150°C for 6 min., derived from entire male pigss fed 4 different feeding treatments 1. control/silage, 2. chicory 1 (fresh), 3. chicory 2 (dried), 4. Inulin, (see Table 9).

| Term [a][b] | | Definitions and reference materials[c] |
|---|---|---|
| *Taste* | | *Taste associated with:* |
| 20. | Sour-T | Ymer/natural yoghurt/formage frais |
| 21. | Sweet-T | Sweet fresh cooked pork |
| 22. | Umami-T | The 'blooming' flavour enhancing taste of mono-sodium glutamate, a solution 0.5g/l MSG in water |
| *Flavour* | | *Aromatic taste sensation associated with:* |
| *Fresh pork flavours* | | |
| 23. | Fresh cooked pork meat like-F | Oven cooked pork meat with no on surface browning |
| 24. | Pork fat-F | Freshly cooked pork fat |
| *Boar taint flavours* | | |
| 25. | Piggy/Animal-F | Cooked pork meat from entire male pigs |
| 26. | Gamey-F | Freshly cooked game meat as exemplified by deer, pheasant or wild boar |

(continued)

| Term [a] [b] | | Definitions and reference materials[c] |
|---|---|---|
| 27. | Parsnip-F | Cooked parsnip/earthy/sweet |
| 28. | Manure/stable-F | Male pig excrement/faeces. Reference presented in sealed vessel with perforated cover for assessment aim to allow it to evoke 'flavour'. |
| *Feeding treatment flavours* | | |
| 29. | Livestock/Barny-F | Flavour of white peper just after the initial soapy notes and before the strong peppery notes |
| 30. | Feedy-F | Blended barley grains and water (50/50) |
| 31. | Hay-F | Flavour of dried grass |
| 32. | Spicy-F | Spicy flavour from salami |
| 33. | Chicory (water)-F | Water cooked with dried chicory root (4:1 w/v) |
| 34. | Chicory (flesh)-F | Dried chicory root flakes after soaking in boiling water |
| *Other-flavours* | | |
| 35. | Cardboard like-F | Wet cardboard |
| 36. | Serum/Metallic-F | Cooking losses from pan fried pork mince meat with high meat content (max 8-12% fat) |
| 37. | Cooked liver/Organy-F | Freshly cooked liver |
| *Aftertas te* | | *Aftertaste sensation associated with:* |
| 38. | Astringent-AT | Solution 0.02g/l aluminum sulphate in water. Drying sensation in mouth and on teeth. |
| 39. | Fresh sour/Lactic-AT | Ymer/natural yoghurt |
| 40. | Flat Bitter-AT | Bitter aftertaste from chicory |
| 41. | Heat/Spicy-AT | Salami heat 30 s after eating |
| 42. | Salty-AT | Sodium Chloride (NaCl) (basic salt) (0.5g/l) after-taste |
| 43. | Fatty mouthcoating-At | Residual fatty coating in mouth once meat expectorated |

[a] Suffix to sensory terms indicates method of assessment by panellists; -O = Odour, -F = Flavour,-T = Taste, -AT = Aftertaste, -Tx = Texture.
[b] Concentrations in g/l were devised to ensure panellists' could recognise clearly the sensory note involved.
[c] Definitions of sensory terms as derived during vocabulary development.

**Data acquisition**

Quantitative data was collected using the FIZZ Network data acquisition software (BIOSYSTEMS, Couternon, France). Unstructured line scales of 15 cm anchored on the left side by the term 'none' and on the right side by the term 'extreme' were used for the scoring of each sensory term (Meilgaard et al., 1999).

**Data analyses**

For initial exploration of the sensory data, Discriminant Partial Least Squares Regression (DPLSR) was performed for each profile. The X-matrix was set as the sensory data (level and range corrected) and the Y-matrix was design main effect 0/1 variables for feeding treatments. For contextual validation in the regression analysis, the conventional loading plot was replaced by a plot of correlation loadings. This allowed easier interpretation since it revealed both the structures in the data and their degree of fit at the same time.

All multivariate analyses were performed using the Unscrambler Software, Version 8.0 (CAMO ASA, Trondheim, Norway). In all regression analysis data were analysed, centred with full cross-validation.

**Results**

The results are presented in the figures 9, 10 and 11.

**Experiment 1b**

**Psoas *Major 1 (PM1)***

Fig. 9. Discriminant Partial Least Squares Regression (DPLSR) sensory profiling versus feeding treatments for *Psoas Major 1* (PM1) (PC 1 v 2) displayed that the animals fed treatment 1. non bioactive control feed and 2. silage were high in boar taint descriptors such as e.g. *manure/stable odour/flavour, piggy/animal odour/flavour, musty odour, urine odour* and *livestock/bamy flavour,* whereas animals fed chicory (treatments 3 and 4) were, relative to the control and silage treatments, not high in boar taint descriptors and were described by *fresh cooked pork meat odour/flavour* and thus, displayed a high *overall impression/liking.* These correlations are in complete agreement with the results from the *Longissimus Dorsi 1* (LD1) sensory profile previously performed and presented as example 4A above.

**Experiment 2 a,b**

***Psoas Major 2* (PM2)**

**Fig. 10.** Discriminant Partial Least Squares Regression (DPLSR) of sensory profiling versus feeding treatment design variables for *Psoas Major 2* (PM2) displayed that the animals fed treatment 1. control/silage were high in boar taint descriptors such as e.g. *manure/stable odour/flavour, gamey-flavour, Flat bitter-aftertaste* and *animal/piggy odour/flavour,* whereas animals fed 3. chicory 1 (fresh) were, relative to the 1. control/silage treatments, not high in boar taint descriptors and were described by *fresh cooked pork meat odour/flavour* and thus, displayed a higher *overall impression/liking.*

***Longissimus dorsi* 2 (LD2)**

**Fig. 11.** Discriminant Partial Least Squares Regression (DPLSR) of sensory profiling versus feeding treatment design variables for *Longissimus dorsi* 2 (LD2), displayed that the animals fed treatments 1. control/silage and 4. inulin were high in boar taint terms such as e.g. *manure/stable odour/flavour, animal/piggy odour/flavour and livestock/barny flavour,* whereas animals fed 2. chicory 1 (fresh) and 3. chicory 2 (dried) were, relative to 1. control/silage and 4. inulin, not high in boar taint descriptors and were described by *fresh cooked pork meat odour/flavour* and thus, and displayed a higher overall impression/liking. Moreover, treatments, 2. chicory 1 (fresh) and 3. chicory 2 (dried) appeared to be similarly effective in reducing bore-taint.

**Conclusions**

- Chicory in fresh and dried form reduced sensory boar-taint relative to control feeding.
- This effect was seen in both *Longissimus dorsi* and *Psoas Major* and thus may be considered independent of muscle type.
- Inulin appeared non effective in the reduction of sensory boar-taint relative to chicory feeding.
- Overall, the chicory fed samples achieved significantly higher overall impression/liking score relative to the control, as the chicory fed samples were high in fresh cooked pork like sensory notes.
- Thus, in reducing boar-taint chicory does not introduce negative sensory characteristics.

**Example 5**

**Organic feed used in the experiments**

Composition of the organic concentrate diet used in all the experiments presented was as listed in table 24.

**Table 24.** Composition of the organic concentrate diet.

|  | Amount, % | Amount, kg | Treatment[5] | Tolerance, % | Tolerance, kg |
|---|---|---|---|---|---|
| Rape[1] | 14.547 | 290.94 | RollerMill | 9.65 | 28.08 |
| Pea, ecologic | 24.000 | 480.00 | RollerMill | 6.25 | 30.00 |
| Wheat, ecologic | 22.315 | 446.30 | RollerMill | 6.72 | 29.99 |
| Barley, ecologic | 22.000 | 440.00 | RollerMill | 6.82 | 30.01 |
| Oat, ecologic | 5.000 | 100.00 | RollerMill | 30.00 | 30.00 |
| Soya bean, ecologic | 10.000 | 200.00 | RollerMill | 15.00 | 30.00 |
| ØKO-VIT[2]. 13.G | 0.200 | 4.00 | None | 12.50 | 0.50 |
| Salt[3] | 0.375 | 7.50 | None | 6.67 | 0.50 |
| Chalk[4] | 1.203 | 24.06 | None | 2.08 | 0.50 |
| Monocalciumphosphate | 0.363 | 7.26 | None | 6.89 | 0.50 |
| Total | 100.00 | 2000.06 |  |  |  |

1: gmo-free rape, grown in Denmark, heat treated
2: Product to include in whole food, includes vitamins from Vitfoss
3: Salt from Mariager
4: Food chalk/lime from Faxe
5: The products are grounded in the rollermill

**Drying of chicory roots**

The procedure for drying minced chicory roots in the presented experiments is as described below.
The fresh or non-dried (kept for less than 12 months) chicory roots were minced/chopped by a lightning fast mincer (brand Wiencken) and dried about 48 hours at 60°C by a drying cupboard (brand Lytzen Type CBM). Percentage of water content differed following the drying process from 6% to 10%. The taste of the dried product was sweet and bitter.

**Sugar content of different feed**

The sugar content of the three of the feed types used in example 2B, 3B and some of the experiments in example 5B are shown in table 25.

**Table 25.** Percentage of content of low-molecular and high-molecular sugars in the feed used in experiment

|  | Glucose | Fructose | Sucrose | Fructans[1] | Total |
|---|---|---|---|---|---|
| Control (organic concentrate) | 0.1 | 0.06 | 2.92 | 1.26 | 4.35 |
| Control + dried chicory | 0.22 | 1.6 | 6.18 | 15.64 | 23.64 |
| Control + pure Inulin | 0.1 | 0.06 | 2.39 | 17.27 | 19.82 |

1: Mainly inulin

Example 6

**The effect of feeding different concentrations of dried chicory for 7, 14 or 21 days prior to slaughter on the chemical and sensory attributes of meat from entire male pigs**
The objective of this experiment is to determine the lowest possible level and shortest possible duration of dried chicory feeding to result in chemical and sensory boartaint reduction.

## Background

Based on the results from ecamples presented above it is that:

1. Fresh and dried chicory feeding resulted in a highly significant reduction of sensory boar-taint and as a result a significant increase in sensory acceptable fresh meaty odour and flavour attributes, both in entire male pigs as well as in female pigs, by feeding the product 3, 5 and 8 weeks before slaughter compared to control treatments fed concentrate plus or minus clovergrass silage as roughage.

2. Skatole concentration in backfat and blood plasma was reduced by finishing feeding entire male pigs fresh or dried chicory for 7 days, 10 days, 14 days, 21 days, 5 and 8 weeks (see also Figure 4).

3. Finishing feeding of entire male pigs at 70 % concentrate plus 25 % dried chicory for 3 days results in a highly significant decrease in blood plasma skatole levels (see also Figure 4).

4. Moreover, after a full week the skatole concentration was very close to zero both in blood plasma and backfat (see also Table 26).

5. It was concluded that dried chicory, as it had a comparable effect to fresh chicory was the form of chicory that had the best potential for development to commercial product in terms of the economic and practical viability of the chicory root as a feedstuff ingredient. Furthermore the dried chicory root feed has the advantage that the pigs do not need an adaptation period before eating the full amount of 25% dried chicory roots on energy basis.

6. Thus, chicory feeding in the dried format provides a potentially viable solution to eradicating the consumer sensory off-flavour problem known as boar-taint, in female and more importantly in entire male pork meat.

The results listed above have been obtained from *at least* 3 weeks supplementation with 25% dried chicory (see Table 26). However, this experiment will show the minimum time and level of dried chicory needed to result in an equal reduction in sensory boar-taint and an equal enhancement of sensory meaty odour and flavour attributes as previously noted.

Such knowledge is critical to the commercial viability of the invention in practice, in that minimising the cost of the final feed developed and the feeding time required is of paramount importance to the acceptance of feeding with chicory as a solution to the boar-taint issue in pork meat. It is quite clear that the less dried chicory required in the feed and the shorter feeding time required the more potential the approach has in terms of commercial acceptance and implementation in the pork industry.

An additional benefit of the chicory, is that feeding e.g. 20 % dried chicory for three weeks may have the potential to decrease driploss. This has been reported when feeding high concentrations of inulin for three weeks by (Rosenvold et al., 2001; Rosenvold, 2002) This hypothesis is made with regard to the inulin levels contained in chicory roots.

In the previous studies the chicory was dried at 60 °C for 2 days and this will be repeated in the present study. The dried chicory will be mixed at 81 °C, with soya, wheat and barley in a feed mill according to the Danish legislation concerning the feed industry.

## Material and methods

The experiment is performed with 28 litters of 4 entire male pigs in total 112 DDLY crossbred pigs (see Table 27).

The experimental period must be 1, 2 or 3 weeks from a beginning weight at 94, 87 or 80 kg liveweight, respectively, so that the liveweight at slaughter will be about 100 kg for all pigs. The pigs will be kept in groups of 4 pigs per pen. The pigs are to be weighed at the beginning of the experiment 3, 2 and 1 week before slaughter and again on the day of slaughter at the abattoir.

The pigs will be fed 2.5, 5, 10 or 20 % dried chicory (according to energy level) for either 7, 14 or 21 days prior to slaughter and compared with control pigs fed concentrate without chicory (see Table 27). The percentage of dried chicory is on energy basis and is a substitution of concentrate.

Table 26. Effect of chicory feeding on skatole and sensory eating quality. An overview of the previous investigations main findings as included in experiments presented above. In addition, the feeding times that require investigation are indicated. Refer to Table 27 for the detailed experimental design with respect to the various levels of chicory to be fed.

| Main effects of chicory feeding in relation to days (weeks) | 0 | 3 | 7 (1) | 10 | 14 (2) | 21 (3) | 42 (6) | 56 (8) |
|---|---|---|---|---|---|---|---|---|
| High skatole concentrations in the majority of entire male pigs | X[a] | | | | | | | |
| Sensory boar-taint off odours and flavours in the majority of entire male pig meat (found in control concentrate fed entire male pigs) | X | | | | | | | |
| Significant reduction of skatole in blood (after 3 days of feeding chicory) | | X | | | | | | |
| Close to a total reduction in skatole in blood and backfat in male pigs | | | X | X | X | X | | |
| No unacceptable sensory boar-taint off odours and flavours present in entire male pig meat | | | | | | X | X | X |
| [a] X = Confirmed from previous investigations | | | | | | | | |

**Table 27.** Experimental design to determine the effect of feeding lower concentrations of dried chicory for shorter durations prior to slaughter on the chemical and sensory attributes of meat from entire male pigs.

| No. of feeding days before slaughter | 0 | 7 | 14 | 21 |
|---|---|---|---|---|
| Treatment 20% dried chicory | **1** Control 100 % concentrate on energy level | **2** 20 % dried chicory plus 80 % concentrate on energy level | **3** 20 % dried chicory plus 80 % concentrate on energy level | **4** 20 % dried chicory plus 80 % concentrate on energy level |
| No. of male pigs | 4 males | 8 males | 8 males | 8 males |
| Treatment 10% dried chicory | **5** Control 100 % concentrate on energy level | **6** 10 % dried chicory plus 90 % concentrate on energy level | **7** 10 % dried chicory plus 90 % concentrate on energy level | **8** 10 % dried chicory plus 90 % concentrate on energy level |
| No. of male pigs | 8 males | 8 males | 8 males | 8 males |
| Treatment 5% dried chicory | **9** Control 100 % concentrate on energy level | **10** 5 % dried chicory plus 95 % concentrate on energy level | **11** 5 % dried chicory plus 95 % concentrate on energy level | **12** 5 % dried chicory plus 95 % concentrate on energy level |
| No. of male pigs | 4 males | 8 males | 8 males | 8 males |
| Treatment 2.5% dried chicory | **13** Control 100 % concentrate on energy level | **14** 2.5 % dried chicory plus 97.5 % concentrate on energy level | **15** 2.5 % dried chicory plus 97.5 % concentrate on energy level | **16** 2.5 % dried chicory plus 97.5 % concentrate on energy level |
| No. of male pigs | 0 males | 8 males | 8 males | 8 males |

**Chemical analysis**

Skatole analysis in blood and backfat will be carried out. Blood samples will be collected three weeks before slaughter and the day before slaughter for all pigs. Skatole will be measured in backfat at slaughter as well as in blood plasma at beginning of the experiment and the day before slaughter. Skatole in blood plasma will be analysed just before slaughter. Frozen blood plasma samples will be available from three weeks before slaughter for later analysis of skatole if results should point for further analysis.

It is not essential to measure androstenone in blood plasma as we know from the previous experiments that the effect of chicory on androstenone may be minor and not significant. Blood plasma samples will be preserved frozen from both three weeks before slaughter and at slaughter for later analysis of androstenone in blood plasma if results should indicate a requirement for further analysis.

Furthermore, pH24 (PH measured 24 hours post slaughter) and driploss of meat juice (Honnikel's method) as well as Minolta colour values will be measured in the control and the 20 % chicory fed pigs fed 7, 14 and 21 days before slaughter.

**Sensory analysis**

Sensory profiling analysis of 1.0 kg of M. *long. dorsi from* all treatments using a 10-member expert panel will be carried out. All sensory work will be carried out in the sensory laboratory at the The Royal Veterinary and Agricultural University (KVL), which fulfils requirements according to the international standards (ASTM, 1986; ISO, 1988).

Prior to sensory profiling the panel will participate in the development of a sensory vocabulary as per (Byrne et al., 1999a,b). Panel input, panel leader input, and multivariate statistical analyses will be utilised to select a set of descriptive terms. Each term will be defined by a reference material and terms will be divided into odours, tastes, flavours and aftertastes.

In addition, with respect to the environmental odour impact of pig rearing facilities during the warmest periods in summer time. Measurement with an olfactometer instrument will allow the presentation of odour qualities of faeces from the experiment with respect to the different treatments with dried chicory (oligosaccharides) of animals in a highly controlled and safe manner directly to human subjects. From this it will be possible to gain insight as to the level of reduction of off-odours relevant to the negative perception of ventilation air from the pig houses. The impact of the present experiment on the environment with respect to odour levels in pig rearing facilities may become very important, particularly if we see an effect of chicory at lower levels and used strategic during the warmest time of the year when the environmental odour impact of pig rearing facilities exists.

**References**

Amon, M., Dobeic, M., Misselbrook, T.H., Pain, B.F., Phillips, V.R. and Sneath, R.W. 1995. A farm scale study on the use of de-odourase for reducing odour and ammonia emissions from intensive fattening piggeries. Bioresource Technology. 51:163-169.

AOAC (Association of Official Analytical chemists), 1990. Official methods of analysis. Journal of AOAC International. Washington D. C.

ASTM (1986). Physical Requirements. Guidelines for Sensory Evaluation Laboratories, STP 913. Pennsylvania: American Society for Testing and Materials.

Bach Knudsen, K. E., 1997. Carbohydrates and lignin of plant materials used in animal production. Animal Feed Science and Technology 67, 319-338.

Bach Knudsen, K. E. and Hessov, I., 1995. Recovery of inulin from Jerusalem artichoke (Helianthus tuberosus L.) in the small intestine of man. British Journal of Nutrition 74, 101-113.

Bais, H.P. and Ravishankar, G.A. 2001. Cichorium intybus L - cultivation, processing, utility, value addition and biotechnology, with an emphasis on current status and future prospects. Journal of the Science of Food and Agriculture. 81:467-484.

Bjørn,H., Roepstorff,A., Nansen,P. 1996. A possible influence of diet composition on the establishment of nematodes in pigs. Veterinary Parasitology. 63, 167-171. Boisen, S. and Fernandez, J. A., 1998. Prediction of the total tract digestability of energy in feedstuffs and pig diets by in vitro analysis. Animal Feed Science and Technology 68, 277-286.

Bonneau,M. and Carelli, C. 1987. Immunological castration of male pigs with a synthetic aqueous vaccine. Annales de Zootechnie, 36: 265-269.

Bonneau, M., Walstra, P., Claudi-Magnussen, C., Kempster, A.J., Tornberg, E., Fischer, K., Diestre, A., Siret, F., Chevillon, P., Claus, R., Dijkterhuis, G. B., Punter, P., Matthews, K.R., Agerhem, H., Béague, M.P., Oliver, M.A., Gispert, M., Weiler, U., von Seth, G., Leask, H., Font i Furnols, M., Homer, D. B., & Cook, G.L. (2000). An international study on the importance of androstenone and skatole for boar taint: IV. Simulation studies on consumer dissatisfaction with entire male pork and the effect of sorting out carcasses on the slaughter line, main conclusion and recommendations. Meat Science, 54, 285-295.

Byrne, D. V., Bak, L. S., Bredie, W. L. P., Bertelsen, G., & Martens, M. (1999a). Development of a sensory vocabulary for warmed-over flavour: part I. in porcine meat. Journal of Sensory Studies, 14, 47-65.

Byrne, D. V., Bredie, W. L. P., & Martens, M. (1999b). Development of a sensory vocabulary for warmed-over flavour: part II. in chicken meat. Journal of Sensory Studies, 14, 67-78.

Byrne, D. V., Bredie, W. L. P., Bak, L. S., Bertelsen, G., Martens, H. and Martens, M. (2001 b). Sensory and chemical analysis of cooked porcine meat patties in relation to warmed-over flavour and pre-slaughter stress. Meat Science, 59, 229-249.

Byrne, D.V., O'Sullivan, M.G., Dijkterhuis, G., Bredie, W.L.P., & Martens, M. (2001 a). Sensory panel consistency during the development of a vocabulary for warmed-over flavour. Food Quality and Preference, 12, 171-187.

Claus, R. P. 1992. Verfahren zur Herstellung von diätetischen Produkten zur gezielten Hemmung der intestinalen Bildung von 3-Methylindol (skatol). Claus, R. P. (DE 42 23 051 A1), 1-5. 14-7-1992. Germany. (GENERIC) Ref Type: Patent

Claus, R. P. 1994. Reducing intestinal skatole production esp. in pigs using feed containing e.g. alkali metal bi:carbonate or inulin, e.g. for improving quality. Claus, R. P. 14-JUL-92 92DE4223051(DE 42 23 051-A1). 20-1-1994. Germany. (GENERIC) Ref Type: Patent

Claus, R., D. Losel, M. Lacorn, J. Mentschel, and H. Schenkel. 2003. Effects of butyrate on apoptosis in the pig colon and its consequences for skatole formation and tissue accumulation. J. Anim Sci. 81:239-248.

Dijksterhuis, G.B., Engel, B., Walstra, P. Font i Furnols, M. Agerhem, H.,Fischer, K., Oliver, M. A., Oliver, Claudi-Magnussen, C., Siret, F., Béague, M.P.,Homer, D.B., Bonneau, M. (2000). An international study on the importance of androstenone and skatole for boartaint: II. Sensory evaluation by trained panels in seven European countries. Meat Science, 54, 261-269.

Dunshea, F.R., McCauley, I. and Corbett, J. 2001. Immunization of pigs against gonadotrophin releasing factor (GnRF) prevents boar taint and affects boar growth and behaviour. Recent advances in animal nutrition in Australia 2001, 16th Symposium, Armidale, Australia, 13:

Gemert, L.L. van and Nettenbreijer, A.H. 1977. Compilation of odour threshold values in air and water. National Inst. For Water Supply, Voorburg and Central Inst. For Nutrition and Food Research TNO, Zeist, Netherlands.

Gibis, M., Hilmes, C. Fischer, A. 1998. Off-flavour in pork caused by skatole. Fleischwirtschaft, 78, 727-730.

Hale, O.M. and Marti, O.G. 1984. Influence of an experimental infection of Strongyloides ransomi on performance of pigs. Journal of Animal Science 58, 1231-1235.

Hale, O.M. and Stewart, T.B. 1979. Influence of an experimental infection of Trichuris suis on performance of pigs. Journal of Animal Science 49,1000-1010.

Hale, O.M., Stewart, T.B., Marti, O.G., Wheat, B.E. and McCormick, W.C. 1981. Influence of an experimental infection of nodular worms (Oesophagostomum spp.) on performance in pigs. Journal of Animal Science 52, 316-322.

Hale, O.M., Stewart, T.B. and Marti, O.G. 1985. Influence of an experimental infection of Ascaris suum on performance of pigs. Journal of Animal Science 60, 220-225.

Hansen, L.L., Larsen, A.E. and Hansen-Møller, J, 1995. Influence of keeping pigs heavily fouled with faeces plus urine on skatole and indole concentration (boar taint) in subcutaneous fat. Acta Agriculturae Scandinavica, 45: 178-185.

Hansen,L.L., Larsen, A.E., Jensen, B.B., Hansen-Møller, J, and Barton-Gade, P. 1994: Influence of stocking rate and faeces deposition in the pen at different temperatures on skatole concentration (boar taint) in subcutaneous fat. Animal Production, 59: 99-110.

Hartung, J. and E. Rokicki. 1984. Occurrence of phenolic compounds in the dust of swine and poultry houses. Zentralblatt fur Bakteriologie Mikrobiologie und Hygiene, 1 179:431-439.

Hansen, B. and Sørensen, N. K., 1996. Metodereferencer til centrallaboratoriets analyser. Research Centre Foulum, Danish Institute of Animal Science 79, 1-21.

Hartung, E., Jungbluth, T. and Büscher, W. 2001. Reduction of ammonia and odor emissions from a piggery with biofilters. Transactions of the ASAE. 44:113-118.

Hazra, B., Sarkar, R., Bhattacharyya, S. and Roy, P. 2002. Tumour inhibitory activity of chicory root extract against ehrlich ascites carcinoma in mice. Fitoterapia. 73:730-733.

Hidaka,H., Eida, T., Takizawa, T., Tokunaga, T., and Tashiro, Y. 1986. Effects of fructooligosaccharides on intestinal flora and human health. Bifidobacteria and Microflora, 5: 37-50.

Hobbs, P.J., Pain, B.F., Kay, R.M. and Lee, P.A. 1996. Reduction of odourous compounds in fresh pig slurry by dietary control of crude protein. J. Sci. Food Agric. 71:508-514.

ISO (1985). International Standard 6564. Sensory analysis-Methodology-Flavour profile methods. Ref. no. ISO 6564:1985 (E). International Organization for Standardization, Genève.

ISO (1988). International Standard 8589. Sensory analysis-general guidance for the design of test rooms. Ref. no. ISO 8589:1988 (E). International Organization for Standardization, Genève.

ISO (1994). International Standard 11035. Sensory analysis-identification and selection of descriptors for establishing a sensory profile by a multidimensional approach. Ref. no. ISO 11035:1994 (E). International Organization for Standardization, Genève.

Jensen, M.T. Cox, R.P. and Jensen, B.B. 1995. Microbial production of skatole in the hind gut of pigs given different diets and its relation to skatole deposition in backfat. Animal Science. 61:293-304.

Jensen, B.B. and M.T. Jensen 1998 Microbial production of skatole in the digestive tract of entire male pigs. (Chapter 3) In: W. Klinth Jensen. (Editor) Skatole and boar taint. ISBN 87-985837-1-9. Danish Meat Research Institute, Roskilde, Denmark, 41-75.

Jensen, T.K. & Svensmark, K. (1996). Trichuriasis hos udendørs slagtesvin. Veterinaerinformation 2, 3-7. (In Danish).

Knarreborg, A., Beck, J. Jensen, M.T. Laue, A. Agergaard, N. and Jensen, B.B. 2002. Effect of non-starch polysaccharides on production and absorption of indolic compounds in entire male pigs. Animal Science, 74: 445-453.

Kelly-Quagliana, K.A., Buddington, R.K, Van Loo, J. & Nelson, P.D. (1998). Immunomodulation by oligofructose and inulin. In Nutritional and Health Benefits of Inulin and Oligofructose. NIH, Bethesda, p. 53.

Kisiel, W. and Michalska, K. 2002. A new coumarin glucoside ester form Cichorium intybus. Fitoterapia. 73: 544-546.

Krebsky, E.O., Geuns, J.M.C. and De Proft, M. 1999. Polyamines and sterols in Cichorium heads. Phytochemistry. 50:549-553.

Larsson, K. and Bengtsson, S., 1983. Metodebeskrivning nr. 22. Statens lantbrukskemiske laboratorium, Uppsala, Sweden.

Lenis, N.P. and Jongbloed, A.W. 1999. New technologies in low pollution swine diets: diet manipulation and use of synthetic amino acids, phytase and phase feeding for reduction of nitrogen and phosphorous excretion and ammonia emission - review. Asian - Aus. J. Anim. Sci. 12:305-327.

Madsen, A., J. S. Petersen, and Aa. Soegaard. 1990. Anatomic content of the female and castrated male pig fed according to scale or ad libitum and slaughtered at 20, 50, 80 or 110 kg. Communication no. 769. National Institute of Animal Science (Denmark), 4 pp.

Meilgaard, M.C. 1975. Flavour chemestri of beer. Part I: Flavour interaction between principal volatiles. Techn. Q., Master Brew. Assoc. Am. 12, 107.

Meilgaard, M., Civille, G. V., & Carr, B. T. (1999). Measuring responses. In Sensory evaluation techniques 3rd ed. (pp. 43-57). Florida: CRC press.

Metz, C., K. Hohl, S. Waidelich, W. Drochner, and R. Claus. 2002. Active immunization of boars against GnRH at an early age: consequences for testicular function, boar taint accumulation and N-retention. Livestock Production Science 74:147-157. Nansen, P. and Roepstorff, A. 1999. Parasitic helminths of the pig: factors influencing transmission and infection levels. International Journal for Parasitology 29, 877-891.

Nørbaek, R., Nielsen, K. and Kondo, T. 2002. Anthocyanins from flowers of Cichorium intybus. Phytochemistry. 60:357-359.

Pahl, O., Williams, A.G. and Sneath, R.W. 2002. Reduction of ammonia and odour emissions from pig slurry under slats using oil and foam layers. Environmental Technology. 22:395-430.

Peters, A.M., Haagsma, N., Gensch, K.-H. and Amerongen, A. van. 1996. Production and characterization of polyclonal antibodies against the bitter sesquiterpene lactones of chicory (Cichorium intybus L.). J. Agric. Food. Chem. 44:3611-3615.

Petkevič ius, S., Bach Knudsen, K.E., Murrell, K.D. & Wachmann, H., 2003. The effect of inulin and sugar beet fibre on Oesphagostomum dentatum infection in pigs. Parasitology 127, 61-68.

Petkevič ius, S., Bach Knudsen, K.E., Nansen, P. & Murrell, K.D. (2001). The effect of dietary carbohydrates with different digestability on the population of Oesophagostomum dentatum in the intestinal tract of pigs. Parasitology, 123, 315-324.

Petkevič ius, S., Bach Knudsen, K.E., Nansen, P., Roepstorff, A., Skjøth, F. & Jensen, K. (1997). The impact of diets varying in carbohydrates resistant to endogenous enzymes and lignin on populations of Ascaris suum and Oesophagostomum dentatum in pigs. Parasitologi 144, 555-568.

Petkevič ius, S., Nansen, P., Bach Knudsen, K.E. & Skjøth, F. (1999). The effect of increasing levels of insolubable dietary fibre on the establishment of Oesophagostomum dentatum in pigs. Parasite 6, 17-26.

Poli, F., Sacchetti, G., Tosi, B., Fogagnolo, M., Chillemi, G., Lazzarin, R. and Bruni, A. 2002. Variation in the content of the main guaianolides and sugars in Cichorium intybus var. "Rosso di Chioggia" selections during cultivation. Food Chemistry. 76:139-147.

Rees, S.B and Harborne, J.B. 1985. The role of sesquiterpene lactones and phenolics in the chemical defence of the chicory plant. Phytochemistry 24:2225-2231.

Roepstorff, A. 1997. Helminth surveillance are a prerequisite for anthelmintic treatment in intensive sow herds. Veterinary Parasitology 73: 139-151.

Roepstorff, A. & Nansen, P. (1994). Epidemiology and control of helmint infections in pigs under intensive and non-intensive productions systems. Veterinary Parasitology 54,69-85.

Roepstorff, A. & Nansen, P. (1998). Epidemiology, diagnosis and control of helminth parasites of swine. FAO Animal Health Manual 3, Rome, Italy, 161 pp.

Roepstorff, A., Eriksen, L., Slotved, H.-C. & Nansen, P. (1997). Experimental Ascaris suum infection in the pig: worm population kinetics following single infections with three doses of infective eggs. Parasitology 115, 443-452.

Rosenvold, K. 2002. Strategic feeding -- a tool in the control of technological pork quality. Acta Universitatis Agriculturae Sueciae Agraria No.314, 59 pp.; thesis; many ref.:59.

Rosenvold, K., H. N. Laerke, S. K. Jensen, A. H. Karlsson, K. Lundstrom, and H. J. Andersen. 2001. Strategic finishing feeding as a tool in the control of pork quality. Meat Sci. 59:397-406.

Seto, M., Miyase, T., Umehara, K., Ueno, A., Hirano, Y., and Otani, N. 1988. Sesquiterpene lactones from Cichorium endivia L. and C. intybus L. and cytotoxic activity. Chem. Pharm. Bull. 36:2423-2429.

Slotved, H.-C., Barnes, E.H., Bjørn, H., Christensen, C.M., Roepstorff, A. & Nansen, P. (1996). Recovery of Oesophagostomum dentatum from pigs by isolation of parasites migrating from large intestinal contents embedded in agar-gel. Veterinary Parasitologi 63, 237-245.

Slotved, H.-C., Barnes, E.H., Eriksen, L., Roepstorff, A., Nansen, P. & Bjørn, H. (1997). Use of an agar-gel technique to recover Ascaris suum larvae from intestinal contents of pigs. Acta Veterinaria Scandinavica 38, 207-212.

Sneath, R.W., Burton, C.H. and Williams, A.G. 1992. Continuous aerobic treatment of piggery slurry for odour control scaled up to a farm-size unit. J. Agric. Engng. Res. 53:81-92.

Stewart TB, Hale OM, Marti OG. 1985. Experimental infections with Hyostrongylus rubidus and the effects on performance of growing pigs. Veterinary Parasitology 17:219-229.

Stoldt, W., 1952. Vorschlag zur vereinheitlichung der fettbestimmung in lebensmitteln. Fette, Seifen und Anstrichsmittel 54, 206-207.

Sultana, S., Perwaiz. S., Iqbal, M. and Athar, M. 1995. Crude extracts of hepatoprotective plants, Solanum nigrum and Cichorium intybus inhibit free radical-mediated DNA damage. Journal of ethnopharmacology. 45:189-192.

Sutton, A.L., Kephart, K.B., Verstegen, M.W.A., Canh, T.T. and Hobbs, P.J. 1999. Potential for reduction of odorous compounds in swine manure through diet modifications. J. Anim. Sci. 77:430-439.

Thamsborg, S.M. & Roepstorff, A. Parasite problems in organic livestock production systems and options for control. Journal of Parasitology 89 (Suppl.), S277-S284.

Thomsen, L.E., Bach Knudsen, K.E., Møller, K., Murrell, K.D. and Roepstorff, A.: The effect of dietary carbohydrates with different digestability on the population of Trichuris suis in the intestinal tract of pigs. (in preparation)

Xu, Z.R., Hu, C.H., and Wang, M.Q. 2002: Effects of fructooligosaccharide on conversion of L-tryptophan to skatole and indole by mixed populations of pig fecal bacteria. J.Gen.Appl.Microbiol., 48: 83-90.

Zahn, J.A., DiSpirito, A.A., Do, Y.S., Brooks, B.E., Cooper, E.E. and Hatfield, J.L. 2001. Correlation of human olfactory responses to airborne concentrations of malodourous volatile organic compounds emitted from swine effluent. J. Environ. Qual. 30:624-634.

**Items**

In the following the invention is described as numbered items starting with the arbitrary number 1.

1. A method for reducing taint in animals, said method comprising feeding to an animal a chicory root product during at least one day prior to slaughtering the animal.

2. The method of item 1, wherein the chicory root product is fed to the animal for at least two days, for example 3 days, such as at least one week, for example at least 1.5 weeks, such as at least 2 weeks, preferably at least 3 weeks, such as at least 4 weeks, for example at least 5 weeks, such as at least 6 weeks, for example at least 7 weeks, such as at least 8 weeks, for example at least 9 weeks, such as at least 10 weeks, for example at least 15 weeks, such as at least 20 weeks.

3. The method of item 1-2, wherein the chicory root product is fed to the animal substantially until slaughter.

4. The method according to any of the preceding items, wherein the chicory root product is fed to the animal daily.

5. The method of item 4, wherein the chicory root product is fed to the animal at least one time a day such as several times daily, such as 2 times, 3 times, 4 times, 5 times, or more than 5 times.

6. The method according to any of the preceding items, wherein the chicory root product part of the ration of the animal is at least 2.5 % on a daily energy basis.

7. The method of item 6, wherein the chicory root product part of the ration of the animal is at least 5 % on a daily energy basis.

8. The method of item 6, wherein the chicory root part comprises at least 10 % of the ration, more preferably at least 15%, more preferably at least 20%, more preferably at least 25%, more preferably at least 30 %, for example at least 35%, such as at least 40%, for example at least 50%, such as at least 60%, for example at least 75%, such as at least 90%, for example substantially 100%.

9. The method according to any of the preceding items, wherein the animal is a ruminant, such as cow, sheep, goat, buffalo.

10. The method according to any of the preceding items 1 to 8, wherein the animal is a monogastric species, such as horse, pig, poultry, dog, and cat.

11. The method according to item 10, wherein the monogastric species is a pig.

12. The method according to item 11, wherein the pig is a male pig.

13. The method according to item 12, wherein the pig is an entire male pig.

14. The method according to item 11-13, wherein weight of the pig is from 25 to 300 kg, preferably as from 55 to 160 kg.

15. The method according to any of the preceding items, wherein the species of Chicory is Cichorium intybus L.

16. The method according to any of the preceding items, wherein the chicory roots contain at least 5% inulin, more preferably at least 10% inulin, more preferably at least 15 % inulin, more preferably at least 20 % inulin, such as at least 25% inulin, for example at least 30 % inulin.

17. The method according to any of the preceding items, wherein the chicory roots contain at least 5% FOS, more preferably at least 10% FOS, more preferably at least 15 % FOS, more preferably at least 20 % FOS, such as at least 25% FOS, for example at least 30 % FOS.

18. The method according to any of the preceding items, wherein the chicory root product comprises a silage product of chicory roots, such as a silage product of essentially whole chicory roots.

19. The method according to any of the preceding items, wherein the chicory root product comprises a fermented product of chicory roots.

20. The method according to any of the preceding items, wherein the chicory root product comprises a dried product of chicory roots, such as a dried product of essentially whole chicory roots.

21. The method according to any of the preceding items, wherein the chicory root product is a disintegrated product, such as a powder, flakes, pulp, slices, flour, pellets.

22. The method according to any of the preceding items, wherein the chicory root product comprises fresh chicory roots.

23. The method according to any of the preceding items, wherein the chicory root product comprises a fraction and/or an extract of chicory roots.

24. The method according to item 23, wherein the fraction and/or extract comprises an inulin fraction and a low molecular weight fraction comprising coumarins and/or sesquiterpenes.

25. A method for reducing the skatole content in animals, said method comprising feeding to a animal a chicory root product for at least one day such as at least two days prior to slaughtering.

26. The method of item 25, wherein the skatole content of blood is reduced by at least 25%, more preferably at least 40%, more preferably at least 50%, more preferably at least 75%, more preferably at least 80%, more preferably at least 90%, more preferably at least 95%, more preferably at least 98%, more preferably to substantially 0.

27. The method of item 25, wherein the skatole content of blood and/or backfat is reduced to below the human sensory threshold.

28. The method of item 25, wherein the skatole content of backfat is reduced by at least 25%, more preferably at least 40%, more preferably at least 50%, more preferably at least 75%, more preferably at least 80%, more preferably at least 90%, more preferably at least 95%, more preferably at least 98%, more preferably to substantially 0.

29. The method of item 25-28, further including the features of item 2 to 24.

30. A method for reducing the androstenone content in meat and/or fat and/or blood said method comprising feeding to an animal a chicory root product for at least one day such as at least two days.

31. The method of item 30, wherein the androstenone content is reduced by at least 10%, more preferably at least 25%, more preferably at least 40%, more preferably at least 50%, more preferably at least 75%, more preferably at least 80%, more preferably at least 90%, more preferably at least 95%, more preferably at least 98%.

32. The method of item 30-31, wherein the androstenone content in meat and/or fat is reduced to below the human sensory threshold.

33. The method of item 30-32, wherein the animal is subsequently slaughtered.

34. The method of item 30-33, further including the features of item 2 to 24.

35. A method for improving the sensory characteristics comprising odour, flavour, taste and/or aftertaste of meat from a human sensory perspective, said method comprising feeding to an animal a chicory root product for at least one day such as at least two days prior to slaughter.

36. The method of item 35, wherein the improvement of sensory characteristics is a reduction of boar taint comprising reducing Piggy/Animaly-odour and/or Piggy/Animaly-flavour to an acceptable level from a human sensory perspective.

37. The method of item 35, wherein the improvement of sensory characteristics is a reduction of boar taint comprising increasing acceptable sensory characteristics selected from the group of Fresh cooked pork meat like-odour and flavour, Sweet meaty-odour, Sweet-taste, Umami-taste, Meat/Gamey-odour and flavour, Herby-flavour, Spicy-flavour and Heat/spicy aftertaste, Nutty-odour, Metallic-flavour, Meat/Gamey-flavour, Herby-flavour, Spicy-flavour, Lactic/fresh sour-flavour.

38. The method of item 35, wherein the improvement of sensory characteristics is a reduction of lipid-oxidation comprising increasing acceptable sensory characteristics selected from the group of Cardboard-odour and flavour and Linseed oil-odour.

39. The method of item 35, wherein the improvement of sensory characteristics comprises reduction of sensory characteristics selected from the group of: Piggy/Animaly-odour and flavour, Manure/Stable-odour and flavour, Livestock/Barney-flavour, Cooked liver/Organy-flavour, Musty-odour, Urine-odour, Sweat-odour, Flat Bitter-aftertaste, White pepper-flavour,, Chemical/medicinal-aftertaste, Unacceptability.

40. The method of item 35, wherein the improvement of sensory characteristics comprises improving sensory characteristics such that Hardness-texture is decreased and Tenderness and Juiciness texture are increased and are involved in improving acceptability

41. The method of item 35-40, further including the features of item 2 to 24.

42. A method for reducing malodour in the environment, said method comprising feeding a chicory root product to animals for at least one day such as at least two days.

43. The method according to item 42, wherein the reduction is caused by a relative reduction in skatole and/or p-cresole and/or indole in the gastrointestinal tract.

44. The method according to item 43-43, wherein the reduction is caused by a relative increase in the amount of 2-pentanon and/or ethylbutyrate and/or propylpropionate and/or propylbutyrate and/or butanoic acid 2-methyl-ethylester in the gastrointestinal tract.

45. The method according to item 42-44, wherein the animal is a ruminant such as cattle, buffalo, sheep, goat.

46. The method according to item 42-44, wherein the animal is a monogastric species.

47. The method of item 46, wherein the monogastric animal is a furred animal, such as mink, fox, mouse, cat, muskrat, rabbit, hare, wolf, dog.

48. The method of item 46, wherein the monogastric animal is an animal used for meat, such as pig, poultry, rabbit, hare, more preferably wherein the monogastric animal is a pig.

49. The method according to any of the preceding items 42 to 48, wherein the malodour is stable malodour and the animal is kept in a stable.

50. The method according to item 49, wherein the animal is kept in the stable for at least 8 hours a day.

51. The method according to any of the preceding items 42 to 50, wherein the malodour is manure malodour and the manure originates from animals fed with the chicory root product.

52. The method of item 42 to 51, further including the features of item 2 to 24.

53. A method for reducing the amount of infections of the gastrointestinal tract in a non-human animal, said method comprising feeding to a non-human animal a chicory root product for at least one day such as at least two days.

54. The method of item 53, wherein the infections are parasites.

55. The method of item 54, wherein the parasites are worms.

56. The method of item 54, wherein the reduction is a reduction of the number of eggs in the animal faeces.

57. The method of item 53, wherein the infections are microbiological infections selected from Coli, Salmonella, Campylobacter and Yersinia

58. The method of item 55, wherein the infections are worms selected from *Ascaris suum, Oesophagostomum dentatum, Oesophagostomum quadrispinulatum, Oesophagostomum brevicaudum, Oesophagostomum granatensis, Oesophagostomum georgianum, Hyostrongylus rubidus, Trichuris suis,* and *Strongyloides ransomi* and *Trichinella* spp.

59. The method of item 53 to 58, further including the features of item 2 to 24.

60. Use of chicory roots as a feed product for "grown up" (>> 7 weeks) pigs.

61. Use of chicory roots for preparing a feed product for "grown up" pigs.

62. Use of chicory roots for preparing a product for the prevention of boar taint.

63. Use of chicory roots for preparing a product for reduction of skatole content in pigs, in particular in boar meat and fat.

64. Use of chicory roots for preparing a product for reduction of androstenone in pigs.

65. Use of chicory roots for preparing a product for reduction or prevention of gastrointestinal tract infections in pigs.

66. The use according to item 60-65 further including the features of item 2-24.

67. Use of a method for reducing taint in animals, said method comprising feeding to a male animal a chicory root product during at least one day such as at least two days prior to slaughtering the animal.

68. The use of the method of item 67, further including the features of item 2-24.

69. A chicory root product comprising components from chicory roots, where said components comprises at least inulin, one or more low molecular sugars and one or more secondary metabolites.

70. The chicory root product according to item 69, wherein said low molecular sugars are selected from the group of glucose, fructose, sucrose, maltose, maltotriose, maltotetraose, fructan (tri to octasaccharides).

71. The chicory root product according to item 69 to 70, wherein said secondary metabolites are selected from the group of terpenes, phytosterols, polyamines, coumarins and flavonoids.

72. The chicory root product according to item 69 to 71, wherein said secondary metabolites are selected from the group of Sesquiterpene lactones such as 8-Deoxylactucin, crepidiaside, lactucin, lactupicrin, crepidraside, 11-β-13-dihydrolactucin, picriside, sonchuside A, sonchuside C, cichoriolide A, cichoriosides A, cichorioside B and cichorioside C; Phytosterols such as Sitosterol, stigmasterol, and campersterol; Coumarines such as Esculetin (=aesculetin), esculin (the glucon of esculetin), cichoriin-6'-p-hydroxyphenyl acetate and cichoriin; Flavonoids such as Luteolin 7-glucuronide, quercetin 3-galactoside, quercetin 3-glucuronide, kaempferol 3-glucoside, kaempferol 3-glucuronide, isorhamnetin 3-glucuronide; Anthocyanins such as Cyanidin 3-O-β-(6-o-malonyl)-D-glucopyranoside and four delphinidin derivatives; Caffeic acid derivatives such as Caffeic acid, chicoric acid, and chlorogenic acid; Polyamines (biogenic amines) such as Putrescine, spermidine, spermine.

73. The chicory root product according to item 69 to 72, wherein said components from chicory comprises at least 50 % of the chicory root product.

74. The chicory root product according to item 69 to 73, wherein said chicory roots are dried.

75. The chicory root product according to item 69 to 74, wherein said chicory roots are fractionated.

76. A feed product comprising a chicory root product according to item 69 to 75.

77. A meat taste increasing product comprising components from chicory roots.

78. A boar taint decreasing product comprising components from chicory roots.

79. A skatole-inhibiting product comprising components from chicory roots

80. A parasite inhibiting product comprising components from chicory roots.

81. A malodour-decreasing product comprising components from chicory roots.

82. Use of a chicory root product as defined in item 69 to 75.

83. Use of a chicory root product for the manufacture of a meat taste increasing product.

84. The use according to item 83 wherein said meat taste increasing product is further used according to any of item 1 to 58.

85. Use of a chicory root product for the manufacture of a boar taint decreasing product.

86. The use according to item 85 wherein said boar taint decreasing product is further used according to any of item 1 to 58.

87. Use of a chicory root product for the manufacture of a parasite inhibiting product.

88. The use according to item 87 wherein said parasite inhibiting product is further used according to any of item 1 to 58.


**Claims**

1. A method for preparing a dried chicory food product comprising inulin and

    • one or more low molecular sugars and
    • one or more secondary metabolites selected from the group of terpenes, phytosterols, polyamines, coumarins and flavonoids,
    said method comprising the steps of
    • providing chicory roots,
    • drying said chicory roots by heating at a temperature above 50°C and such that the material temperature of the chicory is less than about 75°C, and the water content of the dried chicory material is below 10%,
    • obtaining the dried chicory product.

2. The method according to claim 1, wherein the chicory roots are disintegrated before drying said chicory roots.

3. The method according to claim 1 or 2, wherein the drying of said chicory roots by heating is performed until said chicory roots have a water content of from 6% to 10%.

4. The method according to claim 1 or 2, wherein the drying of said chicory roots by heating is performed until said chicory roots have a water content of from 3% to 4%.

5. The method according to any of the preceding claims, wherein said drying is performed at a temperature between 50°C and 800°C.

6. The method according to any of the preceding claims, wherein said drying is performed for about 48 hours at 60°C.

7. The method according to any of the preceding claims 1 to 5, wherein said drying is performed for about 5-10 minutes at a temperature of about 300°C.

8. An animal feed product comprising dried chicory, wherein said dried chicory is dried by a heating process at a temperature above 50°C and such that the maximum material temperature of the chicory is less than about 75°C and the water content of the dried chicory material is below 10%, said dried chicory comprises components from chicory roots comprising at least inulin, and

   • one or more low molecular sugars and
   • one or more secondary metabolites selected from the group of terpenes, phytosterols, polyamines, coumarins and flavonoids.

9. The animal feed product according to claim 8, wherein said chicory roots are disintegrated.

10. The animal feed product according to claim 8 or 9, wherein said low molecular sugars are selected from the group of glucose, fructose, sucrose, maltose, maltotriose, maltotetraose, and fructan.

11. The animal feed product according to any of the claims 8 to 10, wherein said secondary metabolites are selected from the group of

   • 8-Deoxylactucin, crepidiaside, lactucin, lactupicrin, crepidraside, 11-β-13-dihydrolactucin, picriside, sonchuside A, sonchuside C, cichoriolide A, cichoriosides A, cichorioside B and cichorioside C,
   • Sitosterol, stigmasterol, campersterol,
   • Esculetin, esculin, cichoriin-6'-p-hydroxyphenyl acetate, cichoriin,
   • Luteolin 7-glucuronide, quercetin 3-galactoside, quercetin 3-glucuronide, kaempferol 3-glucoside, kaempferol 3-glucuronide, isorhamnetin 3-glucuronide,
   • Cyanidin 3-O-β-(6-o-malonyl)-D-glucopyranoside, delphinidin derivatives,
   • Caffeic acid, chicoric acid, chlorogenic acid,
   • Putrescine, spermidine, and spermine.

12. The animal feed product according to any of the claims 8 to 10, wherein at least 50% of the product is from chicory roots.

13. Use of an animal feed product according to claim 8 to 12 as a feed product for "grown up" pigs at an age above 7 weeks.

14. Use of an animal feed product according to claim 8 to 12 for preparing a feed product for "grown up" pigs.

Figure 1

(a)

(b)

**Figure 2**

(a)

(b)

**Figure 3**

(a)

Figure 4

Skatole in blood plasma

## Figure 5

## Figure 6

Figure 7

Figure 8

Figure 9

Figure 9.

X-expl: 12%,7% Y-expl: 22%,26%

Figure 10

Correlation Loadings (X and Y)

OVERALL IMPRESSION
**3. PM2 chicory 1 (fresh)**

**4. PM2 inulin**

**1. PM2 control/silage**

**2. PM2 chicory 2 (dried)**

Figure 10. X-expl: 10%,9% Y-expl: 23%,23%

EP 2 289 348 A2

69

Figure 11

Figure 11. X-expl: 11%,9% Y-expl: 24%,23%

## REFERENCES CITED IN THE DESCRIPTION

### Patent documents cited in the description

- DE 4223051
- US 4971815 A, Tamatani
- US 4865852 A, Tamatani
- US 4671962 A
- US 4871574 A
- DE 4223051 A1

### Non-patent literature cited in the description

- Fenaroli's Handbook of Flavor Ingredients. 1995, vol. 3
- **Madsen et al.** *Energy level of the experiment is 95 % according to scale,* 1990
- A farm scale study on the use of de-odourase for reducing odour and ammonia emissions from intensive fattening piggeries. **Amon, M. ; Dobeic, M. ; Misselbrook, T.H. ; Pain, B.F. ; Phillips, V.R. ; Sneath, R.W.** Bioresource Technology. 1995, vol. 51, 163-169
- **Bach Knudsen, K. E.** Carbohydrates and lignin of plant materials used in animal production. *Animal Feed Science and Technology,* 1997, vol. 67, 319-338
- **Bach Knudsen, K. E ; Hessov, I.** Recovery of inulin from Jerusalem artichoke (Helianthus tuberosus L.) in the small intestine of man. *British Journal of Nutrition,* 1995, vol. 74, 101-113
- **Bais, H.P ; Ravishankar, G.A.** Cichorium intybus L - cultivation, processing, utility, value addition and biotechnology, with an emphasis on current status and future prospects. *Journal of the Science of Food and Agriculture,* 2001, vol. 81, 467-484
- **Bjørn,H. ; Roepstorff,A. ; Nansen,P.** A possible influence of diet composition on the establishment of nematodes in pigs. *Veterinary Parasitology,* 1996, vol. 63, 167-171
- **Boisen, S ; Fernandez, J. A.** Prediction of the total tract digestability of energy in feedstuffs and pig diets by in vitro analysis. *Animal Feed Science and Technology,* 1998, vol. 68, 277-286
- **Bonneau,M. ; Carelli, C.** Immunological castration of male pigs with a synthetic aqueous vaccine. *Annales de Zootechnie,* 1987, vol. 36, 265-269
- **Bonneau, M. ; Walstra, P. ; Claudi-Magnussen, C. ; Kempster, A.J. ; Tornberg, E. ; Fischer, K. ; Diestre, A. ; Siret, F. ; Chevillon, P. ; Claus, R.** An international study on the importance of androstenone and skatole for boar taint: IV. Simulation studies on consumer dissatisfaction with entire male pork and the effect of sorting out carcasses on the slaughter line, main conclusion and recommendations. *Meat Science,* 2000, vol. 54, 285-295
- **Byrne, D. V. ; Bak, L. S. ; Bredie, W. L. P. ; Bertelsen, G. ; Martens, M.** Development of a sensory vocabulary for warmed-over flavour: part I. in porcine meat. *Journal of Sensory Studies,* 1999, vol. 14, 47-65
- **Byrne, D. V. ; Bredie, W. L. P. ; Martens, M.** Development of a sensory vocabulary for warmed-over flavour: part II. in chicken meat. *Journal of Sensory Studies,* 1999, vol. 14, 67-78
- **Byrne, D. V. ; Bredie, W. L. P. ; Bak, L. S. ; Bertelsen, G. ; Martens, H. ; Martens, M.** Sensory and chemical analysis of cooked porcine meat patties in relation to warmed-over flavour and pre-slaughter stress. *Meat Science,* 2001, vol. 59, 229-249
- **Byrne, D.V. ; O'Sullivan, M.G. ; Dijkterhuis, G. ; Bredie, W.L.P. ; Martens, M.** Sensory panel consistency during the development of a vocabulary for warmed-over flavour. *Food Quality and Preference,* 2001, vol. 12, 171-187
- **Claus, R. ; D. Losel ; M. Lacorn ; J. Mentschel ; H. Schenkel.** Effects of butyrate on apoptosis in the pig colon and its consequences for skatole formation and tissue accumulation. *J. Anim Sci.,* 2003, vol. 81, 239-248
- **Dijksterhuis, G.B ; Engel, B. ; Walstra, P ; Font i Furnols ; M. Agerhem ; H.,Fischer, K. ; Oliver, M. A ; Oliver, Claudi-Magnussen, C. ; Siret, F. ; Béague, M.P.** An international study on the importance of androstenone and skatole for boartaint: II. Sensory evaluation by trained panels in seven European countries. *Meat Science,* 2000, vol. 54, 261-269

- **Dunshea, F.R. ; McCauley, I ; Corbett, J.** Immunization of pigs against gonadotrophin releasing factor (GnRF) prevents boar taint and affects boar growth and behaviour. *Recent advances in animal nutrition in Australia 2001, 16th Symposium, Armidale, Australia,* 2001, 13
- **Gemert, L.L. van ; Nettenbreijer, A.H.** Compilation of odour threshold values in air and water. National Inst. For Water Supply, Voorburg and Central Inst. *For Nutrition and Food Research TNO, Zeist, Netherlands,* 1977
- **Gibis, M. ; Hilmes, C ; Fischer, A.** Off-flavour in pork caused by skatole. *Fleischwirtschaft,* 1988, vol. 78, 727-730
- **Hale, O.M. ; Marti, O.G.** Influence of an experimental infection of Strongyloides ransomi on performance of pigs. *Journal of Animal Science,* 1984, vol. 58, 1231-1235
- **Hale, O.M ; Stewart, T.B.** Influence of an experimental infection of Trichuris suis on performance of pigs. *Journal of Animal Science,* 1979, vol. 49, 1000-1010
- **Hale, O.M. ; Stewart, T.B. ; Marti, O.G. ; Wheat, B.E. ; McCormick, W.C.** Influence of an experimental infection of nodular worms (Oesophagostomum spp.) on performance in pigs. *Journal of Animal Science,* 1981, vol. 52, 316-322
- **Hale, O.M. ; Stewart, T.B. ; Marti, O.G.** Influence of an experimental infection of Ascaris suum on performance of pigs. *Journal of Animal Science,* 1985, vol. 60, 220-225
- **Hansen, L.L. ; Larsen, A.E ; Hansen-Møller, J.** Influence of keeping pigs heavily fouled with faeces plus urine on skatole and indole concentration (boar taint) in subcutaneous fat. *Acta Agriculturae Scandinavica,* 1995, vol. 45, 178-185
- **Hansen,L.L. ; Larsen, A.E. ; Jensen, B.B ; Hansen-Møller, J ; Barton-Gade, P.** Influence of stocking rate and faeces deposition in the pen at different temperatures on skatole concentration (boar taint) in subcutaneous fat. *Animal Production,* 1994, vol. 59, 99-110
- **Hartung, J ; E. Rokicki.** Occurrence of phenolic compounds in the dust of swine and poultry houses. *Zentralblatt fur Bakteriologie Mikrobiologie und Hygiene,* 1984, vol. 1 (179), 431-439
- **Hansen, B. ; Sørensen, N. K.** Metodereferencer til centrallaboratoriets analyser. *Research Centre Foulum, Danish Institute of Animal Science,* 1996, vol. 79, 1-21
- **Hartung, E. ; Jungbluth, T ; Büscher, W.** Reduction of ammonia and odor emissions from a piggery with biofilters. *Transactions of the ASAE,* 2001, vol. 44, 113-118
- **Hazra, B ; Sarkar, R ; Bhattacharyya, S ; Roy, P.** Tumour inhibitory activity of chicory root extract against ehrlich ascites carcinoma in mice. *Fitoterapia,* 2002, vol. 73, 730-733
- **Hidaka, H. ; Eida, T. ; Takizawa, T. ; Tokunaga, T. ; Tashiro, Y.** Effects of fructooligosaccharides on intestinal flora and human health. *Bifidobacteria and Microflora,* 1986, vol. 5, 37-50
- **Hobbs, P.J. ; Pain, B.F. ; Kay, R.M. ; Lee, P.A.** Reduction of odourous compounds in fresh pig slurry by dietary control of crude protein. *J. Sci. Food Agric.,* 1996, vol. 71, 508-514
- **Jensen, M.T. ; Cox, R.P ; Jensen, B.B.** Microbial production of skatole in the hind gut of pigs given different diets and its relation to skatole deposition in backfat. *Animal Science,* 1995, vol. 61, 293-304
- Microbial production of skatole in the digestive tract of entire male pigs. **Jensen, B.B ; M.T. Jensen.** Skatole and boar taint. Danish Meat Research Institute, 1998, 41-75
- **Jensen, T.K. ; Svensmark, K.** Trichuriasis hos udendørs slagtesvin. *Veterinaerinformation,* 1996, vol. 2, 3-7
- **Knarreborg, A. ; Beck, J ; Jensen, M.T ; Laue, A ; Agergaard, N. ; Jensen, B.B.** Effect of non-starch polysaccharides on production and absorption of indolic compounds in entire male pigs. *Animal Science,* 2002, vol. 74, 445-453
- Immunomodulation by oligofructose and inulin. **Kelly-Quagliana, K.A. ; Buddington, R.K ; Van Loo, J ; Nelson, P.D.** Nutritional and Health Benefits of Inulin and Oligofructose. NIH, 1998, 53
- **Kisiel, W ; Michalska, K.** A new coumarin glucoside ester form Cichorium intybus. *Fitoterapia,* 2000, vol. 73, 544-546
- **Krebsky, E.O. ; Geuns, J.M.C ; De Proft, M.** Polyamines and sterols in Cichorium heads. *Phytochemistry,* 1999, vol. 50, 549-553
- **Larsson, K. ; Bengtsson, S.** Metodebeskrivning. *Statens lantbrukskemiske laboratorium,* 1983
- **Lenis, N.P ; Jongbloed, A.W.** New technologies in low pollution swine diets: diet manipulation and use of synthetic amino acids, phytase and phase feeding for reduction of nitrogen and phosphorous excretion and ammonia emission - review. *Asian - Aus. J. Anim. Sci.,* 1999, vol. 12, 305-327
- Anatomic content of the female and castrated male pig fed according to scale or ad libitum and slaughtered at 20, 50, 80 or 110 kg. **Madsen, A. ; J. S. Petersen ; Aa. Soegaard.** Communication no. 769. National Institute of Animal Science, 1990, 4
- **Meilgaard, M.C.** Flavour chemestri of beer. Part I: Flavour interaction between principal volatiles. *Techn. Q., Master Brew. Assoc. Am,* 1975, vol. 12, 107
- Measuring responses. **Meilgaard, M. ; Civille, G. V. ; Carr, B. T.** In Sensory evaluation techniques. CRC press, 1999, 43-57

- **Metz, C. ; K. Hohl ; S. Waidelich ; W. Drochner ; R. Claus.** Active immunization of boars against Gn-RH at an early age: consequences for testicular function, boar taint accumulation and N-retention. *Livestock Production Science,* 2002, vol. 74, 147-157
- **Nansen, P. ; Roepstorff, A.** Parasitic helminths of the pig: factors influencing transmission and infection levels. *International Journal for Parasitology,* 1999, vol. 29, 877-891
- **Nørbaek, R. ; Nielsen, K ; Kondo, T.** Anthocyanins from flowers of Cichorium intybus. *Phytochemistry,* 2002, vol. 60, 357-359
- **Pahl, O. ; Williams, A.G. ; Sneath, R.W.** Reduction of ammonia and odour emissions from pig slurry under slats using oil and foam layers. *Environmental Technology,* 2002, vol. 22, 395-430
- **Peters, A.M. ; Haagsma, N. ; Gensch, K.-H. ; Amerongen, A. van.** Production and characterization of polyclonal antibodies against the bitter sesquiterpene lactones of chicory (Cichorium intybus L.). *J. Agric. Food. Chem.,* 1996, vol. 44, 3611-3615
- **Bach Knudsen, K.E. ; Murrell, K.D. ; Wachmann, H.** The effect of inulin and sugar beet fibre on Oesphagostomum dentatum infection in pigs. *Parasitology,* 2003, vol. 127, 61-68
- **Bach Knudsen, K.E. ; Nansen, P ; Murrell, K.D.** The effect of dietary carbohydrates with different digestability on the population of Oesophagostomum dentatum in the intestinal tract of pigs. *Parasitology,* 2001, vol. 123, 315-324
- **Bach Knudsen, K.E. ; Nansen, P. ; Roepstorff, A. ; Skjøth, F ; Jensen, K.** The impact of diets varying in carbohydrates resistant to endogenous enzymes and lignin on populations of Ascaris suum and Oesophagostomum dentatum in pig. *Parasitologi,* 1997, vol. 144, 555-568
- **Nansen, P. ; Bach Knudsen, K.E. ; Skjøth, F.** The effect of increasing levels of insolubable dietary fibre on the establishment of Oesophagostomum dentatum in pigs. *Parasite,* 1999, vol. 6, 17-26
- **Poli, F. ; Sacchetti, G. ; Tosi, B. ; Fogagnolo, M. ; Chillemi, G. ; Lazzarin, R. ; Bruni, A.** Variation in the content of the main guaianolides and sugars in Cichorium intybus var. "Rosso di Chioggia" selections during cultivation. *Food Chemistry,* 2002, vol. 76, 139-147
- **Rees, S.B ; Harborne, J.B.** The role of sesquiterpene lactones and phenolics in the chemical defence of the chicory plant. *Phytochemistry,* 1985, vol. 24, 2225-2231
- **Roepstorff, A.** Helminth surveillance are a prerequisite for anthelmintic treatment in intensive sow herds. *Veterinary Parasitology,* 1997, vol. 73, 139-151
- **Roepstorff, A. ; Nansen, P.** Epidemiology and control of helmint infections in pigs under intensive and non-intensive productions systems. *Veterinary Parasitology,* 1994, vol. 54, 69-85
- Epidemiology, diagnosis and control of helminth parasites of swine. **Roepstorff, A. ; Nansen, P.** FAO Animal Health Manual. 1998, vol. 3, 161
- **Roepstorff, A. ; Eriksen, L. ; Slotved, H.-C. ; Nansen, P.** Experimental Ascaris suum infection in the pig: worm population kinetics following single infections with three doses of infective eggs. *Parasitology,* 1997, vol. 115, 443-452
- **Rosenvold, K.** Strategic feeding -- a tool in the control of technological pork quality. *Acta Universitatis Agriculturae Sueciae Agraria,* 2002, 59
- **Rosenvold, K. ; H. N. Laerke ; S. K. Jensen ; A. H. Karlsson ; K. Lundstrom ; H. J. Andersen.** Strategic finishing feeding as a tool in the control of pork quality. *Meat Sci.,* 2001, vol. 59, 397-406
- **Seto, M. ; Miyase, T. ; Umehara, K. ; Ueno, A. ; Hirano, Y. ; Otani, N.** Sesquiterpene lactones from Cichorium endivia L. and C. intybus L. and cytotoxic activity. *Chem. Pharm. Bull.,* 1988, vol. 36, 2423-2429
- **Slotved, H.-C. ; Barnes, E.H. ; Bjørn, H. ; Christensen, C.M. ; Roepstorff, A. ; Nansen, P.** Recovery of Oesophagostomum dentatum from pigs by isolation of parasites migrating from large intestinal contents embedded in agar-gel. *Veterinary Parasitologi,* 1996, vol. 63, 237-245
- **Slotved, H.-C. ; Barnes, E.H. ; Eriksen, L. ; Roepstorff, A. ; Nansen, P ; Bjørn, H.** Use of an agar-gel technique to recover Ascaris suum larvae from intestinal contents of pigs. *Acta Veterinaria Scandinavica,* 1997, vol. 38, 207-212
- **Sneath, R.W. ; Burton, C.H. ; Williams, A.G.** Continuous aerobic treatment of piggery slurry for odour control scaled up to a farm-size unit. *J. Agric. Engng. Res.,* 1992, vol. 53, 81-92
- **Stewart TB ; Hale OM ; Marti OG.** Experimental infections with Hyostrongylus rubidus and the effects on performance of growing pigs. *Veterinary Parasitology,* 1985, vol. 17, 219-229
- **Stoldt, W.** Vorschlag zur vereinheitlichung der fettbestimmung in lebensmitteln. *Fette, Seifen und Anstrichsmittel,* 1952, vol. 54, 206-207
- **Sultana, S. ; Perwaiz. S. ; Iqbal, M. ; Athar, M.** Crude extracts of hepatoprotective plants, Solanum nigrum and Cichorium intybus inhibit free radical-mediated DNA damage. *Journal of ethnopharmacology,* 1995, vol. 45, 189-192
- **Sutton, A.L. ; Kephart, K.B. ; Verstegen, M.W.A. ; Canh, T.T. ; Hobbs, P.J.** Potential for reduction of odorous compounds in swine manure through diet modifications. *J. Anim. Sci.,* 1999, vol. 77, 430-439

- **Thamsborg, S.M ; Roepstorff, A.** Parasite problems in organic livestock production systems and options for control. *Journal of Parasitology,* vol. 89, S277-S284
- **Thomsen, L.E. ; Bach Knudsen, K.E. ; Møller, K. ; Murrell, K.D. ; Roepstorff, A.** *The effect of dietary carbohydrates with different digestability on the population of Trichuris suis in the intestinal tract of pigs. (in preparation)*

- **Xu, Z.R. ; Hu, C.H. ; Wang, M.Q.** Effects of fructooligosaccharide on conversion of L-tryptophan to skatole and indole by mixed populations of pig fecal bacteria. *J.Gen.Appl.Microbiol.,* 2002, vol. 48, 83-90
- **Zahn, J.A. ; DiSpirito, A.A. ; Do, Y.S. ; Brooks, B.E. ; Cooper, E.E. ; Hatfield, J.L.** Correlation of human olfactory responses to airborne concentrations of malodourous volatile organic compounds emitted from swine effluent. *J. Environ. Qual,* 2001, vol. 30, 624-634